(19) Europäisches Patentamt
European Patent Office
Office européen des brevets

(11) EP 4 796 634 A1

(12) EUROPEAN PATENT APPLICATION

(43) Date of publication:
**26.08.2026 Bulletin 2026/35**

(21) Application number: **25158845.5**

(22) Date of filing: **19.02.2025**

(51) International Patent Classification (IPC):
***C12N 15/113*** *(2010.01)* ***A61K 31/7088*** *(2006.01)*

(52) Cooperative Patent Classification (CPC):
**C12N 15/113;** C12N 2310/11; C12N 2310/113; C12N 2310/315; C12N 2310/3231; C12N 2310/3341; C12N 2310/341

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Hannover Medical School (MHH)**
**30625 Hannover (DE)**

(72) Inventors:
- **THUM, Thomas**
**30627 Hannover (DE)**
- **BÜHRKE, Anne**
**29308 Winsen/Aller (DE)**
- **BÄR, Christian**
**30519 Hannover (DE)**

(74) Representative: **Gill Jennings & Every LLP**
**The Broadgate Tower**
**20 Primrose Street**
**London EC2A 2ES (GB)**

Remarks:
The complete document including Reference Table(s) and the Sequence Listing(s) can be downloaded from the EPO website

# (54) ANTISENSE OLIGONUCLEOTIDES (ASOS) FOR TREATMENT OF CARDIAC DISORDERS

(57) Provided herein is an antisense oligonucleotide for targeting long non-coding RNA maternally expressed gene 3 (lncRNA Meg3), wherein the oligonucleotide comprises or consists of one of the following sequences in the 5' to 3' direction: (i) C G C dC dG dC dC dA dT dA dT dC dT C C C (SEQ ID NO: 1); (ii) C G G dA dC dA dA dA dA dC dT dG dG T T G (SEQ ID NO: 2); (iii) AA G dA dG dT dC dT dC dC dT dC dC dT T AA (SEQ ID NO: 3); (iv) G A T dT dA dG dC dC dC dT dG dT dG dT T C A (SEQ ID NO: 4); (v) C G G dC dA dC dA dA dG dA dG dC dC dA A A G (SEQ ID NO: 5); (vi) T C T dC dC dT dC dC dT dT dA dA dG dC C C C (SEQ ID NO: 6); or (vii) G T C dT dC dC dT dC dC dT dT dA dA dG C C C (SEQ ID NO: 7); wherein dA, dT, dG and dC are deoxyribonucleotides, or wherein dC is 5-methyl-2'-deoxycytidine; and wherein A, T, G and C are modified or unmodified nucleotide building blocks.

(A)
Less than 50% reduction
Less than 60% reduction
MEG3/TBP
1.4
1.2
1.0
0.8
0.6
0.4
0.2
0.0
HCF Ctrl
1154 16
1154 17
1155 16
1156 16
1209 17
1210 16
1210 17
1211 16
1211 17
1212 16
136 17
14146 17
14185 17
14186 17
14196 17
14197 17
14198 17
14204 17
14205 17
14206 17
14207 17
14208 17
14209 17
14210 17
14343 17
14344 17
14345 17
14421 17
16044 17
16045 16
16045 17
16046 16
16189 17
16191 17
16194 17
16195 17
19749 17
19754 17
19756 16
19756 17
19928 17
19929 16
19930 17
19931 16
19931 17
19933 17
19934 17
19935 16



Processed by Luminess, 75001 PARIS (FR)

## Description

## FIELD OF THE INVENTION

The present invention refers to oligonucleotides, which are effective in the treatment or prevention of cardiac diseases or disorders.

## BACKGROUND

Pathological cardiac remodelling and subsequent heart failure (HF) represent the leading cause of morbidity without relevant curative treatment options. The excess deposition of collagenous scar tissue referred to as cardiac fibrosis represents the main driver of HF development. Therefore, there is a great need for anti-fibrotic treatments.

Several studies indicate that these non-coding RNAs (ncRNAs) have as important biological functions as their protein coding counterparts and suggest that altered expression or function of ncRNAs effects cardiovascular diseases, including cardiac diseases such as, for example, cardiac hypertrophy and cardiac fibrosis. It is known that the expression or dysfunction of long non-coding RNAs (lncRNAs) is closely related to various hereditary diseases, autoimmune diseases, metabolic diseases and tumours (Zhang *et al.,* 2018).

The most reflected ncRNAs in cardiovascular research are microRNAs (miRNAs, miRs). These are endogenous, single-stranded RNAs composed of approximately 20-22 nucleotides that bind other transcripts reducing the stability and/or translation of their targets. For example, it was shown that miR-21 and miR-132 induce cardiac fibrosis or hypertrophy, respectively, and that *in vivo* repression of these miRNAs by specific AntagomiRs (being chemically engineered oligonucleotides silencing miRNAs) rescues fibrosis or hypertrophy in cardiac disease model of pressure-overload (Thum *et al.,* 2008; Ucar *et al.,* 2012).

Similar to miRNAs, long non-coding RNAs (lncRNAs) may also play an important role in various biological processes. LncRNAs are mRNA-like transcripts ranging from 200 nucleotides up to 100 kilo bases and are classified based on their genomic distribution relative to protein-coding genes (sense to exons and/or introns, antisense, bidirectional, or intergenic). Several lncRNA transcripts are exclusively restricted to the nucleus, while others are also found in the cytoplasm. In the cytoplasm, they interact with proteins as well as other RNA or DNA molecules enabling lncRNAs to influence a variety of gene regulatory mechanisms including chromatin modification, genomic imprinting, nuclear compartmentalization and architecture, as well as transcriptional and post-transcriptional regulation (Schonrock *et al.,* 2012; Caley *et al.,* 2010). Not surprisingly, lncRNAs are involved in human disease, such as cancer, metabolic and neuronal disorders.

Little is known about the role of lncRNAs in regulating cardiovascular biology and in particular in fibrotic diseases. Previous studies have shown that the two lncRNAs Braveheart (Bvht) and FOXF1 adjacent non-coding developmental regulatory RNA (Fendrr) are required for the differentiation of cardiomyocytes and the development of lateral mesoderm tissue in the heart and body wall, respectively (Klattenhoff *et al.,* 2013; Grote *et al.,* 2013).

More recently, the role of lncRNAs in the development of cardiovascular diseases has been investigated. For example, genome-wide studies identified singlenucleotide polymorphisms (SNPs) in loci encoding for the lncRNAs MIAT (myocardial infarction-associated transcript) or ANRIL (antisense noncoding RNA in the INK4 locus) that seem to be related to risk of myocardial infarction or coronary artery disease (Ishii *et al.,* 2006; McPherson *et al.,* 2007). The lncRNA Kcnq1ot1 controls the expression of its antisense gene Kcnq1 40 that encodes for a potassium channel. Since the potassium channel activity is essential for a normal cardiac performance, an altered regulation related by lncRNAs might lead to an abnormal heart function (Korostowski *et al.,* 2012). The circulating lncRNA LIPCAR can be used to predict survival in patients with heart failure (Kumarswamy *et al.,* 2014). Moreover, the lncRNA Chast has been shown to promote cardiac remodelling (Viereck *et al.* (2016)).

The lncRNA maternally expressed gene 3 (Meg3) is a significant class of lncRNA known to substantially functions in diverse biological processes such as proliferation, apoptosis, and angiogenesis. Meg3 has been identified to play a role in breast cancer (BC) development and function as a tumour suppressor (Zhang *et al.,* 2022). Also, Meg3 is highly expressed in cardiac fibroblasts (CFs) and known to undergo transcriptional downregulation during late cardiac remodelling (Piccoli *et al.,* 2017). *In vitro,* Meg3 regulated the production of matrix metalloproteinase-2 (MMP-2). Furthermore, lncRNA *Meg3* has been identified as a promising target for an anti-fibrotic strategy as *Meg3* inhibition significantly reduced cardiac fibrosis in a mouse model of HF (Piccoli *et al,* 2017).

WO 2017/191021 relates to compounds inhibiting the expression and/or the activity of Meg3. The inventors used nucleotide-based inhibitors selected from siRNAs, shRNAs and antisense oligonucleotides to target Meg3. The use of a compound inhibiting the expression and/or the activity of Meg3 for treating or preventing cardiac remodelling has previously been described in EP 3 452 593..

Similarly, Gokey *et al.* (2018) showed that pulmonary epithelial cells isolated from idiopathic pulmonary fibrosis (IPF) lung tissue demonstrated altered expression of lncRNAs, including increased MEG3. MEG3 RNA was highly

expressed in subsets of the atypical IPF epithelial cells and correlated with conducting airway epithelial gene expression patterns.

Fibrosis is generally very heterogeneous and an essential process in wound healing. However, excessive fibrosis is common in many disease conditions and plays an important role in disease pathogenesis. The process of fibrosis is initiated when immune cells, such as macrophages, release soluble factors that stimulate fibroblasts. The most well-characterized pro-fibrotic mediator is tumour growth factor-beta (TGF-β), which is released by macrophages and any damaged tissue between interstitial surfaces. Diseases characterized by excessive fibrosis include, amongst others, hypertrophic cardiomyopathy, dilated cardiomyopathy (DCM), atrial fibrillation, ventricular fibrillation, myocarditis, asthma, and idiopathic pulmonary fibrosis. Cardiac fibrosis is a common pathophysiologic process in most heart diseases and characterised by scarring events in the cardiac muscle and hardening of tissues and organs that lead to cardiac fibroblast (CF) activation and their differentiation into myofibroblasts. This is followed by an increase in and excess production of extracellular matrix (ECM) proteins such as collagen and proteases including the matrix metalloproteinases (MMPs) (Jiang *et al.,* 2021). The resultant disturbance of the ECM homeostasis leads to pathological ECM remodelling and profound structural and functional abnormalities in matrix composition and quality (*e.g.*, matrix stiffness), as well as the heart muscle (*e.g.,* diastolic and systolic dysfunction) (Berk *et al.,* 2007; Kong *et al.,* 2014). While cardiac fibrosis is associated with different cardiovascular diseases, including heart failure, hypertension, and cardiomyopathies, fibrotic scaring of the cardiac muscle most commonly occurs after myocardial injury, when CFs are converted into myofibroblasts by upregulating expression of pro-inflammatory cytokines. The use of a compound inhibiting the expression and/or the activity of Meg3 for treating or preventing cardiac remodelling has previously been described in EP 3 452 593.

There are currently no drugs on the market with primarily anti-fibrotic action that have been proven to reverse the conditions or halt their progress associated with cardiac fibrosis. Antifibrotic drugs known from clinical studies include, for example, RASS inhibitors lisinopril, losartan, and spironolactone, inflammatory modulators, such as, etanercept, infliximab, colchicine, and atorvastatin, and TGF-beta signalling inhibitors. However, the role of statins in the treatment of chronic heart failure (HF) is still controversial. Although several retrospective studies have revealed a better prognosis for patients with HF treated with statins, two randomized clinical trials, GISSI-HF (Italian Group for the Study of Survival in Heart Failure Failure) and CORONA (Controlled Rosuvastatin Multinational Trial in Heart Failure), reported no prognostic benefit from rosuvastatin treatment. Furthermore, anti-TGF-beta antibody therapy has also been associated with serious adverse effects (Frantz *et al.,* 2008). More recent studies have even looked at using CAR-T cells engineered *in vivo* to contain or express a receptor directed against the FAP protein, which is expressed by cardiac myofibroblasts (Morfino *et al.,* 2022). However, despite the ongoing research, there is currently no anti-fibrotic drug that has clearly demonstrated the regression of fibrosis and the health improvement in clinical trials.

Accordingly, as the therapeutic and diagnostic methods relating to cardiac and cardiopulmonary inflammation and cardiac fibrosis still require further improvement and new approaches, there is an unmet medical need for new therapeutic agents to diagnose and treat these conditions. As will be shown throughout the specification, with the present invention the inventors aim to address this need.

## SUMMARY OF THE INVENTION

The inventors have discovered that specific antisense oligonucleotides (ASOs) targeting lncRNA Meg3 have the potential to improve or treat fibrosis, and that these ASOs may also be used in the diagnosis thereof. The problem solved by the present invention lies in the provision of improved treatment therapies for the treatment of fibrotic disorders. Specifically, the present invention provides synthetic ASOs that specifically target (endogenous) lncRNA Meg3. To date, no prior art has been identified that teaches or suggest that disorders and/or diseases associated with cardiac fibrosis can be treated using the particular ASOs of the invention. The inventors have surprisingly discovered that the synthetic ASOs of the invention provide the advantage of effectively inhibiting functional lncRNA Meg3 to reduce (cardiac) inflammation and fibrosis. The ASOs of the invention, are mixmers comprising a combination of DNA and RNA. In some instances, the individual nucleotides may be modified to comprise, *e.g.*, 5-methyl-2'-deoxycytidine. In some instances, the ASOs further comprise a combination of LNA building blocks and internucleoside phosphorothioate (PS) linkage modifications. The ASOs of the invention lack significant toxicity in a variety of human cells derived from various tissues and isolated neonatal rat cardiomyocytes. Further, ASOs of the invention targeting Meg3 exhibited superior effects compared to other oligonucleotide analogues having a different nucleotide sequence and a different combination of nucleotide modifications and internucleoside linkage modifications.

The present invention is defined by the claims.

In a first aspect, the present invention provides an antisense oligonucleotide for targeting long non-coding RNA maternally expressed gene 3 (lncRNA Meg3), wherein the oligonucleotide comprises or consists of one of the following sequences in the 5' to 3' direction

(i) C G C dC dG dC dC dA dT dA dT dC dT C C C (SEQ ID NO: 1);

(ii) C G G dA dC dA dA dA dA dC dT dG dG T T G (SEQ ID NO: 2);
(iii) A A G dA dG dT dC dT dC dC dT dC dC dT T A A (SEQ ID NO: 3);
(iv) G A T dT dA dG dC dC dC dT dG dT dG dT T C A (SEQ ID NO: 4);
(v) C G G dC dA dC dA dA dG dA dG dC dC dA A A G (SEQ ID NO: 5);
(vi) T C T dC dC dT dC dC dT dT dA dA dG dC C C C (SEQ ID NO: 6); or
(vii) G T C dT dC dC dT dC dC dT dT dA dA dG C C C (SEQ ID NO: 7); wherein dA, dT, dG and dC are deoxyribonucleotides, or wherein dC is 5-methyl-2'-deoxycytidine (5mdC); and wherein A, T, G and C are modified or unmodified nucleotide building blocks.

In a second aspect, provided herein is a cell comprising an antisense oligonucleotide of the invention.
In a third aspect provided herein is a vector comprising an antisense oligonucleotide of the invention.
In a fourth aspect provided herein is a pharmaceutical composition comprising the antisense oligonucleotide of the invention.
In a fifth aspect provided herein is an antisense oligonucleotide of the invention, a vector of the invention, or a composition of the invention for prophylactic or therapeutic use in a subject.
In a sixth aspect provided herein is an antisense oligonucleotide of the invention, a vector of the invention, or a composition of the invention for use in the prophylaxis or treatment of a cardiac disease.
In a seventh aspect provided herein is a method of delivering an oligonucleotide of the invention or a composition of the invention to cardiac cells, comprising contacting the cardiac cells with an oligonucleotide of the invention or a composition of the invention.
In an eighth aspect provided herein is a method for determining treatment efficacy in a patient using an oligonucleotide of the invention or a composition the invention, the method comprising: (a) detecting the expression level of Meg3 in a sample previously obtained from a patient, and (b) comparing the expression level obtained in (a) with the expression level of Meg3 in a sample previously obtained from at least one healthy subject or with a predetermined standard that has been obtained from a sample of at least one healthy subject, wherein a 2-fold downregulation of Meg3 is indicative for a prophylactic or therapeutic response in the patient.
In a ninth aspect provided herein is the use of the antisense oligonucleotide according to the invention for the treatment and/or prevention of a cardiac disease.
In a tenth aspect provided herein is a kit comprising means for the detection of the expression level of Meg3, and instructions how to use the kit.
It is an advantage of this invention to provide new and improved oligonucleotides. Another advantage of the invention is that it provides new and improved compositions comprising said oligonucleotides. A still further advantage of this invention is that a pharmaceutically acceptable composition is utilized to prevent and reduce cardiac and pulmonary inflammation and fibrotic disorders. Additional advantages of the invention will be set forth in part in the description, which follows and in part will be obvious from the description or may be learned by practice of the invention.

## BRIEF DESCRIPTION OF DRAWINGS

The figures shown in the following are merely illustrative and shall describe the present invention in a further way. The figures shall not be construed to limit the present invention thereto.

**Fig. 1** presents the of antisense oligonucleotides (ASOs) provided by Axolabs GmbH and a map of the *IncMeg3* gene locus. 96 ASOs were bioinformatically generated from the human MEG3 sequence and tested regarding efficiency and safety. Shown is the target size location of the different ASOs in the full genomic sequence and in the splice transcripts of Meg3.
**Fig. 2** relates to the efficiency of *MEG3*-specific antisense oligonucleotides (ASOs) ((A) and (B)) in human cardiac fibroblasts (HCFs).
**Fig. 3** relates to the effect of *MEG3*-specific antisense oligonucleotides (ASOs) ((A) and (B)) on *MMP2* expression levels in human cardiac fibroblasts (HCFs).
**Fig. 4** relates to the effect of *MEG3*-specific antisense oligonucleotides (ASOs) ((A) and (B)) on *COL1A1* expression levels in human cardiac fibroblasts (HCFs).
**Fig. 5** presents graphs showing the cytotoxic evaluation of 22 pre-selected antisense oligonucleotides (ASOs) in HCFs ((A) and (B)) and liver cells (HepG2) ((C) and (D)).
**Fig. 6** presents graphs showing the levels of *Meg3* gene expression and fibrosis-related genes *MMP2, COL1A1* and *COL3A 1* 72h after transfection of ((A)-(D)) cardiac (HCF), ((E)-(H)) embryonic lung (MRC5), ((I)-(L)) primary liver (HPLF) and ((M)-(P)) primary kidney (HPKF) fibroblasts (50nM; n = 1 individual experiment).
**Fig.** 7 represents graphs showing the cell viability and cytotoxic effects of the the 7 pre-selected ASOs using WST-1 and LDH assays in human cardiac fibroblasts (HCF) ((A) and (B)), MRC5 ((C) and (D)), HPLF ((E) and (F)), HPKF ((G)

and (H)), and HepG2 liver cells ((I) and (J)).

**Fig.** 8 presents graphs showing the levels of *Meg3* gene expression and fibrosis-related genes *MMP2, COL1A1* and *COL3A* 1 in the presence of the 7 selected ASO candidates.

**Fig.** 9 presents graphs showing (A) timeline of pharmacokinetic (PK) study in rats and quantification of FIBEX-003 (ng/mL) in rat plasma (B) after intravenous (i.v.) dosing or (C) after subcutaneous (s.c.) dosing.

**Fig. 10** presents graphs showing (A) the concentration of FIBEX-003 in rat cardiac tissue; (B) TNFα expression in rat liver tissue as a result of FIBEX-003, (C) IL-6 in rat kidney tissue as a result of FIBEX-003.

**Fig. 11** presents graphs showing (A) study design of combined pharmacokinetic (PK)/ range finding (RF) study in minipig and the results of (B) FIBEX-003 quantification in minipig plasma and in (C) minipig cardiac tissue.

**Fig. 12** presents graphs showing the results of gene expression analysis in cardiac tissue ((A) and (B)) and in kidney tissue (C) in minipig.

**Fig. 13** presents graphs showing (A) study design of Dose Range Finding (DRF), and (B) serum TNFα levels of the test item dose groups compared to the vehicle group at day -14 (basis levels), day 2 and day 16 of the study in male and female Wistar Han rats.

**Fig. 14** presents graphs showing Serum IL-1β levels of the test item dose groups compared to the vehicle group at day -14 (basis levels), day 2 and day 16 of the study in male and female Wistar Han rats.

**Fig. 15** represents graphs showing serum IL-6 levels of the test item dose groups compared to the vehicle group at day -14 (basis levels), day 2 and day 16 of the study in male and female Wistar Han rats.

**Fig. 16** represents graphs showing serum IFN-γ levels of the test item dose groups compared to the vehicle group at day -14 (basis levels), day 2 and day 16 of the study in male and female Wistar Han rats.

**Fig. 17** represents graphs showing quantification of FIBEX-003 in cardiac tissue of the Dose Range Finding (DRF) study including indicative immune toxicology in rats.

**Fig. 18** represents graphs showing the results of (A) Meg3 and (B) IL6 gene expression level in cardiac tissue; and (C) IL6 in kidney and (C) TNF-α in liver of the Dose Range Finding (DRF) study including immune toxicology in rats.

**Fig. 19** represents graphs showing results identifying *MEG3* interaction partners in HCFs. (A) Probe test to establish the pulldown procedure. (B) Generation of samples for mass spectrometry and (C) identification of potential protein interaction partners (n = 6).

## DETAILED DESCRIPTION

### Terminology

In order that the present invention may be more readily understood, certain terms are first defined.

Articles "a" and "an" used herein refer to one or to more than one (*i.e.,* to at least one) of the grammatical object of the article. By way of example, "an element" means one element or more than one element, *e.g.*, a plurality of elements.

The term "including" is used herein to mean, and is used interchangeably with, the phrase "including, but not limited to". Likewise, the term "comprising" is used herein to mean, and is used interchangeably with, the phrase "comprising, but not limited to".

The terms "about" and "approximately" may be understood to permit standard variation as would be understood by those of ordinary skill in the art; where ranges are provided, endpoints are included.

As used herein, a "nucleotide" refers to a monomeric unit of an oligonucleotide or polynucleotide that includes a nucleoside and an internucleoside linkage.

As used herein, the term "nucleic acid" is intended to include any DNA molecules *(e.g.,* cDNA or genomic DNA) and any RNA molecules *(e.g.,* mRNA) and analogues of the DNA or RNA generated using nucleotide analogues. The nucleic acid can be single-stranded or double-stranded. Each component of the DNA or RNA structure can be modified and be categorized by modification of (1) the internucleoside linkage, (2) the deoxyribose/ribose, and/or (3) the nucleobase.

The term "oligonucleotide" or "oligonucleotides" as used herein is defined as it is generally understood by the person skilled in the art as a molecule including two or more covalently linked nucleosides (*e.g.*, short nucleic acid polymer(s) linked by internucleoside linkage(s)). They can comprise DNA, RNA or a mixture of RNA and DNA. The oligonucleotides of the invention are defined by and can comprise and any of SEQ ID NOs: 1 to 28.

The term "nucleobase" refers to nitrogen-containing biological building blocks that form nucleosides, which, in turn, are components of nucleotides. The naturally occurring bases [guanine, (G), adenine, (A), cytosine, (C), thymine, (T), and uracil (U)] are derivatives of purine or pyrimidine, though it should be understood that naturally and non-naturally occurring base analogues are also included and that the term "nucleobase" also includes "modified nucleobases". In one embodiment, G, A, C, and/or T are RNA or DNA.

Within the context of this invention, the term "modified nucleobase" and "modified base" may be used interchangeably with the term "nucleobase". Nucleobases may be modified or unmodified. Hence, in some embodiments, a

modified nucleobase is a nucleobase which comprises a modification. In some embodiments, a modified nucleobase is capable of at least one function of a nucleobase, *e.g.*, forming a moiety in a polymer capable of base-pairing to a nucleic acid comprising an at least complementary sequence of bases. In one embodiment, the modified nucleobase is capable of increasing hydrogen bonding, base pair stacking interactions and/or stabilizing a nucleic acid complex. In some embodiments, a modified nucleobase is substituted A, T, C, G, or U, or a substituted tautomer of A, T, C, G, or U. In some embodiments, a modified nucleobase in the context of oligonucleotides refer to a nucleobase that is not A, T, C, G or U. Modifications include but are not limited to nonstandard nucleobases 5-methyl-2'-deoxycytidine (d5m), pseudouridine (pU), dihydrouridine, inosine (I), and 7-methylguanosine.

The term "nucleoside(s)" refers to a moiety wherein a nucleobase or a modified nucleobase is covalently bound to a sugar or a modified sugar. In some embodiments, a "nucleoside" refers to a nucleoside unit in an oligonucleotide or a nucleic acid. The term "nucleoside(s)" encompasses all modified versions and derivatives "modified nucleobases".

As used herein, the term "internucleoside linkage(s)" refers to a linkage between adjacent nucleosides. "Internucleoside linkage" and "linkage" may be used interchangeably. Linkages comprise, but are not limited to, phosphate (PO), phosphorodiamidate or phosphorothioate (PS) linkages. Internucleoside linkages may include any modified internucleoside linkage. Unless otherwise specified, description of oligonucleotides and elements thereof (*e.g.*, base sequence, sugar modifications, internucleoside linkages, linkage phosphorus stereochemistry, patterns thereof, *etc.*) is from 5' to 3'. As those skilled in the art will appreciate, in some embodiments, oligonucleotides may be provided and/or utilized as salt forms, particularly pharmaceutically acceptable salt forms, *e.g.*, sodium salts.

As used herein the term "antisense oligonucleotide" or "ASO" refers to a short strand of nucleotide analogue that hybridizes with the complementary RNA (microRNA, mRNA, ncRNA, *etc.*) or any other target RNA in a sequence-specific manner via Watson-Crick base pairing. The ASO can comprise DNA and RNA. The ASO may be chemically modified. As used herein the terms "antisense oligonucleotide" (ASO) and "oligonucleotide" may be used interchangeably.

The term "hydroxy", as used herein, represents an -OH group.

The term "modified sugar" refers to a moiety that can replace a sugar. A modified sugar mimics the spatial arrangement, electronic properties, or some other physicochemical property of a sugar. A modified sugar may be substituted ribose or deoxyribose. In some embodiments, a modified sugar comprises a 2'-modification. Nucleotide modifications include LNA-modification(s) and/or 5-Methyl-2'-deoxycytidine modification(s). The terms "locked nucleic acid" (LNA) or "locked nucleic acids" (LNAs) are known as bridged nucleic acid (BNA) and refer to the modification of the 2'-sugar modification of one or more nucleotides with an extra bridge (connecting the 2' oxygen and 4' carbon of the sugar). Within each oligonucleotide there can be one or more nucleotides having the same modification. In one embodiment, the oligonucleotide comprises LNA(s). In one embodiment, the oligonucleotide comprises DNA and/or RNA and/or LNA(s).

As used herein, the term "complementary" or "partially complementary" or "substantially complementary" refer to nucleic acid sequences, which due to their complementary nucleotides are capable of specific intermolecular base-pairing. For example, the oligonucleotide may comprise a nucleic acid sequence complementary to a target sequence. Hence, the complementarity of the ASOs of the invention may be 100%. In one embodiment, the complementarity is at least 80%, 85%, 90%, 95%. In one embodiment, the complementarity is 85%-99%.

The Term "IncRNA" refers to long non-coding RNA. Within the context of this application the term "IncRNA" may be used interchangeably with "RNA". The oligonucleotides of the invention may be used to downregulate or block IncRNA, specifically long non-coding RNA (IncRNA) known as Meg3 (Maternally Expressed Gene 3).

As used herein, the terms "disease" or "disorder" are used interchangeably to refer to a condition in a subject. In certain embodiments, the condition is a disease in a subject, the severity of which is decreased by inducing an immune response through the administration of a pharmaceutical composition.

As used herein, the term "effective amount" in the context of administering a therapy to a subject refers to the amount of a therapy which has a prophylactic and/or therapeutic effect(s).

As used herein, the term "in combination" in the context of the administration of two or more therapies, refers to the use of more than one therapy (*e.g.*, more than one prophylactic agent and/or therapeutic agent). The use of the term "in combination" does not restrict the order in which therapies are administered.

As used herein, the terms "prevent", "preventing" and "prevention" in the context of the present invention and the administration of a therapy(ies) to a subject refers to the inhibition of the development or onset of a disease or a symptom thereof. In one embodiment, it relates to the administration of the compound to a patient who is known to have an increased risk of developing a certain disorder.

As used herein, the terms "treat", "treating" and "treatment" refer to the administration of the compound to a patient which has already developed signs and/or symptoms of a certain disorder. Treating covers treatment of an existing condition, inhibiting the progress or development of the condition, ameliorating the condition, and providing palliation of the condition.

The terms "subject" and "patient" are used interchangeably and relate to an animal (e.g., mammals) in need of administration of the oligonucleotide or composition of the invention. In specific embodiments, the subject is a human.

As used herein, the term "pharmaceutically acceptable" means that which is useful in preparing a pharmaceutical

composition and is generally safe, non-toxic, and neither biologically nor otherwise undesirable and includes that which is acceptable for veterinary use as well as human pharmaceutical use. Pharmaceutically acceptable substances are those that are generally approved by a regulatory agency.

The term "carrier" refers to a diluent, adjuvant, excipient, or vehicle with which the oligonucleotide or pharmaceutical composition comprising the same of the invention is administered. Saline solutions and aqueous dextrose and glycerol solutions can also be employed as liquid carriers, particularly for injectable solutions. Suitable excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The formulation should suit the mode of administration. As those skilled in the art will appreciate, methods and compositions described herein relating to provided oligonucleotides generally also apply to pharmaceutically acceptable salts of such compounds.

Within the context of this application, the expression "standard of care" (SoC) refers to any given regimen involved in the prevention and/or treatment of a cardiac and/or fibrotic condition, disease or disorder. This may include informal or formal guidelines generally accepted in the medical community for the treatment of the particular condition. SoC may be a medical treatment guideline and/or it may be developed by a specialist society or organization. For instance, renin-angiotensin system (RAS) inhibitors are currently used as standard therapy for heart failure (HF).

Unless otherwise defined, all technical and scientific terms used herein have the same meaning as commonly understood by one of ordinary skill in the art to which this invention belongs. Methods and materials are described herein for use in the present disclosure; other, suitable methods and materials known in the art can also be used.

## Antisense Oligonucleotides (ASOs) and Compositions

Oligonucleotides are useful in various therapeutic, diagnostic, and research applications. Use of naturally occurring nucleic acids may however be limited, for example, by their susceptibility to endo- and exonucleases. As such, various synthetic ASO candidates have been developed to circumvent these shortcomings and/or to further improve various properties and activities. These include synthetic ASOs containing chemical modifications, *e.g.*, base modifications, sugar modifications, internucleoside linkage modifications, *etc.,* which, among other things, render these molecules less susceptible to degradation and improve other properties and/or activities. The present disclosure utilizes technologies for controlling various structural elements, *e.g.*, sugar modifications and patterns thereof, nucleobase modifications and patterns thereof, modified internucleoside linkages and patterns thereof, additional chemical moieties (moieties that are not typically in an oligonucleotide chain) and patterns thereof, *etc.*. With the capability to fully control structural elements of oligonucleotides, the present disclosure provides oligonucleotides with improved properties and/or activities for various applications, *e.g.*, as therapeutic and/or diagnostic agents.

Provided herein are, *inter alia,* chemically modified ASOs comprising varying designs of nucleobase modifications and patterns thereof, sugars and patterns thereof, internucleoside linkage modifications and patterns thereof, and/or additional chemical moieties and patterns thereof. While not intending to be bound by any theory of operation, it is believed that particular nucleobase and backbone linkage modifications of said ASOs are useful in stabilising and improving the effect of the ASOs.

Specifically, provided herein are ASOs that target and/or inhibit lncMeg3. The oligonucleotides provided herein are oligonucleotides that comprise or consist essentially of a sequence complementary to the target lncMeg3. In one embodiment, an ASO of the invention is complementary or at least partially complementary to lncMeg3. In one embodiment, an ASO of the invention is complementary or at least partially complementary to part of the Meg3 gene (Gene ID:55384, ENSG00000214548) transcript. Hence, the ASOs of the invention provide the advantage of effectively modulating lncMeg3 function. In one embodiment, the lncMeg3 is endogenous lncMeg3. Therefore, the ASOs of the invention and compositions comprising the same may be generally useful for therapeutic use and for treating and preventing cardiac and/or pulmonary inflammation and/or fibrotic disorders. At the same time, the ASOs of the invention may be used in diagnostic assays.

Generally described herein are modified ASOs and compositions comprising the same as well as oligonucleotides and compositions comprising the same for therapeutic use and for use in the prophylaxis and/or treatment of fibrotic disorders in a subject.

As is known to those of skill in the art, ASOs can be used as diagnostic agents, therapeutic agents, probes, *etc.*. It is believed that the claimed oligonucleotides, having a particular sequence, nucleotide modification(s), and backbone modification(s), provide highly specific and stable oligonucleotides that target lncMeg3. It is believed that these synthetic ASOs are effective in controlling lncMeg3 compared to unmodified ASOs with less modified backbones that are less stable and less effective in targeting lncMeg3.

Advantageously, the oligonucleotides provided herein are useful in the specific targeting of lncMeg3 to modulate an anti-fibrotic response and thereby provide means against fibrotic disorders, in particular cardiac fibrosis. Thus, the oligonucleotides of this invention and compositions comprising the same are useful as agents in medicine, particularly in the prevention and/or treatment of fibrotic disorders.

The inventors have realised that providing oligonucleotides in the context of non-coding RNA-based therapeutics, it is beneficial to incorporate certain features into the oligonucleotides to provide effective ASOs for the therapeutic targeting of noncoding RNAs. Moreover, the inventors submit that the ASOs of the invention are able to act on several key disease pathways simultaneously, triggering a concerted therapeutic effect against key hallmarks of heart disease including cardiac fibrosis.

Accordingly, provided herein are ASOs for targeting long non-coding RNA maternally expressed gene 3 (IncRNA Meg3), wherein the oligonucleotide comprises or consist of one of the following sequences in the 5' to 3' direction:

(i) C G C dC dG dC dC dA dT dA dT dC dT C C C (SEQ ID NO: 1);
(ii) C G G dA dC dA dA dA dA dC dT dG dG T T G (SEQ ID NO: 2);
(iii) A A G dA dG dT dC dT dC dC dT dC dC dT T A A (SEQ ID NO: 3);
(iv) G A T dT dA dG dC dC dC dT dG dT dG dT T C A (SEQ ID NO: 4);
(v) C G G dC dA dC dA dA dG dA dG dC dC dA A A G (SEQ ID NO: 5);
(vi) T C T dC dC dT dC dC dT dT dA dA dG dC C C C (SEQ ID NO: 6); or
(vii) G T C dT dC dC dT dC dC dT dT dA dA dG C C C (SEQ ID NO: 7); wherein dA, dT, dG and dC are deoxyribonucleotides, or wherein dC is 5-methyl-2'-deoxycytidine; and wherein A, T, G and C are modified or unmodified nucleotide building blocks. In one embodiment, G, A, C, and/or T are RNA or DNA.

Sugars can be bonded to internucleoside linkages at various positions. As non-limiting examples, internucleoside linkages can be bonded to the 2', 3', 4' or 5' positions of sugars. In some embodiments, as most commonly in natural nucleic acids, an internucleoside linkage connects with one sugar at the 5' position and another sugar at the 3' position unless otherwise indicated. In one embodiment, the oligonucleotide comprises at least one modified internucleoside linkage. In one embodiment, at least 1, 2, 3, 4, 5, 6, or 7 internucleoside linkages are modified. In one embodiment, the oligonucleotide comprises one or more phosphate (PO) linkages. In one embodiment, the modified internucleoside linkages are phosphorothioate linkages. In one embodiment, the modified internucleoside linkages are phosphorodiamidate linkages. In one preferred embodiment, the oligonucleotide comprises one or more phosphorothioate (PS) linkages. In one embodiment, the oligonucleotide comprises one or more phosphorodiamidate linkages. In one embodiment the oligonucleotide comprises phosphorothioate, phosphate, and/or phosphorodiamidate linkages. In one embodiment, all internucleoside linkages are modified. In one embodiment, all internucleoside linkages are PS linkages.

Various nucleobases may be utilized in provided oligonucleotides in accordance with the present disclosure. Suitable technologies for nucleobase modification in oligonucleotide synthesis are widely known in the art and may be utilized in accordance with the present disclosure. Nucleobases may be modified. In some embodiments, modified nucleobases improve properties and/or activities of oligonucleotides. For example, in many cases, 5mC may be utilized in place of C to modulate certain undesired biological effects, *e.g.*, immune responses. Hence, in one embodiment, the oligonucleotide comprises 5mC. In some embodiments, a nucleobase is a natural nucleobase, the most commonly occurring ones being A, T, C, and G. In some embodiments, a nucleobase is a modified nucleobase in that it is not A, T, C, or G. In one embodiment, a nucleobase is a substituted purine. In one embodiment, a nucleobase is a substituted pyrimidine base. In a preferred embodiment, each C is 5-methyl-2'-deoxycytidine. In one embodiment, the oligonucleotide comprises 5-methyl-2'-deoxycytidine having a LNA modification.

The oligonucleotides of the invention may comprise various combinations of unmodified and modified nucleobases. In some embodiments, a nucleobase is a natural nucleobase, or a modified nucleobase derived from a natural nucleobase. In some embodiments, the present disclosure provides oligonucleotides comprising one or more modified nucleobases. In some embodiments, the oligonucleotide comprises one or more modified nucleobases and/or modified sugars. In some embodiments, the oligonucleotide comprises one or more modified nucleobases, one or more modified sugars and/or one or more modified internucleoside linkages.

In some embodiments, oligonucleotides are of suitable lengths and sequence complementarity to specifically hybridize with target nucleic acids. In one embodiment, an oligonucleotide is 100% complementary to the target RNA, *i.e.,* IncMeg3.

Oligonucleotides may have varying lengths. In one embodiment, the oligonucleotide has a length of at least 12 or 15 nucleotides. In some embodiments, the oligonucleotide has a length of 10-100, 12-80, 12-70, 12-60, or 12-50 nucleotides. In some embodiment, the oligonucleotide has a length of 15-60, 15-50, 12-40, 12-20, or 15 to 20 nucleotides. In embodiment, the oligonucleotide has a length of 10, 11, 12, 13 , 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides. In one preferred embodiment, an oligonucleotide has a length of 16 nucleotides. In one preferred embodiment, an oligonucleotide has a length of 17 nucleotides. Ranges and lengths intermediate to the above recited ranges and lengths are also contemplated to be part of the invention.

In one embodiment, the oligonucleotide is an inhibitor. In some embodiments, the oligonucleotide hybridizes to a target RNA sequence. In one embodiment, the target is IncMeg3. In one embodiment, the oligonucleotide is an inhibitor of

lncMeg3. In one embodiment, the oligonucleotide decreases or blocks activity of lncMeg3. In one embodiment, the lncMeg3is endogenous lncMeg3. In some embodiments, an oligonucleotide of the invention hybridizes to one or more variants of lncMeg3.

**Table A** identifies LNA/DNA chimeric oligonucleotides of the invention with a phosphorothioate backbone and their specific nucleobase modifications. *Length: 16*-17mer. These sequences are useful in the oligonucleotides, compositions, methods and uses described herein. LNA: locked nucleic acid; 5mdC: 5-methyl-2'-deoxycytidine; dG: deoxyguanosine; dT: deoxythymidine; dA: deoxyadenosine; *: phosphorothioate (PS) linkage.

**Table A. Exemplary lncMeg3 targeting oligonucleotide sequences and their modifications.** + = locked nucleic acids (LNA); dN = 2'-H (deoxyribose; DNA), wherein N = A, C, T, G (nucleotide); 5mdC = 5-methyl-2'-deoxycytidine; A, C, G, T = modified nucleotide building blocks; +C = LNA-modified cytidine; +G = LNA-modified guanosine; +T = LNA-modified thymidine; +A = LNA-modified adenosine; * = phosphorothioate (PS) linkage.

| Candidate ID | Candidate Sequence (5' to 3' direction) | SEQ ID NO. |
|---|---|---|
| ID 497-A | C G C dC dG dC dC dAdT dA dT dC dT C C C | **1** |
| ID 4647-B | C G G dA dC dA dA dA dA dC dT dG dG T T G | **2** |
| ID 14209-C | A A G dA dG dT dC dT dC dC dT dC dC dT T A A | **3** |
| ID 6396-D | G A T dT dA dG dC dC dC dT dG dT dG dT T C A | **4** |
| ID 19749-E | C G G dC dA dC dA dA dG dA dG dC dC dA A A G | **5** |
| ID 14204-F | T C T dC dC dT dC dC dT dT dA dA dG dC C C C | **6** |
| ID 14205-G | G T C dT dC dC dT dC dC dT dT dA dA dG C C C | **7** |
| | | |
| ID 497-A1 | C G C (5mdC) dG (5mdC)(5mdC) dA dT dA dT (5mdC) dT C C C | **8** |
| ID 4647-B1 | C G G dA (5mdC) dA dA dA dA (5mdC) dT dG dG T T G | **9** |
| ID 14209-C1 | A A G dA dG dT (5mdC) dT(5mdC)(5mdC) dT (5mdC)(5mdC) dT T A A | **10** |
| ID 6396-D1 | G A T dT dA dG (5mdC)(5mdC)(5mdC) dT dG dT dG dT T C A | **11** |
| ID 19749-E1 | C G G (5mdC) dA (5mdC) dA dA dG dA dG (5mdC)(5mdC) dA A A G | **12** |
| ID 14204-F1 | T C T (5mdC)(5mdC) dT (5mdC)(5mdC) dT dT dA dA dG (5mdC) C C C | **13** |
| ID 14205-G1 | G T C dT (5mdC)(5mdC) dT (5mdC)(5mdC) dT dT dA dA dG C C C | **14** |
| | | |
| ID 497-A2 | +C +G +C(5mdC)dG(5mdC)(5mdC)dAdTdAdT(5mdC)dT+C+C+C | **15** |
| ID 4647-B2 | +C +G +G dA (5mdC) dA dA dA dA (5mdC) dT dG dG +T+T+G | **16** |
| ID 14209-C2 | +A +A+G dA dG dT (5mdC) dT (5mdC)(5mdC) dT (5mdC)(5mdC) dT +T +A +A | **17** |
| ID 6396-D2 | +G +A +T dT dA dG (5mdC)(5mdC)(5mdC) dT dG dT dG dT +T +C +A | **18** |
| ID 19749-E2 | +C +G +G (5mdC) dA (5mdC) dA dA dG dA dG (5mdC) (5mdC) dA +A +A +G | **19** |
| ID 14204-F2 | +T +C +T (5mdC)(5mdC) dT (5mdC)(5mdC) dT dT dA dA dG (5mdC) +C +C +C | **20** |
| ID 14205-G2 | +G +T +C dT (5mdC) (5mdC) dT (5mdC)(5mdC) dT dT dA dA dG +C +C +C | **21** |
| | | |
| ID 497 | +C*+G*+C*(5mdC)*dG*(5mdC)*(5mdC)*dA*dT*dA*dT*(5mdC)*dT*+C*+C*+C | **22** |
| ID 4647 | +C*+G*+G*dA*(5mdC)*dA*dA*dA*dA*(5mdC) *dT*dG*dG*+T*+T*+G | **23** |

(continued)

| Candidate ID | Candidate Sequence (5' to 3' direction) | SEQ ID NO. |
|---|---|---|
| ID 14209 | +A*+A*+G*dA*dG*dT*(5mdC)*dT*(5mdC)*(5mdC)*dT*(5mdC)*(5mdC) *dT*+T*+A*+A | **24** |
| ID 6396 | +G*+A*+T*dT*dA*dG*(5mdC)*(5mdC)*(5mdC)*dT*dG*dT*dG*dT*+T*+C* +A | **25** |
| ID 19749 | +C*+G*+G*(5mdC)*dA*(5mdC)*dA*dA*dG*dA*dG*(5mdC)*(5mdC)*dA*+ A* +A*+G | **26** |
| ID 14204 | +T*+C*+T*(5mdC)*(5mdC)*dT*(5mdC)*(5mdC)*dT*dT*dA*dA*dG*(5mdC )* +C*+C*+C | **27** |
| ID 14205 | +G*+T*+C*dT*(5mdC)*(5mdC)*dT *(5mdC)*(5mdC)*dT*dT*dA* dA*dG* +C*+C*+C | **28** |

The sequences disclosed herein are also shown in the enclosed sequence listing. However, the sequence listing shows only the sequence of nucleotides, whereas the modification of the nucleotides and of the bonds between the nucleotides is not shown in the sequence listing. The relevant modifications associated with the sequences are disclosed in **Table A** of this application.

According to the invention, an oligonucleotide comprises or consists of a sequence listed in **Table A.**

In one embodiment, the oligonucleotide comprises or consists of one of the following sequences in the 5' to 3' direction selected form the group consisting of:

(i) C G C dC dG dC dC dA dT dA dT dC dT C C C (SEQ ID NO: 1);
(ii) C G G dA dC dA dA dA dA dC dT dG dG T T G (SEQ ID NO: 2);
(iii) A A G dA dG dT dC dT dC dC dT dC dC dT T A A (SEQ ID NO: 3);
(iv) G A T dT dA dG dC dC dC dT dG dT dG dT T C A (SEQ ID NO: 4);
(v) C G G dC dA dC dA dA dG dA dG dC dC dA A A G (SEQ ID NO: 5);
(vi) T C T dC dC dT dC dC dT dT dA dA dG dC C C C (SEQ ID NO: 6); and
(vii) G T C dT dC dC dT dC dC dT dT dA dA dG C C C (SEQ ID NO: 7); wherein dA, dT, dG and dC are deoxyribonucleotide building blocks, or wherein dC is 5-methyl-2'-deoxycytidine; and wherein A, T, G and C are modified or unmodified nucleotide building blocks.

In one embodiment, an antisense oligonucleotide of the invention comprises or consists of one of the following sequences in the 5' to 3' direction selected form the group consisting of:

(i) C G C (5mdC) dG (5mdC) (5mdC) dA dT dA dT (5mdC) dT C C C (SEQ ID NO: 8);
(ii) C G G dA (5mdC) dA dA dA dA (5mdC) dT dG dG T T G (SEQ ID NO: 9);
(iii) A A G dA dG dT (5mdC) dT (5mdC) (5mdC) dT (5mdC) (5mdC) dT T A A (SEQ ID NO: 10);
(iv) G A T dT dA dG (5mdC) (5mdC) (5mdC) dT dG dT dG dT T C A (SEQ ID NO: 11);
(v) C G G (5mdC) dA (5mdC) dA dA dG dA dG (5mdC) (5mdC) dA A A G (SEQ ID NO: 12);
(vi) T C T (5mdC) (5mdC) dT (5mdC) (5mdC) dT dT dA dA dG (5mdC) C C C (SEQ ID NO: 13); and
(vii) G T C dT (5mdC) (5mdC) dT (5mdC) (5mdC) dT dT dA dA dG C C C (SEQ ID NO: 14);

wherein dA, dT, and dG are deoxyribonucleotide building blocks; wherein 5mdC is 5-methyl-2'-deoxycytidine; and wherein A, T, G and C are modified or unmodified nucleotide building blocks.

In one embodiment, an oligonucleotide comprises the sequence 5' - C G C (5mdC) dG (5mdC) (5mdC) dA dT dA dT (5mdC) dT C C C-3'(SEQ ID NO: 8), wherein dA, dT, and dG are deoxyribonucleotide building blocks; wherein 5mdC is 5-methyl-2'-deoxycytidine; and wherein A, T, G and C are modified or unmodified nucleotide building blocks.

In one embodiment, an oligonucleotide comprises 5' - C G G dA (5mdC) dA dA dA dA (5mdC) dT dG dG T T G - 3' (SEQ ID NO: 9), wherein dA, dT, and dG are deoxyribonucleotide building blocks; wherein 5mdC is 5-methyl-2'-deoxycytidine; and wherein A, T, G and C are modified or unmodified nucleotide building blocks.

In one embodiment, an oligonucleotide comprises the sequence 5' - A A G dA dG dT (5mdC) dT (5mdC) (5mdC) dT (5mdC) (5mdC) dT T A A - 3' (SEQ ID NO: 10), wherein dA, dT, and dG are deoxyribonucleotide building blocks; wherein

5mdC is 5-methyl-2'-deoxycytidine; and wherein A, T, G and C are modified or unmodified nucleotide building blocks.

In one embodiment, an oligonucleotide comprises the sequence 5' - G A T dT dA dG (5mdC) (5mdC) (5mdC) dT dG dT dG dT T C A - 3' (SEQ ID NO: 11), wherein dA, dT, and dG are deoxyribonucleotide building blocks; wherein 5mdC is 5-methyl-2'-deoxycytidine; and wherein A, T, G and C are modified nucleotide building blocks.

In one embodiment, an oligonucleotide comprises the sequence 5' - C G G (5mdC) dA (5mdC) dA dA dG dA dG (5mdC) (5mdC) dA A A G - 3' (SEQ ID NO: 12), wherein dA, dT, and dG are deoxyribonucleotide building blocks; wherein 5mdC is 5-methyl-2'-deoxycytidine; and wherein A, T, G and C are modified or unmodified nucleotide building blocks.

In one embodiment, an oligonucleotide comprises the sequence 5' - T C T (5mdC) (5mdC) dT (5mdC) (5mdC) dT dT dA dA dG (5mdC) C C C - 3' (SEQ ID NO: 13), wherein dA, dT, and dG are deoxyribonucleotide building blocks; wherein 5mdC is 5-methyl-2'-deoxycytidine; and wherein A, T, G and C are modified or unmodified nucleotide building blocks.

In one embodiment, an oligonucleotide comprises the sequence 5' - G T C dT (5mdC) (5mdC) dT (5mdC) (5mdC) dT dT dA dA dG C C C - 3' (SEQ ID NO: 14), wherein dA, dT, and dG are deoxyribonucleotide building blocks; wherein 5mdC is 5-methyl-2'-deoxycytidine; and wherein A, T, G and C are modified or unmodified nucleotide building blocks.

In one embodiment, an antisense oligonucleotide of the invention comprises or consists of one of the following sequences in the 5' to 3' direction selected form the group consisting of:

(i) +C +G +C (5mdC) dG (5mdC) (5mdC) dA dT dA dT (5mdC) dT +C +C +C (SEQ ID NO: 15);
(ii) +C +G +G dA (5mdC) dA dA dA dA (5mdC) dT dG dG +T +T +G (SEQ ID NO: 16);
(iii) +A +A +G dA dG dT (5mdC) dT (5mdC) (5mdC) dT (5mdC) (5mdC) dT +T +A +A (SEQ ID NO: 17);
(iv) +G +A +T dT dA dG (5mdC) (5mdC) (5mdC) dT dG dT dG dT +T +C +A (SEQ ID NO: 18);
(v) +C +G +G (5mdC) dA (5mdC) dA dA dG dA dG (5mdC) (5mdC) dA +A +A +G (SEQ ID NO: 19);
(vi) +T +C +T (5mdC) (5mdC) dT (5mdC) (5mdC) dT dT dA dA dG (5mdC) +C +C +C (SEQ ID NO: 20); and
(vii) +G +T +C dT (5mdC) (5mdC) dT (5mdC) (5mdC) dT dT dA dA dG +C +C +C (SEQ ID NO: 21);

wherein +G, +T, +A, and +C are locked nucleic acid (LNA) building blocks; wherein dA, dT, and dG are deoxyribonucleotide building blocks; and wherein 5mdC is 5-methyl-2'-deoxycytidine.

In one embodiment, the oligonucleotide comprises or consists of one of the following sequences in the 5' to 3' direction:

(i) +C*+G*+C*(5mdC)*dG*(5mdC)*(5mdC)*dA*dT*dA*dT*(5mdC)*dT*+C*+C*+C (SEQ ID NO: 22);
(ii) +C*+G*+G*dA*(5mdC)*dA*dA*dA*dA*(5mdC) *dT*dG*dG*+T*+T*+G (SEQ ID NO: 23);
(iii) +A*+A*+G*dA*dG*dT*(5mdC)*dT*(5mdC)*(5mdC)*dT*(5mdC)*(5mdC) *dT* +T* +A *+A (SEQ ID NO: 24);
(iv) +G*+A*+T*dT*dA*dG*(5mdC)*(5mdC)*(5mdC)*dT*dG*dT*dG*dT*+T*+C*+A (SEQ ID NO: 25);
(v) +C*+G*+G*(5mdC)*dA*(5mdC)*dA*dA*dG*dA*dG*(5mdC)*(5mdC)*dA*+A* +A*+G (SEQ ID NO: 26);
(vi) +T*+C*+T*(5mdC)*(5mdC)*dT*(5mdC)*(5mdC)*dT*dT*dA*dA* dG* (5mdC)* +C* +C*+C (SEQ ID NO: 27);
(vii) +G*+T*+C*dT* (5mdC)*(5mdC)*dT*(5mdC)* (5mdC)*dT* dT* dA* dA* dG* +C* +C* +C (SEQ ID NO: 28); wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a phosphorothioate linkage.

In one embodiment, an oligonucleotide comprises the sequence 5' - +C*+G*+C*(5mdC)*dG*(5mdC)*(5mdC) *dA*dT*dA*dT*(5mdC)*dT*+C*+C*+C - 3' (SEQ ID NO: 22), wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a phosphorothioate linkage.

In one embodiment, an oligonucleotide comprises the sequence 5' - +C*+G*+G*dA*(5mdC)*dA*dA*dA*dA* (5mdC) *dT*dG*dG*+T*+T*+G - 3' (SEQ ID NO: 23), wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a phosphorothioate linkage.

In one embodiment, an oligonucleotide comprises the sequence 5' - +A*+A*+G*dA*dG*dT*(5mdC)*dT*(5mdC)* (5mdC)*dT*(5mdC)*(5mdC) *dT*+T*+A*+A - 3' (SEQ ID NO: 24), wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a phosphorothioate linkage.

In one embodiment, an oligonucleotide comprises the sequence 5' - +G*+A*+T*dT*dA*dG*(5mdC)*(5mdC)* (5mdC)*dT*dG*dT*dG*dT*+T*+C*+A - 3' (SEQ ID NO: 25), wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a phosphorothioate linkage.

In one embodiment, an oligonucleotide comprises the sequence 5' - +C*+G*+G*(5mdC)*dA*(5mdC)*dA*-dA*dG*dA*dG*(5mdC)*(5mdC)*dA*+A* +A*+G - 3' (SEQ ID NO: 26), wherein dA, dT, and dG are deoxyribonucleotide building blocks; wherein 5mdC is 5-methyl-2'-deoxycytidine; wherein +G, +T, +A, and +C are locked nucleic acid (LNA)

building blocks; and wherein * is a phosphorothioate linkage.

In one embodiment, an oligonucleotide comprises the sequence 5' - +T*+C*+T*(5mdC)*(5mdC)*dT*(5mdC)*(5mdC)*dT*dT*dA*dA*dG*(5mdC) *+C*+C*+C - 3' (SEQ ID NO: 27), wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a phosphorothioate linkage.

In one embodiment, an oligonucleotide comprises the sequence 5' - +G*+T*+C*dT* (5mdC)*(5mdC)*dT*(5mdC)*(5mdC)*dT*dT*dA*dA*dG*+C*+C*+C*+C - 3' (SEQ ID NO: 28), wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a phosphorothioate linkage.

A number of heterologous moieties or carriers, ranging from small molecules to macromolecules and supramolecular assemblies, may be attached to the oligonucleotides of the invention. Specifically, the oligonucleotides of the invention may be modified at their 3' and and/or 5' end to enhance cellular update and improve target organ delivery. In one embodiment, the oligonucleotide is conjugated to a heterologous moiety. In one embodiment, the oligonucleotide is conjugated to one or more heterologous moieties. In one embodiment, the oligonucleotide is linked to the heterologous moiety via a covalent bond. In one embodiment, conjugation of the oligonucleotide to a heterologous moiety (or carrier) promotes cellular penetration and/or uptake. The heterologous moiety can be a carrier. Specifically, in one embodiment, the carrier is a lipid or a polymer. In some embodiments, the carrier is a cell-penetrating peptide (CPP). In one embodiment, the carrier is cholesterol. In one embodiment, a marrier is a nanoparticle. In one embodiment, the oligonucleotide comprises a covalent attachment of poly(ethylene glycol) (PEG). In some cases, N-acetylgalactosamine (GalNac) may be used as a delivery moiety for oligonucleotides. In one embodiment, the oligonucleotide comprises a GalNAc modification.

In one embodiment, the oligonucleotide is to be administered as such, or the oligonucleotide is to be administered conjugated to a heterologous moiety.

In one embodiment, one or more oligonucleotides of the invention or a combination thereof is used in the treatment of an inflammatory disease, a cardiopulmonary disorder and/or a fibrotic disorder or combination thereof in a subject.

The nucleic acids, oligonucleotides or antisense oligonucleotides (ASOs) provided herein may be incorporated into compositions of the invention.

Provided herein is a composition (*e.g.*, oligonucleotide composition, pharmaceutical composition) containing the oligonucleotide of the invention. The present disclosure provides oligonucleotide compositions of antisense oligonucleotides described herein. In some embodiments, the compositions are pharmaceutical compositions. As used herein, pharmaceutical composition means a mixture of substances suitable for administering to an individual. For example, a pharmaceutical composition may comprise one or more active pharmaceutical agents (such as an antisense oligonucleotide) and a sterile aqueous solution. In one embodiment, the composition contains one or more oligonucleotides of the invention. In one embodiment, provided herein is a pharmaceutical composition or a pharmaceutically acceptable salt thereof comprising an antisense oligonucleotide of the invention, or a vector of the invention. In some embodiments, a composition comprises a plurality of oligonucleotides sharing a common base sequence. In some embodiments, a plurality of oligonucleotides shares the same base sequence, and the same base and sugar modification. In some embodiments, a plurality of oligonucleotides shares the same base sequence, and the same base, sugar and internucleoside linkage modification. In one embodiment, the composition contains one or more oligonucleotides of the invention.

In one embodiment, a composition comprises an oligonucleotide of the invention in an admixture with a pharmaceutically acceptable carrier. Hence, in one embodiment, a pharmaceutical composition comprises an antisense oligonucleotide of the invention and a pharmaceutically acceptable carrier. In some embodiments, the pharmaceutically acceptable carrier can simply be a saline solution. This can be isotonic or hypotonic. In one embodiment, a (pharmaceutical) composition comprises an oligonucleotide of the invention, optionally in an admixture with a pharmaceutically acceptable carrier. In one embodiment, the composition comprises at least one of the oligonucleotides listed in **Table A.** In one embodiment, the composition comprises one or more oligonucleotides having a sequence selected from SEQ ID NO: 1-28. Preferably, in one embodiment, the composition comprises an oligonucleotide selected from SEQ ID NOs: 22-28.

In some embodiments, a composition is characterized in that when it is contacted with a target nucleic acid, levels of the target nucleic acid and/or a product encoded thereby is reduced. In some embodiment, a composition of the invention inhibits the target nucleic acid. According to the invention, a composition of the invention targets IncMeg3.

A composition comprising an oligonucleotide of the invention may be administered as a monotherapy or in combination with one or more further (different) medicaments, particularly a medicament suitable for the prevention or treatment of cardiac inflammation or fibrotic disorders, *e.g.*, cardiac fibrosis. In some embodiments, a pharmaceutical composition comprises one or more other therapies in addition to an oligonucleotide of the invention. In certain embodiments, an oligonucleotide or composition is administered to a subject once as a single dose. In some embodiments, an oligonucleotide or composition is administered to a subject in combination with one or more therapies. The one or more other therapies may be beneficial in the treatment or prevention of inflammatory diseases and/or fibrotic disorders or may ameliorate a symptom or condition associated with the same.

For instance, further heart failure drugs with antifibrotic (cardiac) effects include, for example, Angiotensin-converting enzyme (ACE) inhibitors, Angiotensin II receptor blockers (ARB), Angiotensin Receptor-Neprilysin Inhibitor (ARNI), Aldosterone receptor antagonists (MRA), Beta-blockers and sodium-glucose cotransporter 2 (SGLT2) inhibitors. Specifically, ACE inhibitors may include Captopril, Enalapril, Benazepril, Ramipril, Fosinopril, Trandolapril, Perindopril. ARB inhibitors may include, for example, Losartan, Candesartan and Valsartan. An ARNI may be Sacubitril/Valsartan (*e.g.,* 1:1 ratio). MRAs may include Spironolactone, Eplerenone and Finerenone. Beta-blockers may include Bisoprolol, Carvedilol, and Metoprolol. SGLT2 inhibitors (SGLT2i) may include Empagliflozin, Dapagliflozin, Sotagliflozin, and Canagliflozin.

In other cases, further drugs may include antifibrotic drugs (*e.g.*, pulmonary fibrosis (IPF)) such as TK inhibitors (*e.g.*, Nintedanib and Imatinib), Antifibrotic drugs (*e.g.*, Pirfenidone), Endothelin R (*e.g.*, Macitentan, Bosentan, Ambrisentan), TNF inhibitors (*e.g.,* Etanercept), immunomodulators (*e.g.,* Cyclophosphamide), and antiinflammatory drugs (*e.g.*, Colchicine).

Other drugs with antifibrotic effects may include Galectin-3 inhibitors, NLRP3 protein inhibitors, TGF-beta Inhibitors and CTGF Inhibitors.

According to a further aspect, the invention relates to a kit or kit of parts comprising an oligonucleotide of the invention and/or the (pharmaceutical) composition according to the invention. That is, the pharmaceutical compositions described herein can be included in a container, pack, or dispenser together with instructions for administration. In one embodiment, the kit comprises an oligonucleotide or composition of the invention. In one embodiment, the kit additionally comprises instructions for use.

Prophylactic and Therapeutic Uses

The invention generally describes the use of the chemically modified oligonucleotide and/or composition comprising the same in the medical setting. Hence, a chemically modified oligonucleotide or composition comprising the same may be used in the treatment and/or prevention of a medical condition. In one aspect provided herein is an oligonucleotide or a composition of the invention for therapeutic use in a subject.

In another aspect provided herein is an antisense oligonucleotide, a vector, or a composition for prophylactic or therapeutic use in a subject. In one embodiment, the antisense oligonucleotide, the vector, or the composition is for use in the prevention of treatment of a cardiac disease in a subject.

In one embodiment, the cardiac disease is selected from a group consisting of (acute or subacute) heart failure, chronic and/or worsening chronic heart failure, stable heart failure, a less advanced state of heart failure or an advanced state of heart failure, heart failure of NYHA stage I, II, III and/or IV, myocardial infarction, myocarditis or cardiac fibrosis.

In one embodiment, the cardiac disease is an inflammatory cardiac disease. In one particular embodiment, the inflammatory disease is cardiac inflammation. In one embodiment, the disease is endocarditis. In one embodiment, the disease is myocarditis. In one embodiment, the disease is pericarditis. Cardiac inflammation may be caused by various factors. In one embodiment, cardiac inflammation is caused by viral, bacterial, fungal, and/or parasitic infections. In one embodiment, cardiac inflammation is caused by an autoimmune disease, *e.g.*, rheumatoid arthritis or lupus. In one embodiment, cardiac inflammation is caused by medicines, such as, e.g., heart medicines, antibiotics, antidepressants, diuretics, benzodiazepines, weight loss medicines *etc.*.

Also described herein is an oligonucleotide or a composition of for use in the prophylaxis and/or treatment of fibrosis or fibrotic disorders in a subject. For instance, exemplary types of cardiac fibrosis may include atrial fibrosis and/or endomyocardial fibrosis. Specifically, in one embodiment, the fibrotic disorder is cardiac fibrosis. In one embodiment, the cardiac fibrotic disorder includes atrial fibrosis, endomyocardial fibrosis or fibrosis resulting from a previous myocardial infarction. In one embodiment, cardiac fibrosis include left and/or right ventricular fibrosis. In one embodiment, fibrosis results from a previous myocardial infarction (MI), high blood pressure or myocarditis. In one embodiment, the fibrosis is a cardiac fibrotic disorder. In one embodiment, fibrosis is myocardial fibrosis. In some embodiments, myocardial fibrosis is interstitial fibrosis, subepicardial fibrosis or replacement fibrosis. In some embodiments, fibrosis is reactive interstitial fibrosis. In other embodiments, cardiac fibrosis results from hypertensive heart disease, diabetic hypertrophic cardiomyopathy and/or idiopathic dilated cardiomyopathy or hypertrophic cardiomyopathy. In some embodiments, myocardial fibrosis is associated with myocardial infarction (heart attack).

In some embodiments, the subject suffers from cardiac fibrosis and a further disease. For instance, this further disease may be another type of fibrosis.

The oligonucleotide or composition comprising the same of the invention may cause a decrease in IncRNA Meg3 expression. Hence, in one embodiment, there is a decrease in IncRNA Meg3 expression. In one embodiment, there is a decrease in the expression of fibrosis-related genes MMP2, COL1A1 and/or COL3A1. In one embodiment, there is a decrease in IncRNA Meg3 expression and a decrease in fibrosis-related genes, e.g., MMP2, COL1A1 and/or COL3A1.

The oligonucleotide or the pharmaceutical composition of the invention may be administered to a subject in need thereof. In one embodiment, a subject has an increased risk for developing an inflammatory disease. In one embodiment, a

subject comprises patients suffering from inflammatory disease and/or patients having an increased risk of inflammatory disease progression. In one embodiment, patients have an increased risk for developing one or more cardiac disorders. In one embodiment, patients suffer from cardiac disorders and/or patients having an increased risk of cardiac disorder progression. In one embodiment, patients have an increased risk for developing fibrotic disorders. In one embodiment, patients suffer from fibrotic disorders and/or patients having an increased risk of fibrotic disorder progression. In a particular embodiment, the oligonucleotide or the pharmaceutical composition is administered to the subject having an increased risk for developing cardiac disorders. In one embodiment, the patient suffers from cardiac disorders and/or has an increased risk of cardiac disorder progression. In one embodiment, the patient has an increased risk for developing fibrotic disorders, *e.g.*, cardiac fibrosis. In one embodiment, the patient suffers from fibrotic disorders and/or has an increased risk of fibrotic disorder progression.

In some embodiments, fibrosis is associated with one or more fibrotic marker genes. In one embodiment, the marker genes are MMP2, COL1A1, and/or COL3A1.

The oligonucleotide or the pharmaceutical composition of the invention may be administered to a subject in need thereof. In one embodiment, the oligonucleotide or the pharmaceutical composition is administered to a subject suffering from cardiac disorders and/or patients having an increased risk of cardiac disorder progression; patients having an increased risk for developing fibrotic disorders; and/or patients suffering from fibrotic disorders and/or patients having an increased risk of fibrotic disorder progression.

In certain embodiments, an oligonucleotide or composition is administered to a subject, *i.e.,* a subject that does not have a disease or disorder. In one embodiment, an oligonucleotide or composition comprising the same is administered to a subject that is at risk of developing a disease or disorder. In certain embodiments, an oligonucleotide or a composition provided herein is administered to a subject or patient who has been diagnosed with a disease or disorder. In one embodiment, an oligonucleotide or a composition provided herein is administered to a subject who has been diagnosed with a method according to the invention.

In some embodiments, an oligonucleotide or composition containing an oligonucleotide described herein is administered to a subject before symptoms manifest or symptoms become severe. In one embodiment, the subject is a patient. In some embodiments, a subject to be administered an oligonucleotide or composition is an animal, preferably a mammal. In specific embodiments, a subject to be administered an oligonucleotide or composition is human. In certain embodiments, a subject is a human adult.

In some embodiments, the subject (*e.g.*, a human) to be administered an oligonucleotide or composition containing an oligonucleotide is any individual at risk of fibrotic diseases. In one embodiment, the patient suffers from or is at risk of developing myocardial fibrosis. In one embodiment, the patient suffers from or has an increased risk for developing heart failure.

In some embodiments, the subject (*e.g.*, a human) to be administered an oligonucleotide or composition containing an oligonucleotide of the invention is an individual affected by any condition that increases susceptibility to cardiac inflammation, cardiomyocyte hypertrophy, cardiomyocyte apoptosis, and/or myocardial fibrosis.

Provided herein is a use of an oligonucleotide of the invention in therapy. Also, provided herein is a use of an oligonucleotide of the invention in the manufacture of a medicament for treating a cardiac fibrosis. Also provided herein is a use of an oligonucleotide of the invention in the manufacture of a medicament for treating cardiac fibrotic disorders.

Also provided herein is a method of treating a subject suffering from a cardiac disease. wherein the method comprises administering to the subject in need thereof an effective amount of the antisense oligonucleotide of the invention or composition of the invention.

Also provided herein is a method of preventing a cardiac disease in a subject, wherein the method comprises administering to the subject in need thereof an effective amount of the antisense oligonucleotide of the invention or composition of the invention.

Further provided herein is a method of preventing and/or treating an inflammatory disease in a subject. In one embodiment, the inflammatory disease is cardiac inflammation.

Also described herein is a method of preventing and/or treating fibrotic disorders in a subject. In one embodiment, the fibrotic disorder is cardiac fibrotic disorder. In one embodiment, the fibrotic disorder is left and/or right ventricular fibrosis, atrial fibrosis, endomyocardial fibrosis or fibrosis resulting from a previous myocardial infarction, high blood pressure or myocarditis.

The pharmaceutical compositions provided herein can be in any form that allows for the composition to be administered to a subject. The chemically modified oligonucleotide of the invention or the (pharmaceutical) composition may be administered, for example, orally in any orally acceptable dosage form including, but not limited to, capsules, tablets, aqueous suspensions, or solutions, or parenterally, e.g., by parenteral injection. In some embodiments, formulations suitable for parenteral administration comprise sterile aqueous preparations of at least one embodiment of the present disclosure, which are approximately isotonic with the blood of the intended recipient. In one embodiment, the oligonucleotide is administered by intravenous (i.v.) injection. In one embodiment, the oligonucleotide is administered by subcutaneous injection (s.c.). The amount of oligonucleotide or composition to be administered, the dosage and the

dosing regimen can vary from cell type to cell type, the disease to be treated, the target population, the mode of administration (*e.g.*, systemic versus local), the severity of disease and the acceptable level of side activity. In some embodiments, the amount of oligonucleotides administered in a pharmaceutical composition is dependent on the subject being treated, the subject's weight, the manner of administration, and/or disease to be treated. In one embodiment, the oligonucleotide is administered at a concentration between 0.05 to 20 mg/kg. In one embodiment, the oligonucleotide is administered at a concentration between 0.1 to 20 mg/kg.

The oligonucleotide of the invention may be delivered using a vector.

An oligonucleotide of the invention may be delivered as is (*i.e.,* naked and/or in isolated form). Hence, in one embodiment, the oligonucleotides of the present invention are administered and delivered 'as is', also referred to as 'naked'. In one embodiment, the oligonucleotide is to be administered as such. In one embodiment, the oligonucleotide is delivered in NaCl.

The key problem for oligonucleotide-based therapeutics is to efficiently deliver the oligonucleotide to the site of action. Hence, oligonucleotides of the invention may be modified at their 3'- and/or 5'- ends to promote cellular uptake and/or tissue delivery. In one embodiment, the oligonucleotide is conjugated to a heterologous moiety. In one embodiment, the oligonucleotide is linked to one or more heterologous moieties. In one embodiment, the oligonucleotide is to be administered as such, or the oligonucleotide is to be administered conjugated to a heterologous moiety. In one embodiment, the oligonucleotide is linked to the heterologous moiety via a covalent bond. In one embodiment, conjugation of the oligonucleotide to a heterologous moiety (or carrier) promotes cellular penetration and/or uptake. The heterologous moiety can be a carrier. Specifically, in one embodiment, the carrier is a lipid or a polymer. In some embodiments, the carrier is a cell-penetrating peptide (CPP). In one embodiment, the carrier is cholesterol. In one embodiment, a marrier is a nanoparticle. In one embodiment, the oligonucleotide comprises a covalent attachment of poly(ethylene glycol) (PEG).

The art contains multiple ways of delivering oligonucleotides to cells, tissues or organs, either *in vitro, ex vivo* or *in vivo.* For instance, polycationic polymers, nanoparticles, microparticles or liposomal formulations that may be used for *in vivo* oligonucleotide delivery are well known in the art. In one embodiment, polycationic polymers are used for delivery of the oligonucleotide. In one embodiment, microparticles are used for delivery of the oligonucleotide. In one embodiment, nanoparticles are used for delivery of the oligonucleotide. In some embodiments, oligonucleotides are delivered using lipid nanoparticles (LNPs). In one embodiment, liposomal formulations are used to deliver the oligonucleotides of the invention.

Combination therapy has become critical in developing prevention and treatment strategies across many medical disciplines, as drug combinations have the potential to improve treatment response, minimize development of resistance, allow lower doses of component therapies, or reduce adverse events. The oligonucleotide or composition of the invention may be administered as a monotherapy or in combination with a further different medicament. In some instances, an oligonucleotide or composition of the invention is administered in combination with other oligonucleotides. In one embodiment, the oligonucleotide or composition of the invention is administered in combination with a further different medicament, particularly a medicament suitable for the treatment or prevention of any of the diseases mentioned above. Hence, provided herein is an oligonucleotide or a pharmaceutical composition for use of the invention, wherein the oligonucleotide or the pharmaceutical composition is administered to a subject in combination with one or more other therapies. In one embodiment, the one or more other therapies comprises a standard of care (SoC).

Examples of further medicaments suitable for the prevention or treatment of cardiac disorders are angiotensin-modulating agents, beta-blockers, diuretics, aldosterone antagonists, vasodilators, ionotrophic agents, statins, neprilysin-inhibitors, or SGLT-2 inhibitors or combinations thereof, *e.g.*, a combination of a neprilysininhibitor, *e.g.*, sacubitril, with an angiotensin-II-receptor blocker, *e.g.* valsartan.

Specifically, in some embodiments, the oligonucleotide or composition of the invention is used in combination therapy with another medicament for treating or preventing myocardial fibrosis.

Methods and Uses

A chemically modified oligonucleotide of the invention or a (pharmaceutical) composition may be used in the diagnosis of a genetic condition, disease, or disorder. Hence, also provided herein is an oligonucleotide for use in the diagnosis of a fibrotic disease.

Also provided herein is a method of diagnosing a fibrotic disorder, wherein the method comprises an oligonucleotide of the invention. In one embodiment, the method comprises one or more oligonucleotides of the invention.

Also provided herein is the use of an oligonucleotide of the invention for the diagnosis of a fibrotic disorder. In one embodiment, an oligonucleotide is for the diagnosis of a cardiac fibrotic disorder.

Also provided herein is a method of delivering an oligonucleotide of the invention or a composition of the invention to cardiac cells, comprising contacting the cardiac cells with an oligonucleotide or a composition of the invention.

Also provided herein is a method for determining treatment efficacy in a patient using an oligonucleotide of the invention or a composition the invention, the method comprising: (a) detecting the expression level of Meg3 in a sample previously obtained from a patient, and (b) comparing the expression level obtained in (a) with the expression level of Meg3

in a sample previously obtained from at least one healthy subject or with a predetermined standard that has been obtained from a sample of at least one healthy subject, wherein a 2-fold downregulation of Meg3 is indicative for a prophylactic or therapeutic response in the patient. In one embodiment, there is a 25% downregulation in Meg3 expression. In one embodiment, there is a 30% downregulation in Meg3 expression. In one embodiment, there is at least a 30% downregulation in Meg3 expression. In one embodiment, there is a 35% downregulation in Meg3 expression. Also provided herein is a use of the antisense oligonucleotide according to the invention for the treatment and/or prevention of a cardiac disease. In one embodiment, the oligonucleotide is administered at a concentration between 0.05 to 20 mg/kg. In one embodiment, the oligonucleotide is administered at a concentration between 0.1 to 20 mg/kg.

Also provided herein is a kit comprising means for the detection of the expression level of Meg3, and instructions how to use the kit. The kit may contain various components that are used for the detection of Meg3 expression. In one embodiment, the kit comprises specific primers for the detection of Meg3 RNA levels by means of qPCR. In one embodiment, the kit comprises specific Taqman probe for the detection of Meg3 RNA levels by means of qPCR. In one embodiment, the kit comprises an oligonucleotide of the invention.

## LIST OF FURTHER EMBODIMENTS

The invention is further described by the following non-limiting embodiments:

Provided herein is an antisense oligonucleotide for targeting long non-coding RNA maternally expressed gene 3 (IncRNA Meg3), wherein the oligonucleotide comprises or consists of one of the following sequences in the 5' to 3' direction: (i) C G C dC dG dC dC dA dT dA dT dC dT C C C (SEQ ID NO: 1); (ii) C G G dA dC dA dA dA dA dC dT dG dG T T G (SEQ ID NO: 2); (iii) A A G dA dG dT dC dT dC dC dT dC dC dT T A A (SEQ ID NO: 3); (iv) G A T dT dA dG dC dC dC dT dG dT dG dT T C A (SEQ ID NO: 4); (v) C G G dC dA dC dA dA dG dA dG dC dC dA A A G (SEQ ID NO: 5); (vi) T C T dC dC dT dC dC dT dT dA dA dG dC C C C (SEQ ID NO: 6); or (vii) G T C dT dC dC dT dC dC dT dT dA dA dG C C C (SEQ ID NO: 7); wherein dA, dT, dG and dC are deoxyribonucleotides, or wherein dC is 5-methyl-2'-deoxycytidine; and wherein A, T, G and C are modified or unmodified nucleotide building blocks.

In one embodiment, the antisense oligonucleotide comprises or consists of one of the following sequences in the 5' to 3' direction: (i) C G C (5mdC) dG (5mdC)(5mdC) dA dT dA dT (5mdC) dT CCC (SEQ ID NO: 8); (ii) C G G dA (5mdC) dA dA dA dA (5mdC) dT dG dG TTG (SEQ ID NO: 9); (iii) AAG dA dG dT (5mdC) dT(5mdC)(5mdC) dT (5mdC)(5mdC) dT TAA (SEQ ID NO: 10); (iv) GAT dT dA dG (5mdC)(5mdC)(5mdC) dT dG dT dG dT TCA (SEQ ID NO: 11); (v) CGG (5mdC) dA (5mdC) dA dA dG dA dG (5mdC) (5mdC) dA AAG (SEQ ID NO: 12); (vi) TCT (5mdC) (5mdC) dT (5mdC) (5mdC) dT dT dA dA dG (5mdC) CCC (SEQ ID NO: 13); or (vii) GTC dT (5mdC) (5mdC) dT (5mdC) (5mdC) dT dT dA dA dG CCC (SEQ ID NO: 14); wherein dA, dT, and dG are deoxyribonucleotides; wherein 5mdC is 5-methyl-2'-deoxycytidine; and wherein A, T, G and C are modified or unmodified nucleotide building blocks.

In one embodiment, the oligonucleotide comprises bridged nucleic acid (BNA) building blocks.

In one embodiment, the BNA building blocks are locked nucleic acid (LNA) building blocks.

In one embodiment, the LNA building blocks are located at the 3' and/or 5' terminal end of the oligonucleotide.

In one embodiment, each end comprises no more than 3 LNA building blocks.

In one embodiment, each end contains 3 LNA building blocks.

In one embodiment, the oligonucleotide comprises one of the following sequences in the 5' to 3' direction: (i) +C +G+C(5mdC)dG(5mdC)(5mdC)dAdTdAdT(5mdC)dT+C+C+C (SEQ ID NO: 15); (ii) +C +G +G dA (5mdC) dA dA dA dA (5mdC) dT dG dG +T+T+G (SEQ ID NO: 16); (iii) +A +A+G dA dG dT (5mdC) dT (5mdC) (5mdC) dT (5mdC) (5mdC) dT +T +A +A (SEQ ID NO: 17); (iv) +G +A +T dT dA dG (5mdC)(5mdC)(5mdC) dT dG dT dG dT +T +C +A (SEQ ID NO: 18); (v) +C +G +G (5mdC) dA (5mdC) dA dA dG dA dG (5mdC) (5mdC) dA +A +A +G (SEQ ID NO: 19); (vi) +T +C +T (5mdC)(5mdC) dT (5mdC)(5mdC) dT dT dA dA dG (5mdC) +C +C +C (SEQ ID NO: 20); or (vii) +G +T +C dT (5mdC) (5mdC) dT (5mdC) (5mdC) dT dT dA dA dG +C +C +C (SEQ ID NO: 21); wherein +G, +T, +A, and +C are locked nucleic acid (LNA) building blocks; wherein dA, dT, and dG are deoxyribonucleotide building blocks; and wherein 5mdC is 5-methyl-2'-deoxycytidine.

In one embodiment, the oligonucleotide comprises one or more internucleoside linkage modifications.

In one embodiment, the one or more internucleoside linkage modification is a phosphorothioate (PS) linkage.

In one embodiment, all internucleoside linkages are PS linkage modifications.

In one embodiment, the oligonucleotide comprises or consists of one of the following sequences in the 5' to 3' direction: (i) +C*+G*+C*(5mdC)*dG*(5mdC)*(5mdC)*dA*dT*dA*dT*(5mdC)*dT*+C*+C*+C (SEQ ID NO: 22); (ii) +C*+G* +G*dA*(5mdC)*dA*dA*dA*dA*(5mdC) *dT*dG*dG*+T*+T*+G (SEQ ID NO: 23); (iii) +A*+A*+G*dA*dG*dT*(5mdC)*dT* (5mdC)*(5mdC)*dT*(5mdC)*(5mdC) *dT*+T* +A *+A (SEQ ID NO: 24); (iv) +G*+A*+T*dT*dA*dG*(5mdC)*(5mdC)* (5mdC)*dT*dG*dT*dG*dT*+T*+C*+A (SEQ ID NO: 25); (v) +C*+G*+G*(5mdC)*dA*(5mdC)*dA*dA*dG*dA*dG* (5mdC)*(5mdC)*dA*+A* +A*+G (SEQ ID NO: 26); (vi) +T*+C*+T*(5mdC)*(5mdC)*dT*(5mdC)*(5mdC)*dT*dT*dA*-dA*dG*(5mdC)*+C* +C*+C (SEQ ID NO: 27); (vii) +G*+T*+C*dT* (5mdC)*(5mdC)*dT*(5mdC)* (5mdC)*dT* dT* dA* dA* dG* +C* +C* +C (SEQ ID NO: 28); wherein dA, dT, and dG are deoxyribonucleotide building blocks; wherein 5mdC is 5-methyl-2'-deoxycytidine; wherein +G, +T, +A, and +C are locked nucleic acid (LNA) building blocks; and wherein * is a

phosphorothioate linkage.

In one embodiment, the oligonucleotide comprises 15-20 nucleotides.

In one embodiment, the oligonucleotide is 16 or 17 nucleotides long.

In one embodiment, the oligonucleotide is conjugated to one or more heterologous moieties.

In one embodiment, the heterologous moiety enhances cellular uptake of the oligonucleotide.

In one embodiment, the heterologous moiety is covalently bound to the oligonucleotide via a linker.

Also provided herein is a cell comprising an antisense oligonucleotide of the invention.

Also provided herein is a vector comprising an antisense oligonucleotide of the invention.

Also provided herein is a pharmaceutical composition comprising the antisense oligonucleotide of the invention.

In one embodiment, the pharmaceutical composition comprises a pharmaceutically acceptable carrier.

Provided herein is an antisense oligonucleotide of the invention, a vector of the invention, or a pharmaceutical composition of the invention, for prophylactic or therapeutic use in a subject.

Provided herein is an antisense oligonucleotide of the invention, a vector of the invention, or a pharmaceutical composition of the invention, for use in the prevention or treatment of a cardiac disease in a subject.

In one embodiment, the antisense oligonucleotide, the vector, or the pharmaceutical composition for use of the invention is administered by subcutaneous (s.c.) administration, intravenous (i.v.) administration, intramuscular administration, or oral administration.

In one embodiment, the cardiac disease is selected from a group consisting of (acute or subacute) heart failure, chronic and/or worsening chronic heart failure, stable heart failure, a less advanced state of heart failure or an advanced state of heart failure, heart failure of NYHA stage I, II, III and/or IV, myocardial infarction, myocarditis or cardiac fibrosis.

In one embodiment, the cardiac disease is acute or subacute heart failure.

In one embodiment, the cardiac disease is chronic and/or worsening chronic heart failure.

In one embodiment, the cardiac disease is stable heart failure.

In one embodiment, the cardiac disease is a less advanced state of heart failure or an advanced state of heart failure.

In one embodiment, the cardiac disease is heart failure of NYHA stage I, II, III and/or IV.

In one embodiment, the cardiac disease is myocardial infarction.

In one embodiment, the cardiac disease is myocarditis.

In one embodiment, the cardiac disease is cardiac fibrosis.

In one embodiment, there is a decrease in lncRNA Meg3 expression.

In one embodiment, there is an at least 30% reduction in Meg3 expression compared to control.

In one embodiment, a greater than 2-fold downregulation of Meg3 is indicative for an anti-fibrotic response in the patient.

In one embodiment, there is a decrease in the expression of fibrosis-related genes MMP2, COL1A1 and/or COL3A1.

In one embodiment, there is a decrease in the expression of MMP2.

In one embodiment, there is a decrease in the expression of COL1A1.

In one embodiment, there is a decrease in the expression of COL3A1.

Provided herein is a method of delivering an oligonucleotide of the invention, a vector of the invention or a pharmaceutical composition of the invention, comprising contacting the cardiac cells with the oligonucleotide, the vector or the pharmaceutical composition of the invention.

Provided herein is a method of treating or preventing a cardiac disease in a subject, wherein the method comprises administering a therapeutically effective amount of an oligonucleotide of the invention to the subject.

Also provided herein is a use of an antisense oligonucleotide according to the invention for the treatment and/or prevention of a cardiac disease.

Provided herein is a method for determining treatment efficacy in a patient using an oligonucleotide of the invention or a pharmaceutical composition of the invention, the method comprising: (a) detecting the expression level of Meg3 in a sample previously obtained from a patient, and (b) comparing the expression level obtained in (a) with the expression level of Meg3 in a sample previously obtained from at least one healthy subject or with a predetermined standard that has been obtained from a sample of at least one healthy subject, wherein a greater than 2-fold downregulation of Meg3 is indicative for a prophylactic or therapeutic response in the patient.

In one embodiment, there is an at least 30% reduction in Meg3 expression compared to control.

In one embodiment, the subject is a human.

In one embodiment, the subject is an adult.

In one embodiment, the subject is a human child.

In one embodiment, the oligonucleotide of the invention is administered at a concentration between 0.05 to 20 mg/kg.

Also provided herein is a kit comprising means for the detection of the expression level of Meg3, and instructions

how to use the kit.

The present invention shall be described in more detail by the following Figures and Examples.

## EXAMPLES

Assays for testing the effects of the different oligonucleotides may be conducted using any assay known in the art. Further, any assays known to those skilled in the art can be used to evaluate the prophylactic and/or therapeutic utility of the oligonucleotides and compositions described herein, for example, by measuring a condition or symptoms associated with cardiac inflammation and/or cardiac fibrosis. Oligonucleotides and/or compositions thereof can be tested for *in vitro* efficacy and/or toxicity by standard experimental procedures in cell culture and/or experimental animals.

## MATERIALS AND METHODS

Primary fibroblasts: Cryopreserved human cardiac fibroblasts (HCF) of multiple donors were obtained from Promocell (#C-12375). After thawing, the cells which were preserved in cryovials were transferred into pre-warmed human fibroblast medium (1 ng/mL Basic Fibroblast Growth Factor (bFGF) and 5 μg/mL Insulin (Promocell), 1 % Penicillin-Streptomycin (P/S) (Gibco), and 10 % Fetal Bovine Serum (FBS) (Gibco) in Fibroblast Basal Medium 3 (Promocell)). The cells were incubated at 37°C and 5 % $CO_2$. The medium was exchanged 24 h after thawing and every 96 h after passaging. HCFs were passaged every 7 days. First, cells were washed 2x with PBS (Invitrogen) and afterwards incubated with 1X Trypsin/EDTA (Invitrogen) for 3 - 5 min. To block trypsinization, Dulbecco's Modified Eagle's Medium (DMEM) with high glucose (Gibco) including 10 % of FBS (Gibco) was used. Subsequently, HCFs were centrifuged with 300 g at 4°C for 5 min. Then, cells were counted using a cell counter (Countess, Invitrogen) and the respective cell number was seeded. Cryopreserved human primary liver fibroblasts (HPLFs, Cell Biologic, Cat.-no: H-6019, LOT: F092116, female) and human primary kidney fibroblasts (HPKFs) were cultured similar to HCFs but in Complete Fibroblast Medium (10% FBS (Gibco), FGF, hydrocortisone, antibiotics) (Cell Biologics)). Neonatal rat cardiac fibroblasts (NRCFs) were isolated from neonatal rat pups at the age of 0.5 to 3 days using the Neonatal Heart Dissociation Kit (Miltenyi) following the manufacturer's protocol and cultured in DMEM with high glucose (Gibco) including 10 % FBS (Gibco) and 1 % P/S (Gibco).

Culturing of cell lines: HepG2 cells (human liver cancer cell line) were cultured in RPMI1640 including 10 % heat-inactivated FBS (Gibco)). Human embryonic lung fibroblasts (MRC5s) were cultured in DMEM with high glucose (Gibco), 10 % FBS (Gibco), 1 % P/S (Gibco). Splitting and culturing was performed as described for primary fibroblasts.

Oligonucleotide transfections and chemical treatments: To induce post-transcriptional silencing, cells were transiently transfected with 50 nM GapmeRs (hsa MEG3 (Axolabs); Antisense LNA™ GapmeR (only used for *in vitro* experiments) (Qiagen), or *in vitro* standard negative control (Qiagen)) applying Lipofectamine 2000 (Invitrogen) according to manufacturer's instructions. The Antisense LNA™ GapmeR (Qiagen, Cat. No. 339515 LG00000002-DDA) contains phosphorothioate backbone modifications indicated by "*", the position of the LNA modifications is not shown. Antisense LNA™ GapmeR sequence: A* A *C* A *C G* T*C*T* A *T* A* C* G*C (SEQ ID NO: 59). The Antisense LNA™ GapmeR was prepared and used according to manufacturer's protocol. Briefly, oligonucleotides were mixed with Opti-MEM I (Invitrogen) to obtain the desired concentration (mix A). Separately, Lipofectamine 2000 was mixed with Opti-MEM I: 4 μL per well for a 6-well plate (mix B). Both, mix A and B were incubated for 5 min at RT. Afterwards, they were mixed 1:1 and incubated for a further 20 min at RT (transfection mix). Cells, which were pre-seeded 24 h before, were washed 1x with PBS (Invitrogen). Transfection mix was added onto the cells and evenly distributed. After 4 h of incubation at 37°C and 5 % $CO_2$, the cells were washed again 1x with PBS. After 48 h, functional *in vitro* assay (WST-1 and Lactate dehydrogenase release assay) were performed. To perform RNA isolation, cells were harvested after 72 h and frozen in 1 mL TriFast (Peqlab)/Qiazol (Qiagen).

RNA isolation: Total RNA was isolated from cultured cells and tissue samples using Qiazol (Qiagen) according to the manufacturer's instructions. Briefly, tissue samples (50-100 mg) were homogenized in TriFast/Qiazol (1 mL) into a 2 mL Precelly24 tube containing ceramic beads (2.8 mm). The samples were homogenized at 5500 rpm for 2x 20 sec in a Precelly24 (Bertin Instruments.) For cellular samples Qiazol was used to lyse the cells only by pipetting. Then, samples were incubated at RT for 5 min. Chloroform (200 μL) was added to the samples which were then vortexed for 15 sec and centrifuged for 15 min at 11.000 x g and 4°C. The upper aqueous phase (500 μL) was transferred into a 1.5 mL tube and the same volume of Isopropanol (500 μL) was added. After mixing, the samples were incubated for 10 min at RT and centrifuged for 10 min at 11.000 x g and 4°C. The precipitated RNA pellet was washed twice with 75 % ethanol (1 mL) and then dried at RT for 5 - 10 min. Finally, the RNA pellet was dissolved in nuclease-free water (20 μL) (Life Technologies (Gibco)). Finally, RNA quantity and quality were assessed with the Synergy HT Reader (BioTek Instruments Inc.).

DNAse digestion: Prior to hexamer reverse transcription, 500 - 1000 ng RNA was treated with DNase I to remove DNA contaminations. To do so, the RNAse-free DNase kit (Qiagen) was used according to manufacturer's protocol. A total volume of 20 μL contained 0.3 units (U) of DNase I, 1X RDD buffer, 10 U RNAseOUT and the RNA dilution. Samples were incubated at 37°C for 30 min. To stop the DNase-digest, 1.25 mM EDTA was added to the samples which were then

incubated for 5 min at 65°C.

cDNA synthesis: The cDNA Synthesis Kit (Biozym) was used according to manufacturer's instructions. First, dilutions containing 500 - 1000 ng RNA were prepared in a volume of 5.75 μL and then filled up to 10 μL by adding a Master mix which was composed of cDNA Synthesis buffer (2 μL), dNTP Mix (1 μL), Hexamer Primers (0.5 μL), RNAse Inhibitor (0.25 L) and Reverse Transcriptase (RT) (0.5 μL). To confirm successful removal of DNA contaminations a - RT control was prepared lacking reverse transcriptase. The reverse transcription was performed in a thermocycler (T3000, Biometra) according to the specific protocol (**Table 1**). Then, cDNA was diluted in nuclease-free waster (Life Technologies (Gibco)) to a final concentration of 15 ng/μL.

**Table 1: Thermocycler program.**

| Hexamer primer reaction | |
|---|---|
| 30°C | 10 min |
| 50°C | 60 s |
| 99°C | 05 s |
| 08°C | Hold |

Quantitative polymerase chain reaction (qPCR): Amplification of mRNAs and IncRNAs was performed via quantitative PCR (qPCR) in a 384-well format using iQ SYBR Green Supermix (BioRad) or AbsoluteBlue SYBR Thermo Mix (Thermo Fisher Scientific) according to manufacturer's instructions. The master mix composed of 5 μL iQ SYBR Green Supermix, 0.05 μL of ROX Reference Dye (1:50 dilution) (Thermo Scientific), 0.025 μL Precision Blue™ Real-Time PCR Dye (BioRad), 0.5 μL of pre-mixed primer (10 μM forward and 10 μM reverse primer) or 1 μL 10X QuantiTect primer assay (Qiagen) and 2.45 μL nuclease-free water (Life Technologies (Gibco)) was mixed with 2 μL cDNA (10 ng/μL). The AbsoluteBlue SYBR Thermo Mix (5 μL) needed only to be mixed with 0.5 μL of pre-mixed primer (10 μM forward and 10 μM reverse primer) or 1 μL 10X QuantiTect primer assay, 2.45 μL nuclease-free water and 2 μL cDNA (10 ng/μL). The respective qPCR protocol was run on a ViiA™ 7 (ABI) or Quant Studio 77 Flex (ABI) using the protocols described in **Table 2.**

**Table 2: Protocols for qPCR.**

| iQ SYBR Green Supermix | | AbsoluteBlue SYBR Thermo Mix | |
|---|---|---|---|
| 95°C | 3 min | 95°C | 15 min |
| 95°C | 15 s | 95°C | 15s |
| 60°C | 30 s | 60°C | 30 s |
| 72°C | 40 s | Cycles: 40 | |
| Cycles: 45 | | | |

Both qPCR protocols ended with the generation of a melting curve with fluorescence detection every 0.5°C from 95°C to 55°C for 10 sec to ensure PCR amplicon-specific amplification. The gene-specific expression levels were normalized to the reference genes TATA-binding protein *(TBP)* (for human samples) or hypoxanthine-guanine phosphoribosyl transferase (*Hprt*) (for rat samples). The qPCR data were analyzed according to the ΔΔ-Ct method. A list of all used primers is provided in **Table 3**.

**Table 3: List of all primers used for qPCR.** The SEQ ID NOs for each primer are shown in parentheses.

| Rat qPCR primers | | |
|---|---|---|
| **Gene name** | **Forward primer (5' → 3')** | **Reverse primer (5' → 3')** |
| *Hprt* | CAGTCAACGGGGGACATAA (SEQ ID NO: 29) | GCTGTACTGCTTGACCAAGG (SEQ ID NO: 30) |
| *Meg3* | ATGCCTTACCTGGAGCTCTC (SEQ ID NO: 31) | GTTGCTTGTCCCTCGATGTC (SEQ ID NO: 32) |
| *Mmp2* | ACAACTTCTTTCCCCGCAAG (SEQ ID NO: 33) | GCTCCATACTTTTAAGGCCCG (SEQ ID NO: 34) |
| *Cola1a1* | GAGTTTCCGTGCCTGGCCCC (SEQ ID NO: 35) | ACCTCGGGGACCCATCTGGC (SEQ ID NO: 36) |

(continued)

| Rat qPCR primers | | |
|---|---|---|
| **Gene name** | **Forward primer (5' → 3')** | **Reverse primer (5' → 3')** |
| *Col3a1* | QuantiTect Assay: Rn Col3a1 1 SG | |
| *TNF-a* | ACACACGAGACGCTGAAGTA (SEQ ID NO: 37) | TCCACTCAGGCATCGACATT (SEQ ID NO: 38) |
| *IL-6* | CTCTCCGCAAGAGACTTCCA (SEQ ID NO: 39) | AGTCTCCTCTCCGGACTTGT (SEQ ID NO: 40) |
| **Minipig qPCR primers** | | |
| *Hprt* | CCATCACATCGTAGCCCTCT (SEQ ID NO: 41) | TATATCGCCCGTTGACTGGT (SEQ ID NO: 42) |
| *Meg3* | GGAGGAGCGTTAGCATCTTG (SEQ ID NO: 43) | GACAGTGGGACAGCCTGTTT (SEQ ID NO: 44) |
| *IL-6* | CTGCTTCTGGTGATGGCTACTG (SEQ ID NO: 45) | GGCATCACCTTTGGCATCTT (SEQ ID NO: 46) |
| **Human qPCR primers** | | |
| **Gene name** | **Forward primer (5' → 3')** | **Reverse primer (5' → 3')** |
| *TBP* | CCACTCACAGACTCTCACAAC (SEQ ID NO: 47) | CTGCGGTACAATCCCAGAACT (SEQ ID NO: 48) |
| *MEG3* | GAAGAACTGCGGATGGAAGC (SEQ ID NO: 49) | CACGTAGGCATCCAGGTGAT (SEQ ID NO: 50) |
| *MMP2* | TGACATCAAGGGCATTTCAGGAGC (SEQ ID NO: 51) | GTCCGCCAAATGAACCGGTCCTTG (SEQ ID NO: 52) |
| *C0L1A1* | ACGAAGACATCCCACCAATC (SEQ ID NO: 53) | CTTGGTCGGTGGGTGACTCT (SEQ ID NO: 54) |
| *COL3A1* | GAGGATGGTTGCACGAAACAC (SEQ ID NO: 55) | GGTAGTCTCACAGCCTTGCG (SEQ ID NO: 56) |

Acid Anion-Exchange High-Performance Liquid Chromatography: For quantification of FIBEX-003 in rat and minipig plasma and tissue samples. Axolabs GmbH developed an AEX-HPLC analytical method. The analytical method is based on an Acid Anion-Exchange High-Performance Liquid Chromatography (AEX-HPLC) method with fluorescence detection that enables the sensitive and specific detection of FIBEX-003 in rat plasma and tissue samples. The assay is based on the specific hybridization of a 16-mer complementary PNA probe conjugated at the N-terminus with an Atto425 dye. The duplex of PNA and parent compound of FIBEX-003 yields a specific signal in the subsequent analysis by AEX-HPLC coupled to a fluorescence detector. Quantification was performed on an external calibration curve generated from a standard dilution series in rat plasma and tissue lysates. Linear calibration curves (weighted 1/X) were calculated from 1.00 ng/mL to 1.000,0 ng/mL for plasma and 10.0 ng/g to 10,000.0 ng/g for tissue. Non-compartmental analysis (NCA) was conducted for the concentration-time data utilizing Phoenix WinNonlin and carried out under GLP conditions.

Analysis of pro-inflammatory cytokines: For analysis of pro-inflammatory cytokines, multiplex analysis was performed. Cytokines (TNF$\alpha$, IL-1$\beta$, IL-6, IFN-$\gamma$) were measured using customized multiplex from Mesoscale Diagnostics (MSD, Rockville) according to the manufacturer's instructions. After collecting serum from week -2, day 2 and 16, samples were stored at -80$\pm$°C and analyzed together. 50 $\mu$L of serum was used for measurements in duplicates. Analysis was performed by the Fraunhofer Institute for Toxicology and Experimental Medicine (ITEM).

Hematology: The hematological investigations **(Table 4),** performed by the Fraunhofer ITEM, were conducted two weeks prior to the intravenous application of FIBEX-003, on day 2, and on day 16 after the administration. Blood was drawn from the retrobulbar vein plexus. Specifically, 0.5 mL of blood was collected from the retrobulbar vein plexus and placed in tubes coated with K3-EDTA (1.5-2 mg/mL; Sarstedt, Nümbrecht). Samples were analyzed in a randomized sequence. Clinical laboratory data were checked by internal statistical quality control using commercially available control samples.

**Table 4: Laboratory investigations.**

| | |
|---|---|
| Hematocrit | Hemoglobin |
| Leukocytes | Erythrocytes |
| MPV | Mean corpuscular volume |
| Mean corpuscular hemoglobin concentration | Mean corpuscular hemoglobin |
| Thrombocyte count (PLT) | PDW |
| RDW-SD and RDW-CV | Reticulocytes (absolute and relative) |
| Leukocyte differential count (absolute and relative):<br>Lymphocytes<br>Neutrophils<br>Eosinophilia<br>Basophils<br>Monocytes | |

**Example 1. *In vitro* screening of Meg3 specific antisense oligonucleotides.**

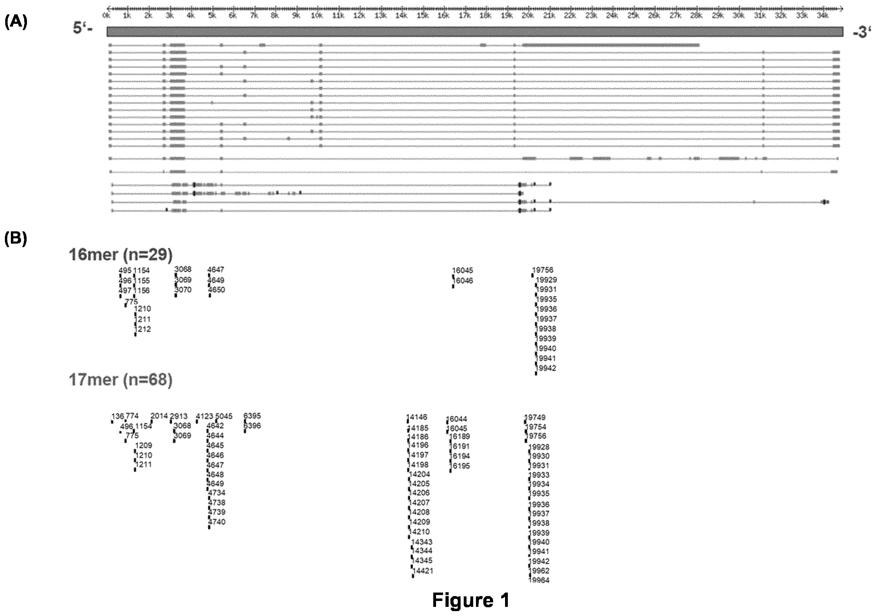

A total of 96 antisense oligonucleotides (ASOs) were bioinformatically created from the human *Meg3* sequence. A schematic representation of the human Meg3 locus and target size together with its splice variants are shown in **Fig. 1(A).** The different 16mer and 17mer oligonucleotides are shown in relative alignment to the Meg3 target sequence **(Fig. 1(B)**). Reference sequence is human Meg3 locus (NCBI Gene ID: 55384; Location: NC_000014 (100826108..100861026); annotation release RS_2023_10). Length: 34919 nt.

All ASOs were predicted to meet high specificity in human and pig with additional maximal cross-reactivity and good specificity in rat and mouse. During the screening, multiple rounds of selections were performed and ASOs were analysed for their efficiency to reduce *Meg3* expression and to induce an anti-fibrotic response as observed by reduced expression of fibrosis-related genes (*MMP2* and *COL1A1*).

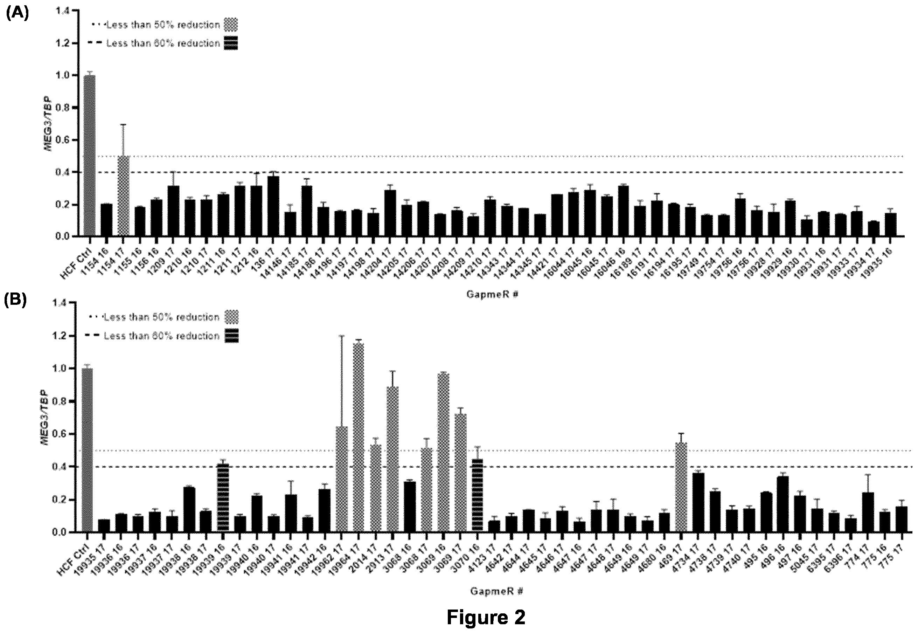

The efficiency of the ASOs was initially evaluated by performing gene expression analysis in primary human cardiac fibroblasts (HCFs) representing the primary target cell type (see, **Fig. 2**). HCFs were transfected with all 96 ASOs and gene expression levels of MEG3 and the fibrosis-related genes MMP2 and COL1A1 were analysed after 72h (50nM; n = 1 individual experiment). Specifically, 72 h post transfection, RNA of the cells was isolated and following reverse transcription, the expression level of the IncRNA *MEG3* was detected by qPCR. Cut-off threshold values were set at less than 50% (< 50%) and less than 60% (< 60%) reduction in *IncMeg3* expression. Mean ±SD.

As shown in **Fig. 2(A)** and **(B)**, only few ASOs showed reduction levels of less than 50% (< 50%) or 60% (< 60%). For instance, ASO candidates 1154.17 **(Fig. 2(A)**) and 469.17 **(Fig. 2(B))** showed about 50% reduction in IncRNA *MEG3.* However, most ASO candidates showed a reduction in IncRNA MEG3 of about 60% or more. For instance, as shown in **Fig. 2(A)**, ASO candidates 1154.16 and 14146017 showed enhanced repression of *IncRNA MEG3.*

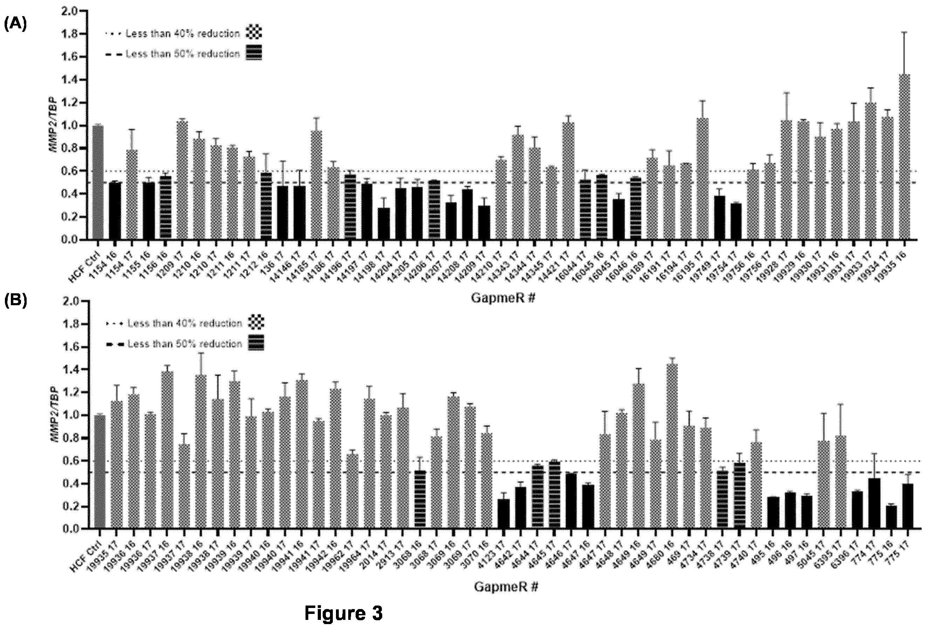

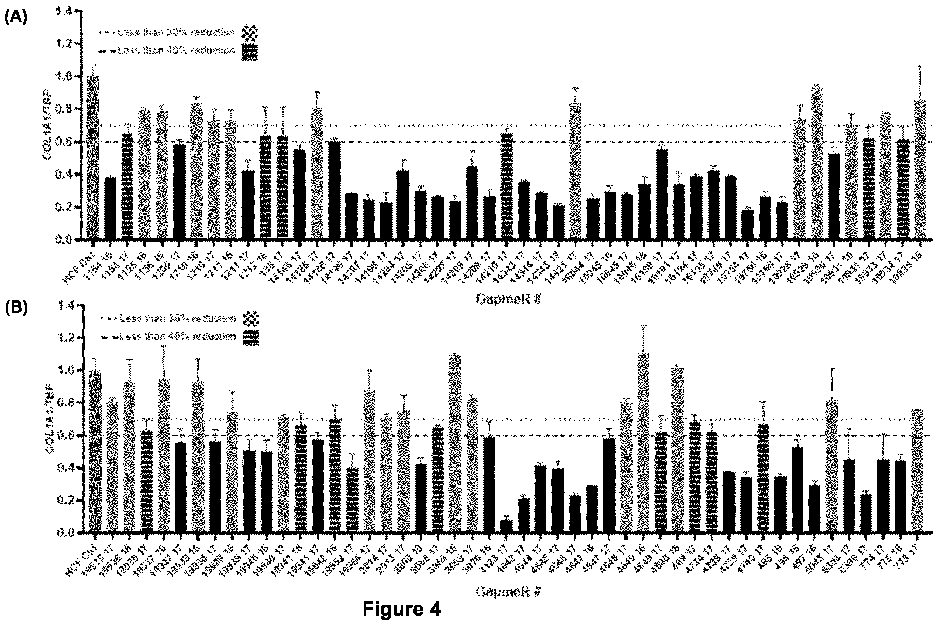

Further, gene expression levels of the fibrosis-related genes *matrix metalloproteinase (MMP)-2* **(Fig. 3(A)** and **(B)**), also representing a downstream target of *MEG3,* and *collagen 1 (COL1A1)* **(Fig. 4(A)** and **(B)**) were measured to investigate the ability of the ASOs to induce a MEG3-specific anti-fibrotic response.

The inhibitory effect of the different ASOs on MMP2 expression is shown in **Fig. 3(A)** and **(B)**. Cut-off threshold values were set at less than 40% and less than 50% reduction in MMP2 expression. Similarly, the cut-off threshold values were set at less than 30% and less than 40% reduction in *COL1A1* expression **(Fig. 4(A)** and **(B)**).

Overall, the results show that only a reduced number of the tested ASOs were able to induce a strong *MEG3* knockdown and simultaneously a clear inhibition of fibrosis-related genes. Based on the results, several ASO candidates were identified that could effectively downregulate expression of *IncMeg3* and fibrosis-related genes *MMP2* and *COL1A1* in HCF cells. The ASO candidates that showed consistent downregulation of all three targets, *IncMeg3, MMP2* and *COL1A1* are listed in **Table 5.**

**Table 5: 22 identified ASO candidates that mediate downregulation of *IncMeg3, MMP2* and *COL1A1.***

| Candidate ASO identified | |
|---|---|
| 1154.16 | 19754.17 |
| 14146.17 | 4123.17 |
| 14197.17 | 4642.17 |

(continued)

| Candidate ASO identified | |
|---|---|
| 14198.17 | 4646.17 |
| 14204.17 | 4647.16 |
| 14205.17 | 495.16 |
| 14207.17 | 496.16 |
| 14208.17 | 497.16 |
| 14209.17 | 6396.17 |
| 16045.17 | 774.17 |
| 19749.17 | 775.16 |

Based on these results, 22 ASOs were selected in a first round for further analysis.

**Example 2. Cytotoxic evaluation of pre-selected ASOs in human cardiac fibroblasts (HCF) and liver cells (HepG2).**

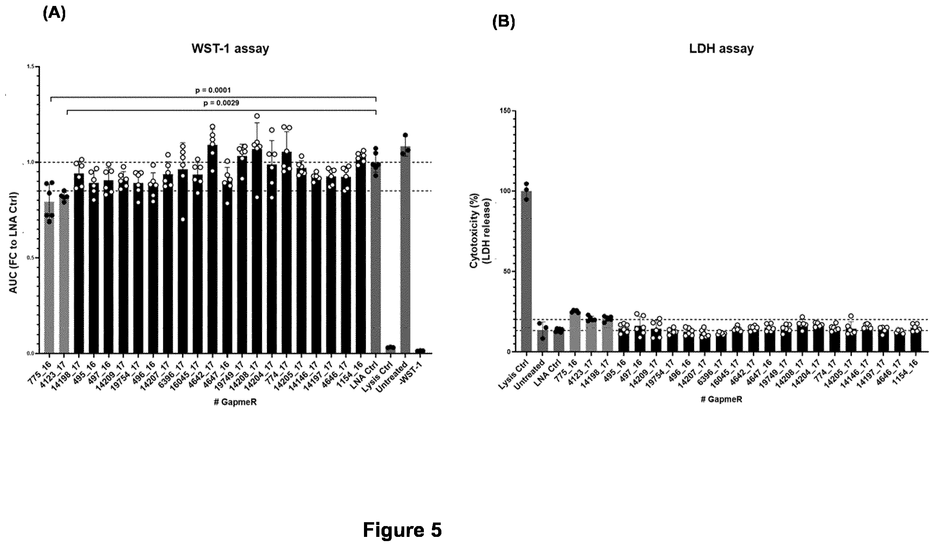

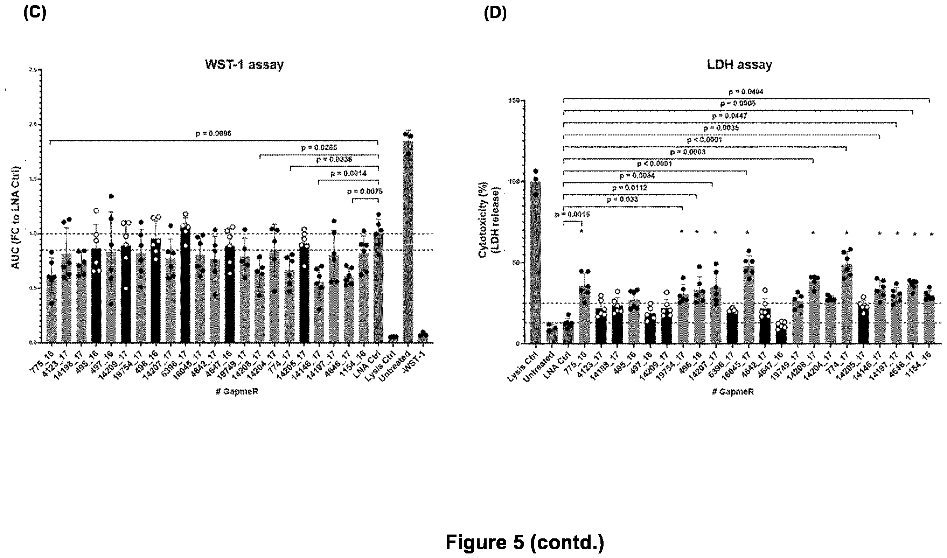

As the ASOs are investigated as potential therapeutics and with regard to future clinical application, the inventors went on to investigate the cytotoxic effect of the 22 pre-selected ASO (GapmeR) candidates in human cardiac fibroblasts (HCF) and liver cells (HepG2). HCFs and HepG2 cells were used for viability assays as ASOs with GapmeR structure are reported to potentially induce hepatotoxic effects. Accordingly, WST-1 and LDH assays were performed in HCFs and HepG2 cells. The results are shown in **Fig. 5**. Mean ±SD.

Transfection: Cells were transfected as described under Materials and Methods above with 50nM of the respective ASO and harvested 48 hrs post transfection. Untreated cells and a scramble, LNA modified oligonucleotide (Antisense LNA™ GapmeR (Qiagen, Cat. No. 339515 LG00000002-DDA)) (SEQ ID NO: 59) were used as control.

WST-1 Assay: The WST-1 assay was performed using Cell Proliferation Reagent WST-1 (water-soluble tetrazolium 1, Roche) according to manufacturer's protocol. The WST-1 assay is a metabolic assay providing information on proliferation, metabolic activity, cell viability and cytotoxicity. To focus on the last three aspects independent from proliferation cells were seeded in high density. Briefly, cells were seeded in 48- or 96-well plates and transfection was performed 24 h afterwards. 48 h post transfection, the WST-1 assay was performed. First, 100 μL substrate solution (1:10 dilution of WST-1 in the respective cell culture medium) was added per well and after 30 min, 60 min, 90 min, 120 min and 150 min of incubation at 37°C the absorbance was measured at 450 nm using a multimode live cell imaging system (Cytation 3). The wavelength 630 nm was additionally measured as a background reference. The area under curve (AUC) was calculated and samples values were normalized to the LNA control (Antisense LNA™ GapmeR (Qiagen, Cat. No. 339515 LG00000002-DDA). As technical controls, untreated cells, lysis control and blank control (medium control indicated as -WST-1) were included.

LDH Assay: Cell damage was monitored by Lactate dehydrogenase (LDH) release. LDH is a soluble cytosolic enzyme present in many cell types that is rapidly released into the cell culture medium upon disruption of the plasma membrane. Before starting the WST-1 assay, 50 μL of the conditioned medium was transferred into a fresh 96-well plate to measure the level of released lactate dehydrogenase using the CytoTox 96 Non-Radioactive Cytotoxicity Assay kit (Promega) following the manufacturer instructions. Briefly, 50 μL CytoTox 96 Reagent were added and incubated for 30 min at RT in the dark. Afterwards, an equal amount of stop solution was added per well. Subsequently, the absorbance at 490 nm was measured with the Synergy HT Reader (Biotek).

Statistical Analysis: For statistical analysis, GraphPad 6 (GraphPad Software) was used. One-way ANOVA statistical analysis and Kruskai-Wallis statistical analysis were used.

To assess cytotoxic effects, the inventors considered the combined results from the WST-1 and the LDH assays. Grey columns without fill pattern represent controls. Dashed lines were included in the graphs to improve visibility: WST-1 - upper line represents the value of the LNA Ctrl (FC = 1) and lower line represents FC = 0.85; LDH - upper line represents 25 % cytotoxicity and lower line represents the cytotoxicity (%) of the control condition (LNA Ctrl). AUC = Area under curve. Mean ±SD.

As shown in **Fig. 5(A)**, transfection of HCFs with candidate ASOs resulted in AUC values that were similar to those of untreated control. Similarly, when assessing the effect of the ASOs on HCF damage all ASOs showed levels comparable to that of untreated control **(Fig. 5(B))**. Interestingly, in HCFs only transfection of three ASOs (775_16, 4213_17, 14198_17) induced mild cytotoxic effects as indicated by reduced cell viability (WST-1) (Fig. 5(A)) and an increased LDH release **(Fig. 5(B))**. However, in HepG2 cells, a stronger effect could be observed as expected. As shown in

**Fig. 5(D)**, more ASOs induced cytotoxic effects as indicated by higher levels of cytotoxicity (%).

Following the effects uncovered through the WST-1 and LDH assays in combination, another round of selection was performed, narrowing down the number of promising ASOs to a total of seven. The sequences and modification pattern of the seven identified ASO candidates are listed in **Table 6.**

**Table 6. 7 identified lncMeg3 targeting oligonucleotide sequences and their modifications.** + = locked nucleic acids (LNA); dN = 2'-H (deoxyribose; DNA), wherein N = A, C, T, or G nucleotide; 5mdC = 5-methyl-2'-deoxycytidine; * = phosphorothioate (PS) linkage.

| Candidate ID | Candidate Sequence (5' to 3' direction) | SEQ ID NO. |
|---|---|---|
| 497 | +C*+G*+C*(5mdC)*dG*(5mdC)*(5mdC)*dA*dT*dA*dT*(5mdC)*dT*+C* +C*+C | **22** |
| 4647 | +C*+G*+G*dA*(5mdC)*dA*dA*dA*dA*(5mdC) *dT*dG*dG*+T*+T*+G | **23** |
| 14209 | +A*+A*+G*dA*dG*dT*(5mdC)*dT*(5mdC)*(5mdC)*dT*(5mdC)*(5mdC) *dT*+T*+A*+A | **24** |
| 6396 | +G*+A*+T*dT*dA*dG*(5mdC)*(5mdC)*(5mdC)*dT*dG*dT*dG*dT*+T*+ C*+A | **25** |
| 19749 | +C*+G*+G*(5mdC)*dA*(5mdC)*dA*dA*dG*dA*dG*(5mdC)*(5mdC)*dA* +A* +A*+G | **26** |
| 14204 | +T*+C*+T*(5mdC)*(5mdC)*dT*(5mdC)*(5mdC)*dT*dT*dA*dA*dG*(5md C) *+C*+C*+C | **27** |
| 14205 | +G*+T*+C*dT* (5mdC)*(5mdC)*dT*(5mdC)*(5mdC)*dT*dT*dA* dA* dG* +C*+C*+C | **28** |

In this screening, seven ASOs were identified that were particularly active and safe representing promising candidates for further preclinical analysis.

**Example 3. Efficient downregulation of Meg3 in different human fibroblasts and induction of an anti-fibrotic response by all 7 ASO candidates.**

The inventors further investigated the efficiency and cytotoxicity of the 7 identified ASOs in different human fibroblasts. To determine the impact of each ASO candidate, gene expression experiments were conducted to assess the impact of each ASO candidate on the expression level of MEG3 and different fibrosis-related genes.

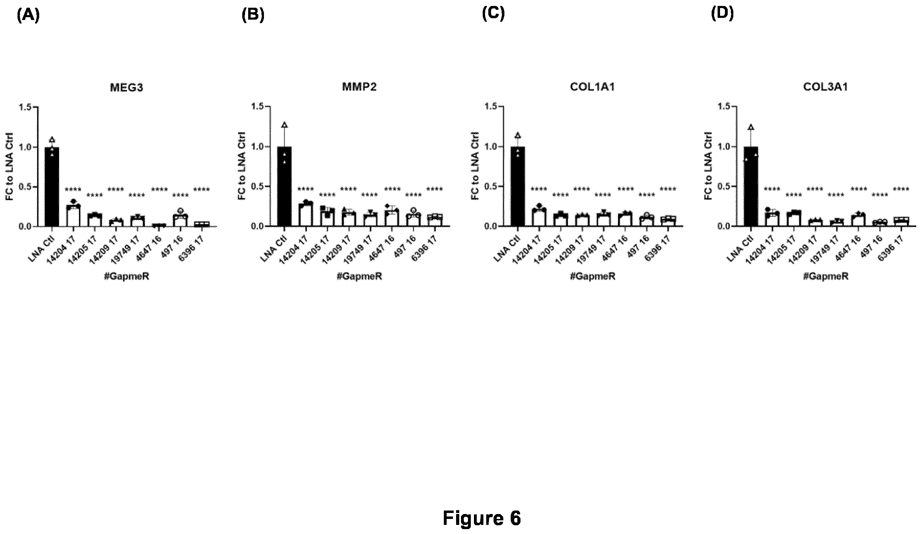

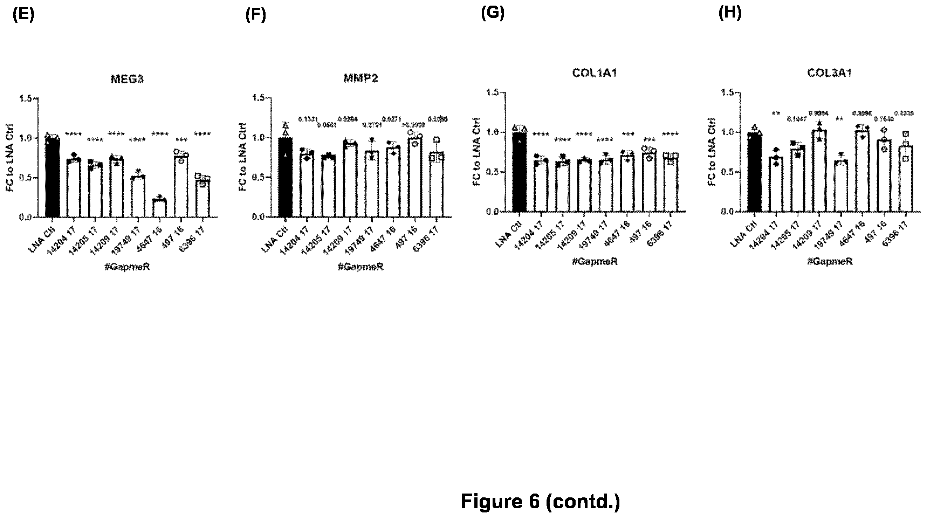

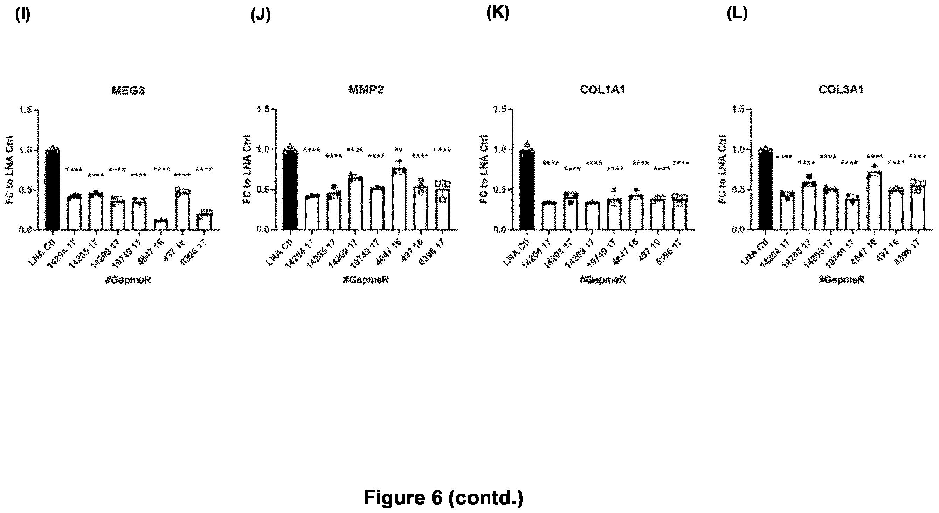

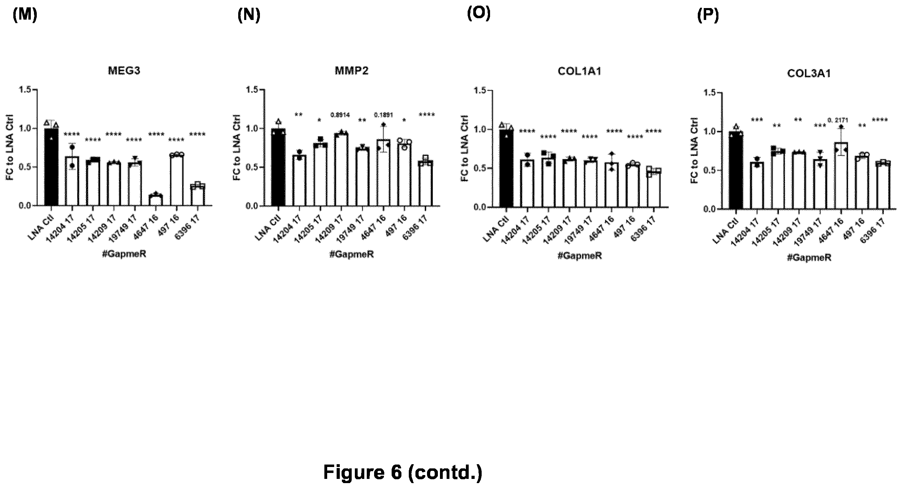

Gene expression levels of *MEG3* and fibrosis-related genes *MMP2, COL1A1* and *COL3A 1* were assessed 72h after transfection (50 nM). As shown in **Fig. 6**, all 7 selected ASOs were efficient in human fibroblasts and induced an anti-fibrotic response. One-way ANOVA. Mean ±SD.

Specifically, all 7 ASOs exhibit high efficiency in HCFs representing the primary target cell type (**Fig. 6(A) to (D)**). Gene expression levels of *MEG3* and fibrosis-related genes *MMP2, COL1A1* and *collagen 3* (*COL3A1*) were significantly reduced when compared to control. Significant effects were also observed in noncardiac fibroblasts HPLFs (**Fig. 6(I) to (L)**). For HPKFs, most of the reducing effect was significant (Fig. 6(M) to (P)). Interestingly, the smallest effects were induced in MRC5s (Fig. 6(E) to (H)).

Given that HCFs represent the cell type of interest to treat cardiac fibrosis, the weaker effects observed in other cell types are deemed acceptable.

**Example 4. ASOs show no cytotoxic effects in different types of human fibroblasts.**

To determine the cytotoxic effect of the seven leads candidates, their effect on different types of human fibroblast cells was assessed *in vitro.* WST-1 and LDH assay were performed 48 h upon transfection (50nM) of HCFs, HepG2s, MRCSs, HPLFs and HPKFs as described under **Example 2** above. One-way ANOVA and Kruskal-Wallis test. Mean ±SD.

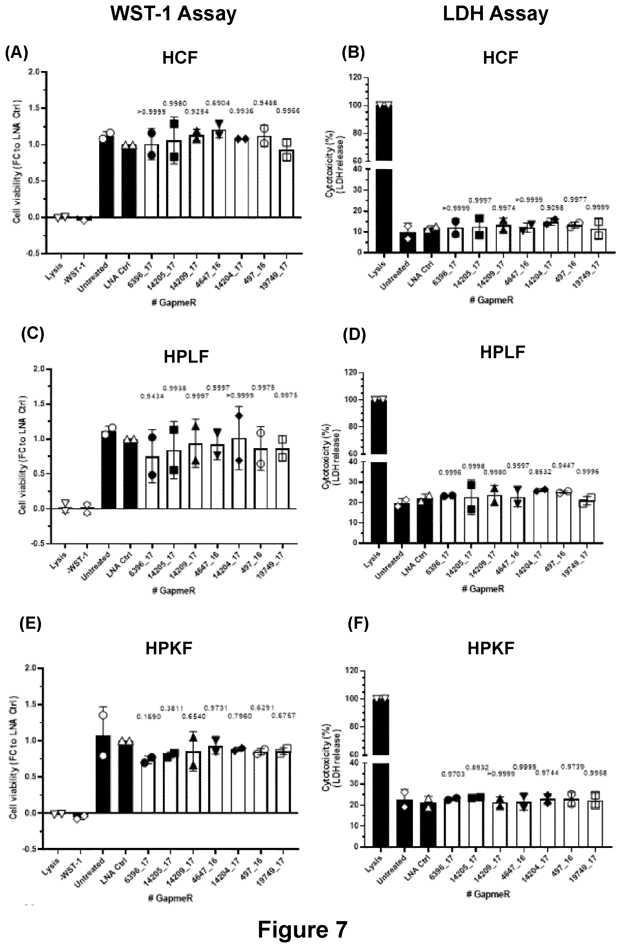

WST-1 and LDH assays revealed no cytotoxic effects in HCFs **(Fig. 7(A)** and **(B)),** HPLFs **(Fig. 7(C)** and **(D)**), and HPKFs **(Fig. 7(E)** and **(F)**).

Overall, these results shown that none of the 7 ASOs induced cytotoxic effects in human fibroblasts (*i.e.*, HCFs)

and liver cells (*i.e.,* HPLFs).

**Example 5. Induction of anti-fibrotic response in neonatal rat cardiac fibroblasts (NRCF).**

Then tested the ASO efficiency in rodent fibroblasts and performed gene expression analysis in neonatal rat cardiac fibroblast (NRCF) 72h post transfection. Gene expression levels of MEG3 and fibrosis-related genes MMP2, COL1A1 and COL3A1 were measured 72 h after transfection (50nM; n = 1 individual experiment) normalized to HPRT (50nM; n = 1 individual experiment). One-way ANOVA. Mean ±SD.

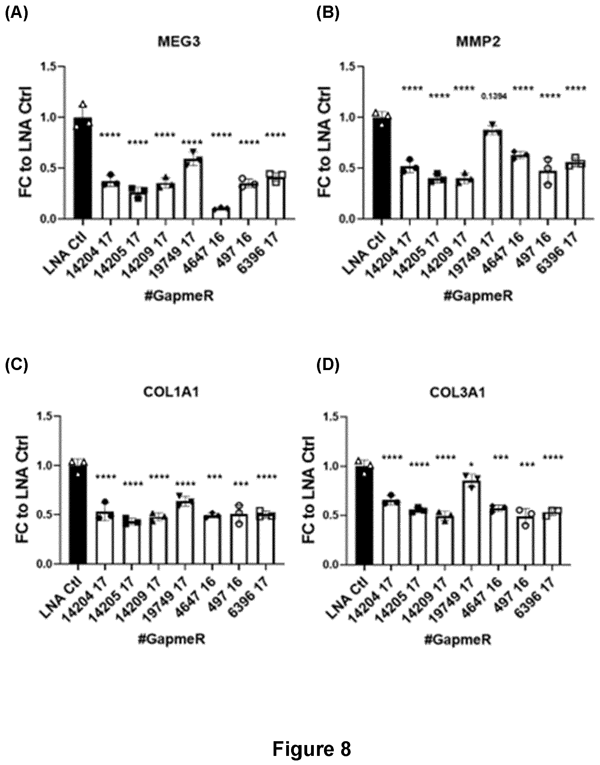

All 7 antisense oligonucleotides (ASOs) are effective in neonatal rat cardiac fibroblasts (NRCF) and induce an anti-fibrotic response. Surprisingly, all ASOs led to decreased gene expression levels of *MEG3, MMP2, COL1A1* and *COL3A1* **(Fig. 8),** despite only the following ASO sequences being identified as crossreactive in rat species: 4647 (SEQ ID NO: 23), 14209 (SEQ ID NO: 24) and 14204 (SEQ ID NO: 27). Presumably, a partial match of the ASO sequence is sufficient to induce significant effects. The smallest effects were observed for ASO 19749, which need to be considered for further development.

Most promising effects could be revealed using ASO candidate 4647 (SEQ ID NO: 23) (subsequently referred to as FIBEX-003), which was further developed preclinically.

**Example 6. Quantification of FIBEX-003 level in rat plasma and cardiac tissue.**

The objectives of this study are to establish the PK of FIBEX-003 in blood and tissue after single subcutaneous (s.c.) or intravenous (i.v.) administration in rats and to characterize the toxic potential of FIBEX-003 using clinical observation, body weight, macroscopic observations during necropsy and organ preservation.

*In vivo* non-GLP PK Study Design in rats:

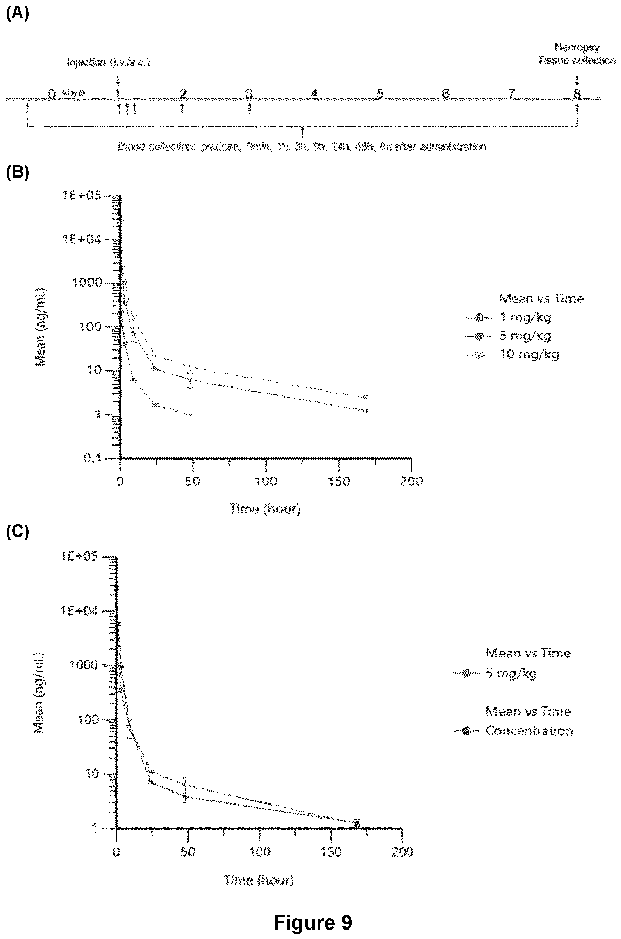

The study was performed by the Fraunhofer ITEM. Three different doses of FIBEX-003 have been tested, which were administered s.c. or i.v. via the tail vein. The application of the test item or control substance (0.9% NaCl) was performed on day 1 **(Fig. 9(A)).** Blood samples were collected before the administration (predose) and at different timepoints afterwards: 9 min, 1 h, 3 h, 9 h, 24 h, 48 h and at the experimental end point (day 8, 168 h). 1 mg/kg bodyweight (bw), 5 mg/kg bw, and 10 mg/kg bw were used for the test item in the low i.v., mid i.v., and high dose i.v. groups, respectively. 5 mg/kg bw of the test item were administered s.c. For each group six animals were used as this number of animals is sufficient to calculate the mean value of the substance in the blood. An overview is provided in **Table 7.** All rats were sacrificed on day 8 painless with $CO_2$, followed by exsanguination for gross necropsy. At necropsy, organs were divided and tissue samples were collected from heart, liver, kidney, brain, spleen, skeletal muscle, bone marrow and lung which were either stored in 10 % neutral-buffered formalin or snap frozen in liquid nitrogen.

Generally, the investigations in the PK study were conducted under non-GLP. Thus, regular phases of the study were not inspected by the Quality Assurance personnel. In practice, however, the experimental procedures follow the GLP rules (e.g., use of SOPs and documentation/archiving).

**Table 7: Treatment groups in pharmacokinetic (PK) study in rats.**

| **Dose Group (**according to Study Plan**)** | **Treatment** | **FIBEX-003 Dose (mg/kg)** | **Main study** [No. of rats] |
|---|---|---|---|
| **1** | FIBEX-003 Low Dose i.v. | 1 | 6 females |
| **2** | FIBEX-003 Mid Dose i.v. | 5 | 6 females |
| **3** | FIBEX-003 High Dose i.v. | 10 | 6 females |
| **4** | FIBEX-003 Mid Dose s.c. | 5 | 6 females |
| Total animals | | | 24 females |
| Animals for randomization | | | 2 females |

Quantification of FIBEX-003 in plasma: The concentration-time data were evaluated using Phoenix WinNonlin. The semi-logarithmic presentations of s.c. dosing and i.v. dosing show similar plasma levels over time **(Fig. 9(B)).** For i.v. dosing, the different dosing groups show the expected progress, 1 mg/kg dosing group (Low Dose) is the lowest, 5 mg/kg dosing group (Mid Dose) is much higher and 10 mg/kg dosing group (High Dose) is the highest with less difference to the Mid Dose group than the Mid Dose group has to the low dose group. PK progression of s.c. and i.v. dosing at 5 mg/kg is

approximately equal **(Fig. 9(C)),** except for $C_{max}$.

The results show that dose proportionality occurs when increases in the administered dose are accompanied by proportional increases in the measure of overall exposure (AUC) and maximal exposure (cmax). Linear kinetics applies when absorption, distribution and elimination of a drug do not depend on plasma concentration, so Cl, tmax, t1/2 and Vz are constant. Dose-normalized AUClast and cmax-values as well as Cl, tmax, t1/2 and Vz, which should be equal at linear kinetics, are listed in **Table 8:**

**Table 8: Dose normalized pharmacokinetic (PK) parameters of all dosing groups in i.v. bolus application in rats.**

| Dose | $AUC_{last}$/D | $c_{max}$/D | tmax | t1/2 | Cl_obs | Vz_obs |
|---|---|---|---|---|---|---|
| 1 mg/kg | 3900 | 5200 | 0.15 | 16 | 0.00026 | 0.0058 |
| 5 mg/kg | 4400 | 5300 | 0.15 | 47 | 0.00022 | 0.015 |
| 10 mg/kg | 4000 | 4300 | 0.15 | 47 | 0.00025 | 0.017 |

For the i.v. dosing tmax and Cl values are equal over all dosing groups. Concerning AUClast/D and cmax/D, the highest dose level shows smaller results as expected, but results are comparable. Vz and t1/2 is smallest in the lowest dosing group. The half-life calculated for the s.c. dosing group is much higher compared to i.v. dosing groups, which could be related to the simultaneous absorption, distribution and elimination phase. In i.v. dosing, the absorption phase is skipped. Regarding cmax, bioavailability determined in systemic exposure is significantly smaller in s.c. dosing, bioavailability for AUClast is significantly closer to the values observed in i.v. dosing. However, it is important to note, that the calculation of bioavailability in plasma depends on the selection of timepoints and that the purpose of the study was not to show bioequivalence in systemic exposure, but to show comparability of tissue exposure. The graphical representation suggests a proportional increase and dose linearity, however not reaching 90 % confidence, probably due to the low number of datapoints. Dose proportionality could thus not be confirmed but appears likely. Further studies with more different dosing groups would be advisable to draw firm conclusions. Clearance and volume of distribution are comparable over the tested dose range (i.v. application). Tmax appears at nine (9) minutes after i.v. bolus application, respectively one (1) hour after s.c. dosing. Dose normalized cmax is around five (5) times higher in the i.v. dosing groups compared to the s.c. dosing group. Regarding AUC values, this difference is diminished. The PK progression of s.c. dosing and i.v. dosing at 5 mg/kg is approximately equal, except of cmax.

Quantification of FIBEX-003 in cardiac tissue: Quantification of FIBEX-003 in cardiac tissue was performed in rat. Necropsy was performed 10 days after single FIBEX-003 treatment. 1 mg/kg, 5 mg/kg and 10 mg/kg doses were tested and administered i.v. or s.c. Shown is the median; i.v. = intravenously, s.c. = subcutaneously.

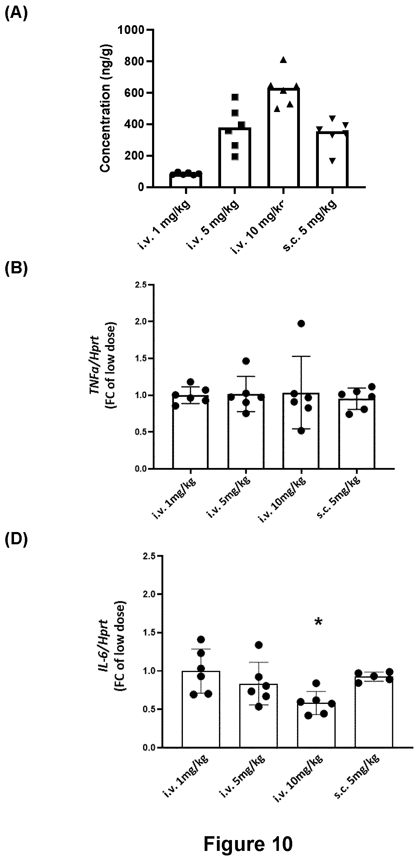

Quantification of FIBEX-003 concentration in cardiac tissue samples showed the expected dose-dependent proportional increase **(Fig. 10(A)).** Lowest levels were detected in the 1 mg/kg (Low Dose) and highest level in the 10 mg/kg dosing group (High Dose). Intravenous (i.v.) and subcutaneous (s.c.) dosing of 5 mg/kg resulted in comparable tissue concentrations, supporting the use of s.c. administration as an equally effective alternative to i.v. for achieving sufficient FIBEX-003 tissue levels.

Analysis of the target organs liver and kidney revealed similar outcomes. No dysregulation of the pro-inflammatory genes TNF$\alpha$ (tumor necrosis factor alpha) and IL-6 on mRNA level **(Fig. 10(B)** and **(C)**).

Overall, the results demonstrate that FIBEX-003 administration does not induce safety-relevant issues in major target organs of healthy animals independent of the applied different doses.

### Example 7. Quantification of FIBEX-003 level in minipig plasma and cardiac tissue and its effect on *Meg3* and IL-6 expression in target organs.

Due to the many similarities between minipigs and humans in terms of anatomy, physiology, and biochemistry, minipigs have previously been used as a potential alternative to non-human primates (NHPs) for testing the tolerability, safety, and efficacy of single-stranded oligonucleotides (Braendli-Baiocco *et al.,* 2017). Hence, the inventors analyzed minipig plasma and tissue samples (including PK and RF study samples) by the PNA-AEX-HPLC Assay for the determination of FIBEX-003.

Non-GLP PK-RF Study in minipigs: The objective of the PK part of this study is to investigate the PK profile of FIBEX-003, when given i.v. to female Göttingen minipigs. This study was combined with RF study to determine the potential toxicity of FIBEX-003, when given i.v. to female Göttingen minipigs on two occasions, two weeks apart. In addition, the toxicokinetic characteristics of FIBEX-003 were determined. Detailed information are provided in **Table 9-10** and **Fig. 9(A).** The study was performed by Charles River Laboratories Den Bosch B. V..

**Table 9: Overview on combined study parts: pharmacokinetic (PK) and range finding (RF) study in minipig.**

| Study | Subcontractor | GLP Status | Animals | Study Design |
|---|---|---|---|---|
| **PK pig study** | Charles River Laboratories Den Bosch B. V. | Non-GLP | Göttingen minipig (female) | Dosing: 1, 5 mg/kg i.v. at day 1 study endpoint: day 10 |
| **14 days RF minipig study** | Charles River Laboratories Den Bosch B. V. | Non-GLP | Göttingen minipig (female) | Dosing: 0, 20, 40 mg/kg i.v. at day 1 and 15 study endpoint: day 17 |

PK part: The day of dosing was designated as day 1. FIBEX-003 doses were 1 and 5 mg/kg in i.v. groups of the PK part of the study in three animals per group and time point **(Table 10).** Thereby the dose volume for each animal is based on the most recent body weight measurement. For assessment of plasma levels, blood was collected before the dosing (predose), 9 min, 1 h, 3 h, 9 h, 12, h, 24 h, 48 h, 72 h and 168 h **(Fig. 9(A)).** Brain (cerebrum), heart (left ventricle), kidney (cortex), liver (sinister lateralis), lung (left lobe), skeletal muscle, bone marrow and spleen were harvested 10 days after administration.

**Table 10: Experimental design pharmacokinetics (PK) part.**

| **Dose Group** (according to Study Plan) | **FIBEX-003 Dose (mg/kg) i.v.** | **Main study** [No. of minipigs] |
|---|---|---|
| **4** | 1 (FIBEX-003) | 3 females |
| **6** | 5 (FIBEX-003) | 3 females |

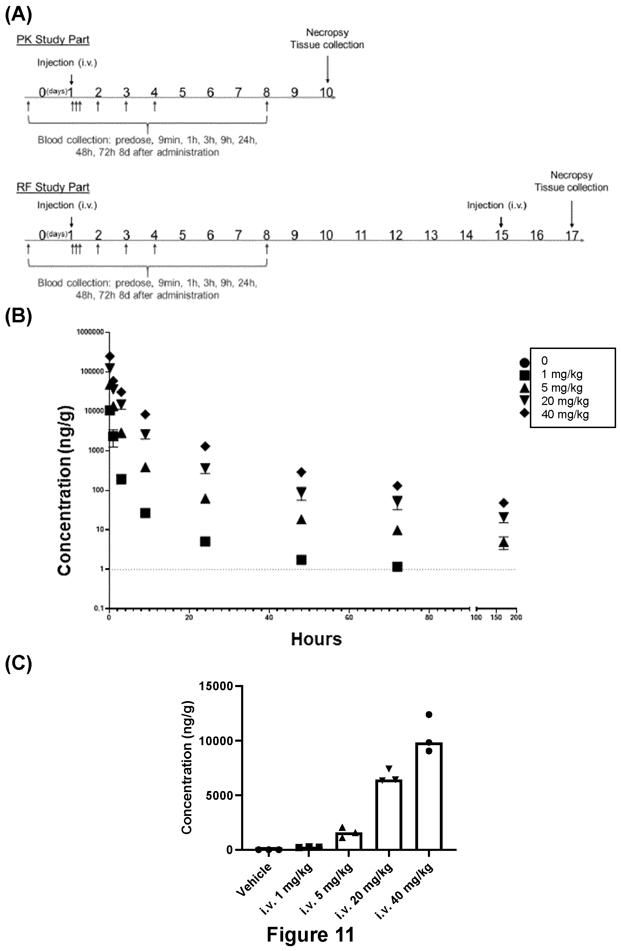

RF part: The first day of i.v. dosing was designated as day 1 and second i.v. dosing was performed on day 15. Three different dose levels were tested in this study part: 0mg/kg (vehicle), 20mg/kg and 40 mg/kg with 3 minipigs per group **(Table 11).** Similar to the PK part blood samples were collected at the following time points from all animals of groups 8 and 9: before first dosing (predose), 9 min, 1 h, 3 h, 9 h, 12 h, 24 h, 48 h, 72 h and 168 h **(Fig. 11(A)).** For group 7 only predose blood samples were collected. At the experimental endpoint (day 17), brain (cerebrum), heart (left ventricle), kidney (cortex), liver (sinister lateralis), lung (left lobe), skeletal muscle, bone marrow and spleen were harvested.

**Table 11: Experimental design range finding (RF) part.**

| **Dose Group** (according to Study Plan) | **FIBEX-003 Dose (mg/kg) i.v.** | **Main study** [No. of minipigs] |
|---|---|---|
| **7** | 0 (vehicle) | 3 females |
| **8** | 20 (FIBEX-003) | 3 females |
| **9** | 40 (FIBEX-003) | 3 females |

In minipig plasma, the semi-logarithmic presentation of i.v. dosing showed the expected progression. The 1 mg/kg dosing group had the lowest concentration of FIBEX-003 at each time point measured **(Fig. 11(B)).** The concentration in the 5 mg/kg dosing group was much higher and even higher in the 20 mg/kg dosing group with less difference to the 5 mg/kg group compared to the 1 mg/kg group and the highest concentration is measured in the 40 mg/kg dosing group with more difference to the 5 mg/kg group than the 1 mg/kg group.

Subsequently, the level of FIBEX-003 in cardiac tissue was determined. The quantification of FIBEX-003 concentration in cardiac tissue samples showed the expected dose-dependent proportional increase (**Fig. 11(C)**). The highest levels were detected in the 40 mg/kg group and the increase in comparison to the other dosing groups is smaller for the 20 mg/kg group than for the 5 mg/kg group as expected.

Overall, the results show that FIBEX-003 was stably expressed in the plasma and cardiac tissue of minipigs.

Subsequently, the inventors proceeded to investigate the effect of FIBEX-003 on gene expression in major minipig target organs.

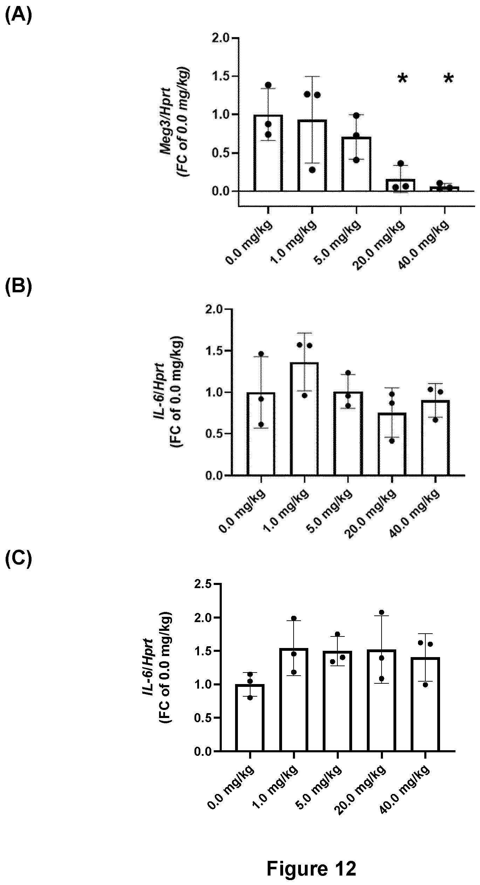

Gene expression analysis was performed in heart and kidney tissue representing major target organs after i.v. administration of FIBEX-003. Thereby, an effective reduction in *Meg3* gene expression in a dose-dependent manner was observed indicating successful Target Engagement in healthy minipigs with physiological *Meg3* level **(Fig. 12(A)).**

To investigate whether FIBEX-003 administration induces an inflammatory response, which lead to upregulated levels of inflammatory marker genes, *IL-6* level was analyzed. However, no increase could be observed either in heart tissue **(Fig. 12(B)**) or kidney tissue **(Fig. 12(B)).**

Overall, the results demonstrate that FIBEX-003 administration induced a dose-dependent response in the

minipigs, effectively downregulating Meg3 expression without elevating levels of inflammatory marker genes, such as IL-6.

**Example 8. DRF Study including indicative immune toxicology in rats.**

The objectives of the study are to investigate the adverse and adaptive effects of FIBEX-003 following two intravenous applications in rat to characterize its toxic potential. Plasma samples were used to perform hematology and cytokine analysis (TNF$\alpha$, IL-1$\beta$, IL-6, IFN-y).

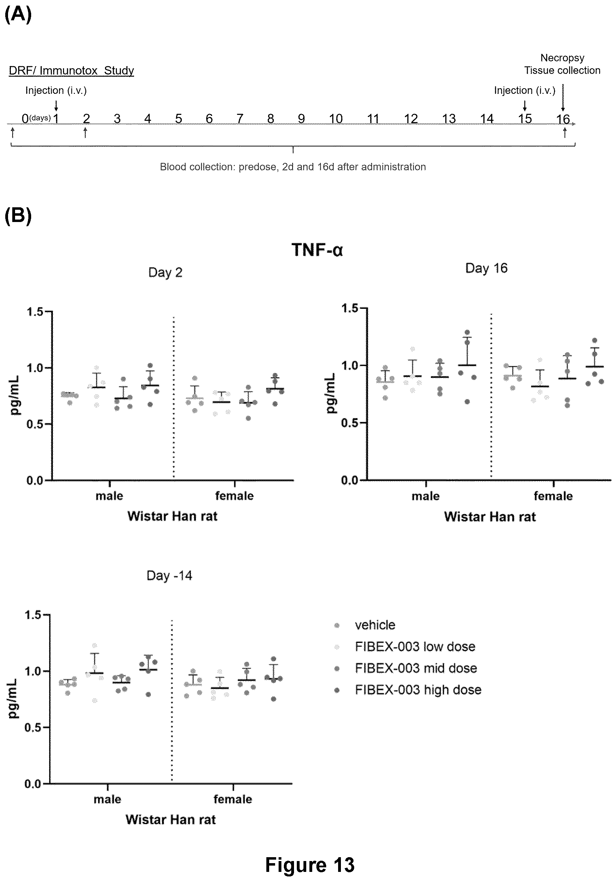

Study: The study was performed by the Fraunhofer ITEM. The FIBEX-003 was administered to the test animals via intravenous application. Doses of 4 mg/kg bw, 20 mg/kg bw, and 100 mg/kg bw were used for the test item in the low, mid, and high dose groups, respectively. In this DRF study, a 5x dose scaling scheme was also employed, ranging from 4 mg/kg bw to 100 mg/kg bw. The highest dose was selected with the aim of causing mild to moderate toxic effects. The test item was injected via tail vain on day 1 and 15 in female and male rates (n = 5 each). Study endpoint was planned for day 16 **(Fig. 13(A), Table 12).**

Brain (cerebrum), heart (ventricle), kidney (cortex), liver (sinister lateralis), lung, spleen were harvested 14 days after administration. Blood samples were taken pre-dose and on day 2 and day 16 for hematology and cytokine analysis (TNFa, IL-1$\beta$, IL-6, IFN-y). The design the Dose Range Finding (DRF) Study including indicative immune toxicology of FIBEX-003 is shown in **Fig. 13(A).**

**Table 12: Treatment groups in Dose Range Finding (DRF) study including indicative immune toxicology in rats.**

| **Dose Group** (according to Study Plan) | **Treatment (i.v.)** | **FIBEX-003 Dose (mg/kg)** | **Main study** [No. of **rats]** |
|---|---|---|---|
| **1** | Vehicle | 0 | 5 males<br>5 females |
| **2** | FIBEX-003 Low Dose | 4 | 5 males<br>5 females |
| **3** | FIBEX-003 Mid Dose | 20 | 5 males<br>5 females |
| **4** | FIBEX-003 High Dose | 100 | 5 males<br>5 females |
| Total animals | | | 20 males<br>20 females |
| Animals for randomization | | | 1 males |
| | 1 females | | |

Hematology: Hematology was conducted 14 days prior to the administration of FIBEX-003 (day -14), as well as 24 h after the 1st administration (day 2) and after the 2nd administration of the test item (day 16). Few statistically significant differences were observed in males. However, since this change already occurred prior to the treatment, it is unrelated to the treatment. Further on day 16, a few statistically significant differences were observed that are considered incidental and due to biological or analytical variance.

Immunotoxicity: Immunotoxic potential of FIBEX-003 was evaluated by assessing changes of pro-inflammatory cytokine levels (TNF$\alpha$, IL-1$\beta$, IL-6, IFN-y) in serum. Therefore, serum samples collected 14 days prior to the administration of the test item (day -14) to determine endogenous levels, as well as 24 h after the first administration (day 2) and second administration of the test item (day 16).

TNF$\alpha$ Level: The basis level of TNF$\alpha$ measured in all groups 14 days before treatment was 0.8 +/- 0.3 pg/mL in male and 0.8 +/-0.3 pg/mL in female Wistar Han **(Fig. 13(B)).** No significant changes in TNF$\alpha$ values were observed in any of the treatment groups on days 2 and 16 of the study, compared to the vehicle controls and baseline levels measured on day -14 **(Fig. 13(B)).** Summary data are provided in **Tables 13** and **14.**

**Table 13: Summary of mean+/-SD values and significant changes in TNFa levels in male and female rats on day 2, 24h after 1st application of the drug.**

| ***Male*** | | | | ***Changes vs vehicle control*** | | |
|---|---|---|---|---|---|---|
| ***Parameter*** | ***Group*** | | | *Group/change/significance level (unpaired t-test)* | | |
| | | *Mean* | *SD* | *Fold* | *Increase/Decrease* | *P-Value* |
| *TNFa in [pg/mL]* | *Basis levels* | | | | | |
| | vehicle | 0,75 | 0,03 | - | - | - |
| | FIBEX-003 low dose | 0,83 | 0,13 | - | - | - |
| | FIBEX-003 mid dose | 0,73 | 0,10 | - | - | - |
| | FIBEX-003 high dose | 0,84 | 0,13 | - | - | - |
| ***Female*** | | | | ***Changes vs vehicle control*** | | |
| ***Parameter*** | ***Group*** | | | *Group/change/significance level (unpaired t-test)* | | |
| | | *Mean* | *SD* | *Fold* | *Increase/Decrease* | *P-Value* |
| *TNFa in [pg/mL]* | *Basis levels* | | | | | |
| | vehicle | 0,73 | 0,11 | - | - | - |
| | FIBEX-003 low dose | 0,69 | 0,09 | - | - | - |
| | FIBEX-003 mid dose | 0,69 | 0,10 | - | - | - |
| | FIBEX-003 high dose | 0,82 | 0,10 | - | - | - |

**Table 14: Summary of mean+/-SD values and significant changes in TNF-α levels in male and female rats on day 16, 24h after 2nd application of the drug.**

| *Male* | | | | *Changes vs vehicle control* | | |
|---|---|---|---|---|---|---|
| *Parameter* | *Group* | | | *Group/change/significance level (unpaired t-test)* | | |
| | | *Mean* | *SD* | *Fold* | *Increase/Decrease* | *P-Value* |
| *TNFa in [pg/mL]* | *Basis levels* | | | | | |
| | vehicle | 0,86 | 0,10 | - | - | - |
| | FIBEX-003 low dose | 0,91 | 0,14 | - | - | - |
| | FIBEX-003 mid dose | 0,90 | 0,12 | - | - | - |
| | FIBEX-003 high dose | 1,00 | 0,24 | - | - | - |
| *Female* | | | | *Changes vs vehicle control* | | |
| *Parameter* | *Group* | | | *Group/change/significance level (unpaired t-test)* | | |
| | | *Mean* | *SD* | *Fold* | *Increase/Decrease* | *P-Value* |
| *TNFa in [pg/mL]* | *Basis levels* | | | | | |
| | vehicle | 0,91 | 0,08 | - | - | - |
| | FIBEX-003 low dose | 0,82 | 0,14 | - | - | - |
| | FIBEX-003 mid dose | 0,89 | 0,20 | - | - | - |
| | FIBEX-003 high dose | 0,99 | 0,16 | - | - | - |

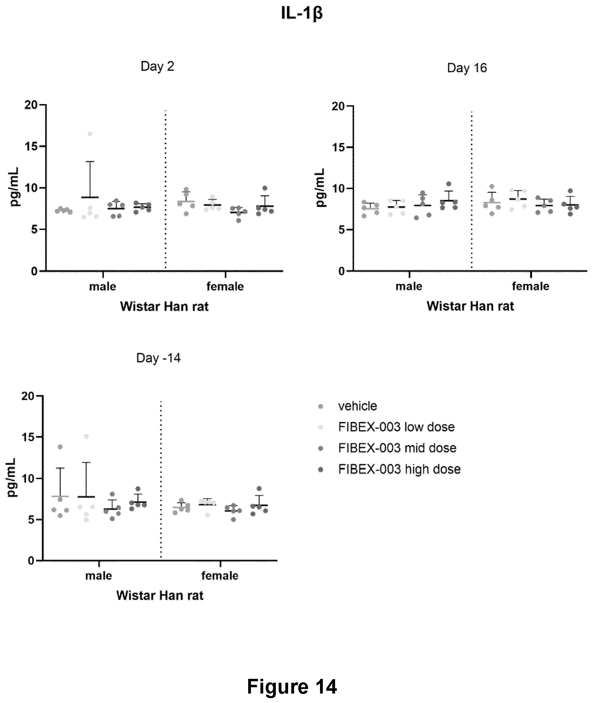

IL-1β Level: The basis level of IL-1β measured in all groups 14 days before treatment was 6.6 +/- 2.9 pg/mL in male and 5.7/-2.2 pg/mL in female Wistar Han **(Fig. 14,** "Day -14"). No significant changes in IL-1β values were observed in any of the treatment groups on days 2 and 16 of the study, compared to the vehicle controls and baseline levels on day -14 **(Fig. 14).** A summary of the data is provided in **Tables 15** and **16.**

**Table 15: Summary of mean+/-SD values and significant changes in IL-1β levels in male and female rats on day 2, 24h after 1st application of the drug.**

| ***Male*** | | | | ***Changes vs vehicle control*** | | |
|---|---|---|---|---|---|---|
| ***Parameter*** | ***Group*** | | | *Group/change/significance level (unpaired t-test)* | | |
| | | *Mean* | *SD* | *Fold* | *Increase/Decrease* | *P-Value* |
| *IL-1β in [pg/mL]* | *Basis levels* | | | | | |
| | vehicle | 7,29 | 0,18 | - | - | - |
| | FIBEX-003 low dose | 8,84 | 4,32 | - | - | - |
| | FIBEX-003 mid dose | 7,51 | 0,87 | - | - | - |
| | FIBEX-003 high dose | 7,65 | 0,45 | - | - | - |
| ***Female*** | | | | ***Changes vs vehicle control*** | | |
| ***Parameter*** | ***Group*** | | | *Group/change/significance level (unpaired t-test)* | | |
| | | *Mean* | *SD* | *Fold* | *Increase/Decrease* | *P-Value* |
| *IL-1β in [pg/mL]* | *Basis levels* | | | | | |
| | vehicle | 8,37 | 1,17 | - | - | - |
| | FIBEX-003 low dose | 7,94 | 0,68 | - | - | - |
| | FIBEX-003 mid dose | 7,05 | 0,62 | - | - | - |
| | FIBEX-003 high dose | 7,80 | 1,24 | - | - | - |

**Table 16: Summary of mean+/-SD values and significant changes in IL-1β levels in male and female rats on day 16, 24h after 2nd application of the drug.**

| ***Male*** | | | | ***Changes vs vehicle control*** | | |
|---|---|---|---|---|---|---|
| ***Parameter*** | ***Group*** | | | *Group/change/significance level (unpaired t-test)* | | |
| | | *Mean* | *SD* | *Fold* | *Increase/Decrease* | *P-Value* |

| | | | | | | |
|---|---|---|---|---|---|---|
| *IL-1β in [pg/mL]* | *Basis levels* | | | | | |
| | vehicle | 7,54 | 0,68 | - | - | - |
| | FIBEX-003 low dose | 7,74 | 0,80 | - | - | - |
| | FIBEX-003 mid dose | 7,92 | 1,30 | - | - | - |
| | FIBEX-003 high dose | 8,50 | 1,19 | - | - | - |

| ***Female*** | | | | ***Changes vs vehicle control*** | | |
|---|---|---|---|---|---|---|
| ***Parameter*** | ***Group*** | | | *Group/change/significance level (unpaired t-test)* | | |
| | | *Mean* | *SD* | *Fold* | *Increase/Decrease* | *P-Value* |
| *IL-1β in [pg/mL]* | *Basis levels* | | | | | |
| | vehicle | 8,28 | 1,25 | - | - | - |
| | FIBEX-003 low dose | 8,70 | 1,05 | - | - | - |
| | FIBEX-003 mid dose | 7,92 | 0,79 | - | - | - |
| | FIBEX-003 high dose | 8,00 | 1,05 | - | - | - |

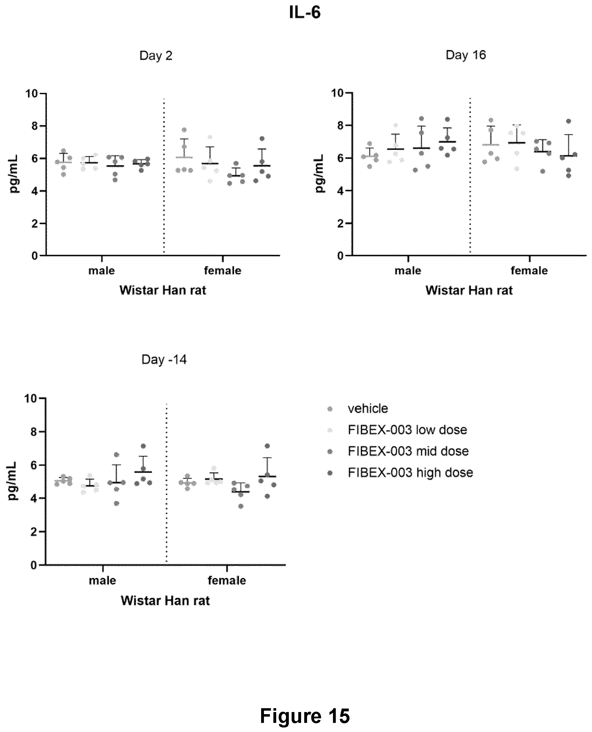

IL-6 Level: Basis level of IL-6 measured in all groups 14 days before treatment was 4.5 +/-1.7 pg/mL in male and 4.3/-1.7 pg/mL in female Wistar Han **(Fig. 15,** "Day - 14"). No significant changes in IL-6 levels were observed in any of the treatment groups on days 2 and 16 of the study, compared to the vehicle controls and baseline levels on day -14 **(Fig. 15).** A summary of the data is provided in **Tables 17** and **18.**

**Table 17: Summary of mean+/-SD values and significant changes in IL-6 levels in male and female rats on day 2, 24h after 1st application of the drug.**

| ***Male*** | | | | ***Changes vs vehicle control*** | | |
|---|---|---|---|---|---|---|
| ***Parameter*** | ***Group*** | | | *Group/change/significance level (unpaired t-test)* | | |
| | | *Mean* | *SD* | *Fold* | *Increase/Decrease* | *P-Value* |
| *IL-6 in [pg/mL]* | *Basis levels* | | | | | |
| | vehicle | 5,75 | 0,56 | - | - | - |
| | FIBEX-003 low dose | 5,73 | 0,39 | - | - | - |

| | FIBEX-003 mid dose | 5,53 | 0,64 | - | - | - |
|---|---|---|---|---|---|---|
| | FIBEX-003 high dose | 5,67 | 0,27 | - | - | - |
| ***Female*** | | | | ***Changes vs vehicle control*** | | |
| ***Parameter*** | ***Group*** | | | *Group/change/significance level (unpaired t-test)* | | |
| | | *Mean* | *SD* | *Fold* | *Increase/Decrease* | *P-Value* |
| *IL-6 in [pg/mL]* | *Basis levels* | | | | | |
| | vehicle | 6,06 | 1,14 | - | - | - |
| | FIBEX-003 low dose | 5,69 | 1,02 | - | - | - |
| | FIBEX-003 mid dose | 4,93 | 0,48 | - | - | - |
| | FIBEX-003 high dose | 5,55 | 1,03 | - | - | - |

**Table 18: Summary of mean+/-SD values and significant changes in IL-6 levels in male and female rats on day 16, 24h after 2nd application of the drug.**

| ***Male*** | | | | ***Changes vs vehicle control*** | | |
|---|---|---|---|---|---|---|
| ***Parameter*** | ***Group*** | | | *Group/change/significance level (unpaired t-test)* | | |
| | | *Mean* | *SD* | *Fold* | *Increase/Decrease* | *P-Value* |
| *IL-6 in [pg/mL]* | *Basis levels* | | | | | |
| | vehicle | 6,10 | 0,52 | - | - | - |
| | FIBEX-003 low dose | 6,54 | 0,92 | - | - | - |
| | FIBEX-003 mid dose | 6,61 | 1,35 | - | - | - |
| | FIBEX-003 high dose | 6,99 | 0,86 | - | - | - |
| ***Female*** | | | | ***Changes vs vehicle control*** | | |
| ***Parameter*** | ***Group*** | | | *Group/change/significance level (unpaired t-test)* | | |
| | | *Mean* | *SD* | *Fold* | *Increase/Decrease* | *P-Value* |
| *IL-6 in [pg/mL]* | *Basis levels* | | | | | |
| | vehicle | 6,82 | 1,14 | - | - | - |
| | FIBEX-003 low dose | 6,94 | 1,09 | - | - | - |

| | FIBEX-003 mid dose | 6,39 | 0,74 | - | - | - |
|---|---|---|---|---|---|---|
| | FIBEX-003 high dose | 6,14 | 1,30 | - | - | - |

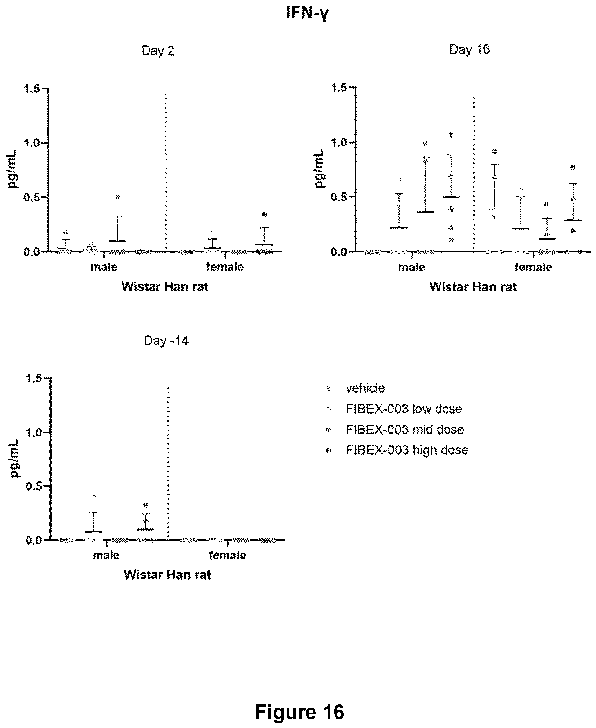

IFN-γ Level: The basis level of IFN-γ measured in all groups 14 days before treatment was 0 +/-0 pg/mL in male and 0/-0.1 pg/mL in female Wistar Han **(Fig. 16,** "Day -14"). No significant changes in the level of IFN-γ were observed in any of the treatment groups at day 2 and day 16 of the study in comparison to the vehicle controls and basis levels at day -14 **(Fig. 16).** A summary of the data is provided in **Tables 19** and **20.**

**Table 19: Summary of mean+/-SD values and significant changes in IFN-γ levels in male and female rats on day 2, 24h after 1st application of the drug.**

| *Male* | | | | *Changes vs vehicle control* | | |
|---|---|---|---|---|---|---|
| *Parameter* | *Group* | | | *Group/change/significance level (unpaired t-test)* | | |
| | | *Mean* | *SD* | *Fold* | *Increase/Decrease* | *P-Value* |
| *IFN-γ in [pg/mL]* | *Basis levels* | | | | | |
| | vehicle | 0,04 | 0,08 | - | - | - |
| | FIBEX-003 low dose | 0,02 | 0,03 | - | - | - |
| | FIBEX-003 mid dose | 0,10 | 0,23 | - | - | - |
| | FIBEX-003 high dose | 0 | 0 | - | - | - |
| *Female* | | | | *Changes vs vehicle control* | | |
| *Parameter* | *Group* | | | *Group/change/significance level (unpaired t-test)* | | |
| | | *Mean* | *SD* | *Fold* | *Increase/Decrease* | *P-Value* |
| *IFN-γ in [pg/mL]* | *Basis levels* | | | | | |
| | vehicle | 0 | 0 | - | - | - |
| | FIBEX-003 low dose | 0,04 | 0,08 | - | - | - |
| | FIBEX-003 mid dose | 0 | 0 | - | - | - |
| | FIBEX-003 high dose | 0,07 | 0,15 | - | - | - |

**Table 20: Summary of mean+/-SD values and significant changes in IFN-γ levels in male and female rats on day 16, 24h after 2nd application of the drug.**

| *Male* | | | | *Changes vs vehicle control* | | |
|---|---|---|---|---|---|---|
| *Parameter* | *Group* | | | *Group/change/significance level (unpaired t-test)* | | |
| | | *Mean* | *SD* | *Fold* | *Increase/Decrease* | *P-Value* |
| *IFN-γ in [pg/mL]* | *Basis levels* | | | | | |
| | vehicle | 0 | 0 | - | - | - |
| | FIBEX-003 low dose | 0,22 | 0,31 | - | - | - |
| | FIBEX-003 mid dose | 0,36 | 0,50 | - | - | - |
| | FIBEX-003 high dose | 0,50 | 0,39 | - | - | - |
| *Female* | | | | *Changes vs vehicle control* | | |
| *Parameter* | *Group* | | | *Group/change/significance level (unpaired t-test)* | | |
| | | *Mean* | *SD* | *Fold* | *Increase/Decrease* | *P-Value* |
| *IFN-γ in [pg/mL]* | *Basis levels* | | | | | |
| | vehicle | 0,39 | 0,41 | - | - | - |
| | FIBEX-003 low dose | 0,21 | 0,29 | - | - | - |
| | FIBEX-003 mid dose | 0,12 | 0,19 | - | - | - |
| | FIBEX-003 high dose | 0,29 | 0,33 | - | - | - |

**Example 9. FIBEX-003 level in rat cardiac tissue.**

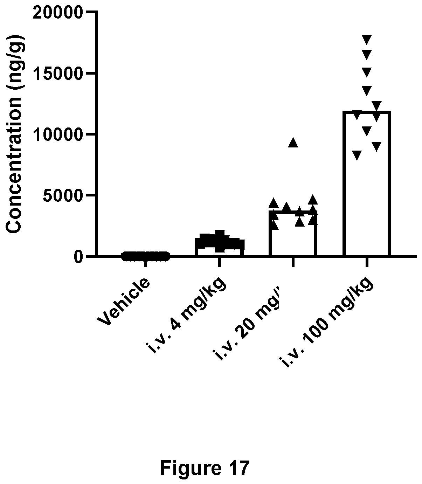

To determine the level of FIBEX-003 in cardiac tissue of the Dose Range Finding (DRF) study including indicative immune toxicology in rats, animals were injected FIBEX-003 intravenously (i.v.) at day 1 and day 15. At day 16 necropsy was performed. 4 mg/kg, 20 mg/kg and 100 mg/kg doses were tested (n = 10 per group). The results are shown in **Fig. 17** as the median. Vehicle was used as negative control.

As shown in **Fig. 17,** quantification of FIBEX-003 concentration (ng/g) in cardiac tissue samples revealed a dose-dependent increase. Lowest level was observed in the 4 mg/kg (Low Dose) group and highest level was observed in the 100 mg/kg dosing group (High Dose).

These data confirm that there is a dose dependent increase in FIBEX-003 concentration in rat cardiac tissue.

**Example 10. Gene expression and Histopathology analysis in rat heart, liver and kidney tissue.**

The heart, liver and kidneys represent the major target organs after i.v. administration of FIBEX-003. To determine the extent of FIBEX-003 expression in those organs, gene expression analysis was performed on heart, liver and kidney tissue.

Specifically, expression levels of long noncoding RNA *Meg3,* which is targeted by FIBEX-003, and inflammatory marker genes (IL-6 and TNFα) were determined. Intravenous (i.v.) injections were performed at day 1 and day 15 and necropsy was performed at day 16. 4 mg/kg, 20 mg/kg and 100 mg/kg were tested, and expression was normalized to physiological level detected in the vehicle control group (n = 9-10 per group). Kruskal-Wallis test. Mean $\pm$ SD.

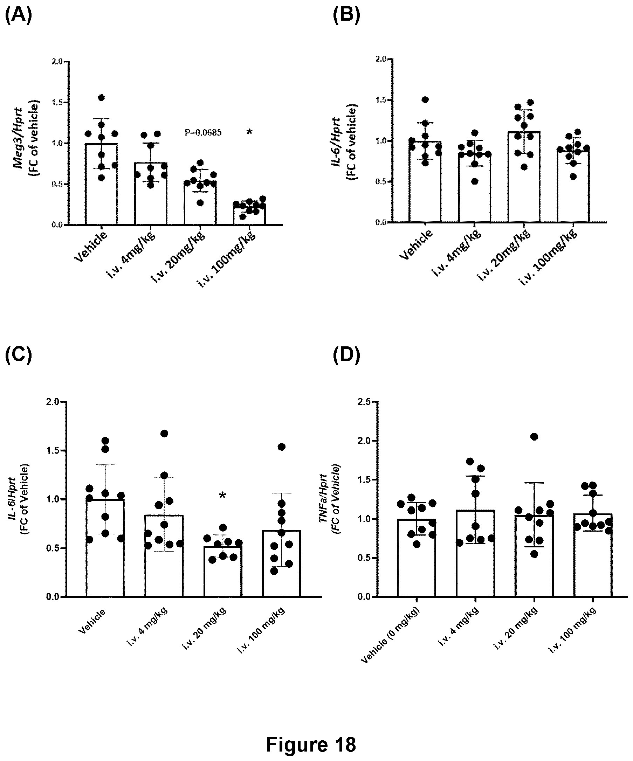

Although treatment was performed in healthy animals, with not malignantly upregulated *Meg3* levels in the heart, a dose-dependent decrease of *Meg3* levels could be observed indicating successful target engagement in healthy animals

with physiological *Meg3* level in the heart **(Fig. 18(A)).** There was a significant decrease in *Meg3* levels in the cardiac tissue of the group receiving i.v. 100 mg/kg. Furthermore, *IL-6* gene expression was not dysregulated after FIBEX-003 treatment indicating that administration does not induce an inflammatory response in cardiac tissue **(Fig. 18(B)).** Analysis of the target organs liver (for TNF-$\alpha$ expression) and kidneys (for IL-6 expression) revealed similar outcomes. No dysregulation of the pro-inflammatory genes *IL-6* and *TNF$\alpha$* on mRNA level was detectable **(Fig. 18(C)** and **(D)**).

These results demonstrate that FIBEX-003 administration does not induce safety-relevant effects in major target organs of healthy animals independent of the applied different doses.

To further determine any changes in the tissues at the microscopic level, further histologic analysis of the kidneys was performed. The histopathological summary is presented in **Table 21.**

Histopathology: The kidneys of all animals of the vehicle (control), mid dose and high dose groups were analyzed. Treatment-related findings were detected within the kidney in the investigated mid and high dose animals. The lesions comprised dose-dependent basophilic granules in the cytoplasm of the tubular epithelium. This change is considered to represent an adaptive non-adverse change. Furthermore, treatment-related tubule degeneration was observed only in the high dose group. This change is interpreted as an adverse finding. Therefore, under the condition of this study, the no-observed adverse effect level (NOAEL) represents the mid dose (20 mg/kg).

**Table 21: Histopathological summary.**

| Pathology - Intergroup Comparison of Pathology Observations<br>01N23522 - 14-Day intravenous Dose Range Finding Study including indicative immune toxicology of FIBEX-003 in Wistar Rats (Crl:WI (Han)) | | | | | | |
|---|---|---|---|---|---|---|
| Removal Reason(s): ALL<br>Summary: Incidence | Male<br>Vehicle | Male<br>Mid Dose 20 mg/kg | Male<br>High Dose 100 mg/kg | Female<br>Vehicle | Female<br>Mid Dose 20 mg/kg | Female<br>High Dose 100 mg/kg |
| Number of Animals: | 5 | 5 | 5 | 5 | 5 | 5 |
| **kidney** | | | | | | |
| Examined | 5 | 5 | 5 | 5 | 5 | 5 |
| No Visible Lesions | 3 | 0 | 0 | 4 | 0 | 0 |
| Infiltration, Mononuclear Cell; focal | 2 | 0 | 1 | 0 | 0 | 0 |
| ....very slight | 2 | 0 | 1 | 0 | 0 | 0 |
| Basophilia, Tubule; focal | 1 | 0 | 1 | 0 | 0 | 0 |
| ....very slight | 1 | 0 | 1 | 0 | 0 | 0 |
| tubular; Mineralization; multifocal | 0 | 0 | 0 | 1 | 1 | 0 |
| ....very slight | 0 | 0 | 0 | 1 | 1 | 0 |
| Basophilic Granules; multifocal | 0 | 5 | 5 | 0 | 5 | 5 |
| ....very slight | 0 | 5 | 0 | 0 | 5 | 0 |
| ....slight | 0 | 0 | 5 | 0 | 0 | 5 |
| Degeneration, Tubule; multifocal | 0 | 0 | 5 | 0 | 0 | 4 |
| ....very slight | 0 | 0 | 5 | 0 | 0 | 4 |

**Example 11. Identification of MEG3 interaction partners in HCFs.**

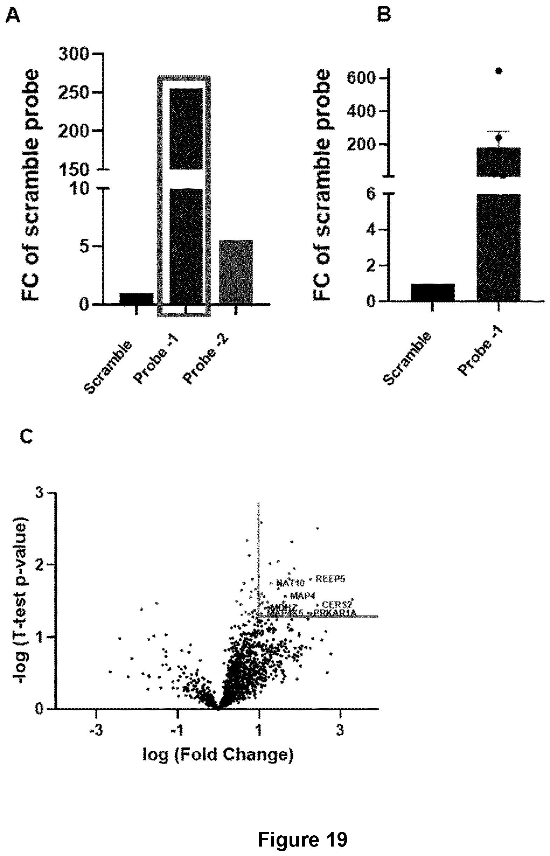

To identify new interaction partners of *MEG3,* which are potentially mediating the described anti-fibrotic functions, pulldown experiments were performed in lysates prepared from HCFs. Thereby, *MEG3* including its binding partners are enriched using a *MEG3*-specific probe, which is coupled to biotin and therefore can be pulled down using streptavidin coupled magnetic beads **(Fig. 19(A) and (B)**). Subsequently, potential protein binding partners are analyzed by mass spectrometry **(Fig. 19(C)).**

RNA Pulldown for the characterization of potential *MEG3* interaction partners: To investigate direct interaction partners of lncRNA MEG3 pulldown experiments were performed in HCFs. First, $10 \times 10^6$ cells per probe were harvested by trypsinization and washed with 1X DPBS. Cells were resuspended in cell lysis buffer, stored on ice for 10 min, and afterwards sheared mechanically using a douncer-homogenizer (Hartenstein). After incubation on ice for another 10 min,

the cell lysate was centrifuged in a low binding tube (Eppendorf) at 14,000 × g at 4 °C for 10 min. 5 % of the cleared supernatant was transferred to a new tube representing the input control and QIAzol was added. The remaining supernatant was divided among the different probes and twice the amount of hybridization buffer was added. Subsequently, incubation with 10 μg biotin labelled DNA probes (IDT) for 3 h was performed. The probes were designed to specifically bind *MEG3*, while the scramble probe does not bind to anything and serves as a negative control **(Table 22).** During incubation, streptavidin C1 magnetic beads (Invitrogen) were prepared. They were washed three times with 1000 μl of bead wash buffer, twice with solution A, and once with solution B. Composition of all buffers and solutions are provided in table 5. After each washing step, the tube containing the bead suspension was placed on a DynaMag™-2 magnet (Invitrogen) for 2 min in order to allow aspiration of the supernatant. In addition, solution A was incubated for 2 min at RT. After washing, 200 μL of bead-blocking solution containing yeast tRNA and BSA to block nonspecific binding positions was added to the beads. After incubation for two hours at 4 °C 90 μl Streptavidin C1 magnetic beads were added to each probe and further incubated at RT for 1 h. The beads were washed with 300 μL wash buffer five times and additionally three times with MS buffer when the samples were prepared for mass spectrometry (MS) analysis. First, for testing different probes all beads were used for RNA analysis. Therefore, the beads were resuspended in 1 mL QIAzol. After a specific probe was chosen, 80 % of the beads were used for the MS analysis or for protein analysis by western blotting. The remaining 20 % of the sample was used to confirm successful enrichment of MEG3 by expression level analysis via qPCR. Beads were stored at -70 °C.

**Table 22: MEG3-specific Pulldown probes tested in HCFs.** BIO = biotinylated.

| Name | Sequence (5' → 3' direction) | SEQ ID NO: |
|---|---|---|
| *Meg3* Probe 1 | 5' TTAGGTAAGAGGGACAGCTGGCTGGAAA 3' BIO | 57 |
| *Meg3* Probe 2 | BIO 5' AAAGACATCATAAGGGTGATGACAGAGTCAGT 3' | 58 |

**Table 23: Solutions and buffers used for RNA pulldown.**

| Working solutions | |
|---|---|
| **Solutions and buffers** | **Composition** |
| Bead Wash Buffer | 5 mM Tris-HCl (pH 7.5, Roth), 0.5 mM EDTA (Ivitrogen), 1 M NaCl (Sigma-Aldrich) |
| Solution A | 100 mM NaOH (Sigma-Aldrich), 50 mM NaCl |
| Solution B | 100 mM NaCl |
| Bead Blocking Solution | 1 μg/μL BSA (Ambion), 1 μg/μL Yeast tRNA (Life Technologies) |
| Cell Lysis Buffer | 50 mM Tris (pH 7, Roth), 10 mM EDTA, 1 % SDS (Roth), 1 mM DTT (Sigma-Aldrich), 100 U/mL RNasin (Invitrogen), 1X Protease Inhibitor (Complete Mini EDTA-free Protease Inhibitor Cocktail Roche) |
| Hybridization Buffer | 0.75 M NaCl, 1 % SDS, 50 mM Tris (pH 7), 1 mM EDTA, 15 % Formamide, 1 mM DTT, 100 U/mL RNasin , 1X Protease Inhibitor |
| Wash Buffer | 10 mM Tris-HCl (pH 7.5), 10 mM KCl (Roth), 1.5 mM MgCl2 (Merck), 1 M NaCl, 5 mM DTT, 60 U/mL RNasin , 1X Protease Inhibitor |
| MS Buffer | 10 mM Tris (pH 7), 150 mM NaCl |

Sodium dodecyl sulfate polyacrylamide gel electrophoresis (SDS-PAGE) for Proteomics: In order to prepare the obtained pulldown samples for mass spectrometry, beads were resuspended in a total volume of 15 μL containing 6X loading buffer (100 mM Tris-HCl pH 6.8, 10% SDS, Glycerine, 0.3% Bromphenol Blue) and 30X Dithiothreitol (DTT, New England Biolabs) with a final concentration of 1X. All samples were mixed thoroughly and heated at 95 °C for 5 min. After the samples cooled to RT, 1.5 μL 40 % acrylamide (Aliquots from Toxicology Department) was added. Mini-PROTEAN TGX precast Gel 4-15% (Bio-Rad) were placed into the electrophoresis chamber (Bio-Rad), which was filled with 1X SDS-PAGE electrophoresis buffer (10X composed of 60 g Tris (Roth), 285 g Glycine (Sigma-Aldrich) and 100 mL 20 % SDS, filled up to 2 L with water and dissolved thoroughly). The comb was removed and pockets rinsed with 1X SDS-PAGE electrophoresis buffer. Afterwards, 15 μL of the samples were loaded into the pocket. The electrophoresis was performed at 20 V for approximately 60 min. Finally, the gel was gently removed from the plate and converted into a staining dish filled with FastGene Q-Stain (Nippon Genetics). After 45 min gentle shaking at RT the gel was transferred into Millipore water for destaining. During washing water was changed three times until the bands appeared clear. Subsequently samples were

analyzed by MS at the Core Facility Proteomics, Hannover Medical School, Carl-Neuberg-Str. 1, 30625 Hannover, Germany.

Mass spectrometry analysis of pulldown samples: To screen for potential interaction partners, MS analysis of the pulldown lysates was performed by the MHH Core Facility Proteomics. Briefly, protein samples were digested in gel with trypsin and the extracted peptides analyzed by liquid chromatography with tandem mass spectrometry (LC-MS) using orbitrap MS. Raw data were searched against reviewed human entries of uniprot database, whereby 3068 proteins were detected in total. To identify relevant interaction partners in unstimulated HCFs different filtering steps were used. First, only those proteins were taken into account, for which at least 5 of 6 quantitative values could be detected in the 6 biological replicates. The remaining 1269 proteins **(Fig. 19C)** were filtered for significantly enriched proteins compared to the scramble probe (P≤0.05) and highly abundant proteins (Fold Change≥2) revealing 33 proteins **(Table 24).**

**Table 24: Potential *MEG3* interaction partners in HCFs.** 33 proteins could be identified by performing a MEG3-specific pulldown and subsequent mass spectrometry analysis filtering candidates with FC≥2 and p≤0.05.

| Protein Name | Abbreviation |
|---|---|
| Translocating chain-associated membrane protein 1 | TRAM1 |
| Vesicle transport protein GOT1B | GOLT1B |
| Ceramide synthase 2 | CERS2 |
| DnaJ homolog subfamily B member 4 | DNAJB4 |
| Receptor expression-enhancing protein 5 | REEP5 |
| cAMP-dependent protein kinase type I-alpha regulatory subunit;cAMP-dependent protein kinase type I-alpha regulatory subunit, N-terminally processed | PRKAR1A |
| Laminin subunit beta-2 | LAMB2 |
| Oxygen-dependent coproporphyrinogen-III oxidase, mitochondrial | CPOX |
| 60S ribosomal protein L30 | RPL30 |
| Ribosome maturation protein SBDS | SBDS |
| Magnesium transporter protein 1 | MAGT1 |
| Microtubule-associated protein 4 | MAP4 |
| 60S ribosomal protein L23 | RPL23 |
| 26S proteasome non-ATPase regulatory subunit 14 | PSMD14 |
| Ribosome-binding protein 1 | RRBP1 |
| 7-dehydrocholesterol reductase | DHCR7 |
| 60S ribosomal protein L35 | RPL35 |
| Electron transfer flavoprotein subunit alpha, mitochondrial | ETFA |
| Perilipin-3 | PLIN3 |
| Phosphofurin acidic cluster sorting protein 1 | PACS1 |
| N-acetyltransferase 10 | NAT10 |
| Protein bicaudal C homolog 1 | BICC1 |
| Methylsterol monooxygenase 1 | MSMO1 |
| 40S ribosomal protein S25 | RPS25 |
| 60S ribosomal protein L27a | RPL27A |
| Malate dehydrogenase, mitochondrial | MDH2 |
| 5-AMP-activated protein kinase subunit gamma-1 | PRKAG1 |
| 40S ribosomal protein S14 | RPS14 |
| D-dopachrome decarboxylase;D-dopachrome decarboxylase-like protein | DDT;DDTL |
| Mitogen-activated protein kinase kinase kinase kinase 5 | MAP4K5 |

(continued)

| Protein Name | Abbreviation |
|---|---|
| Myosin regulatory light chain 12B;Myosin regulatory light chain 12A | MYL12B;MYL12 A |
| Ankyrin repeat domain-containing protein 17 | ANKRD17 |
| 40S ribosomal protein S11 | RPS11 |

Overall, the inventors have shown that a mixture of backbone modifications of the oligonucleotides can have a significant impact on their ability to target and effectively suppress expression of lncMeg3. In particular, the inventors have shown that a specific combination of precisely placed LNA, DNA and phosphorothioate (PS) backbone modifications provides for effective lncMeg3 targeting.

Those having ordinary skill in the art will appreciate that the disclosure can be modified in ways not specifically described herein.

## REFERENCES

1. Berk, B. C.; Fujiwara, K.; Lehoux, S. (2007). ECM remodeling in hypertensive heart disease. J. Clin. Invest. 117(3): 568-575.
2. Braendli-Baiocco, A., M. Festag, K. Dumong Erichsen, et al. (2017). From the Cover: The Minipig is a Suitable Non-Rodent Model in the Safety Assessment of Single Stranded Oligonucleotides. Toxicol. Sci. 157(1): 112-128.
3. Caley, D. P., R. C. Pink, D. Trujillano, and D. R. F. Carter (2010). Long noncoding RNAs, chromatin, and development. Scientific World Journal. 10: 90-102.
4. Frantz, S.; Hu, K.; Adamek, A.; et al. (2008). Transforming growth factor beta inhibition increases mortality and left ventricular dilatation after myocardial infarction. Basic Res. Cardiol. 103(5): 485-492.
5. Gokey, J. J., J. Snowball, A. Sridharan, J. P. Speth, et al. (2018). MEG3 is increased in idiopathic pulmonary fibrosis and regulates epithelial cell differentiation. JCI Insight. 3(17): e122490.
6. Grote, P., Wittler, L., Hendrix, D., Koch, F., Wahrisch, S., Beisaw, A., Macura, K., Blass, G., Kellis, M., Werber, M., and Herrmann B. G. (2013) The tissue-specific lncRNA Fendrr is an essential regulator of heart and body wall development in the mouse. Dev Cell. 24: 206-214.
7. Ishii et al. (2006). Identification of a novel non-coding RNA, MIAT, that confers risk of myocardial infarction. J Hum Genet. 51(12): 1087-1099.
8. Jiang W.; Xiong, Y.; Li, X.; Yang, Y. (2021). Cardiac Fibrosis: Cellular Effectors, Molecular Pathways, and Exosomal Roles. Frontier Cardio. Med. 8: Article 715258.Klattenhoff, C. A., Scheuermann, J. C., Surface, L. E., Bradley, R. K.., et al., (2013). Braveheart, a Long Noncoding RNA Required for Cardiovascular Lineage Commitment. Cell. 152(3): 570-583.
9. Kong, P.; Christia, P.; Frangogiannis, N. G. (2014). The Pathogenesis of Cardiac Fibrosis. Cell. Mol. Life Sci. 71(4): 549-574.
10. Korostowski, L., N. Sedlak, and N. Engel. (2012). The Kcnq1ot1 long non-coding RNA affects chromatin conformation and expression of Kcnq1, but does not regulate its imprinting in the developing heart. PloS Genet. 8(9): e1002956.
11. Kumarswamy, R., C. bauters, I. Volkmann, F. Maury, et al. (2014). Circulating long noncoding RNA, LIPCAR, predicts survival in patients with heart failure. Circ. Res. 114(10): 1569-1575.
12. McPherson, R., et al. (2007) A common allele on chromosome 9 associated with coronary heart disease. Science. 316(5830): 1488-1491.
13. Morfino, P.; Aimo, A.; Castiglione, V.; Galvez-Monton, C.; Emdin, M.; and Bayes-Genis, A. (2022). Treatment of cardiac fibrosis: from neuro-hormonal inhibitors to CAR-T cell therapy. Heart Fail. Rev. 11: 1-15.
14. Piccoli, M.-T; Gupta, S. K.; Viereck, J.; Foinquinos, A.; Samolovac, S.; Kramer, F. L.; Garg, A.; Remke, J.; Zimmer, K.; Batkai, S. and Thum, T. (2017). Inhibition of the Cardiac Fibroblast-Enriched lncRNA Meg3 Prevents Cardiac Fibrosis and Diastolic Dysfunction. Circulation Research. 121(5): 575-583.
15. Schonrock, N., Harvey, R. P., and J. S. Mattick (2012). Long noncoding RNAs in cardiac development and pathophysiology. Circ Res. 111(10): 1349-1362.
16. Thum, T.; Gross, C.; Fiedler, J.; Fischer, T.; Kissler, S.; Bussen, M.; et al. (2008) MicroRNA-21 contributes to myocardial disease by stimulating MAP kinase signalling in fibroblasts. Nature. 456: 980-984.
17. Ucar, A., Gupta, S. K., et al. (2012). The miRNA-212/132 family regulates both cardiac hypertrophy and cardiomyocyte autophagy. Nat Commun. 3: 1078.

18. Viereck, J., R. Kumarswamy, A. Foinquinos et al. (2016). Long noncoding RNA Chast promotes cardiac remodeling. Sci. Tranl Med. 8(326): 326ra22.
19. Zhang, Y.; G. Luo; Y. Zhang: M. Zhang; J. Zhou; W. Gao; X. Xuan; X. Yang; D. Yang; Z. Tian; B. Ni, and J. Tang. (2018). Critical effects of long non-coding RNA on fibrosis diseases. EMM. 50: e428.
20. Zhang, L., F. Zhao, W. Li, G. Song, V. Kasim, and S. Wu (2022). The Biological Roles and Molecular Mechanisms of Long Non-Coding RNA MEG3 in the Hallmarks of Cancer. Cancers (Basel). 14(24): 6032.

## Claims

**1.** An antisense oligonucleotide for targeting long non-coding RNA maternally expressed gene 3 (IncRNA Meg3), wherein the oligonucleotide comprises or consists of one of the following sequences in the 5' to 3' direction:

(i) C G C dC dG dC dC dA dT dA dT dC dT C C C (SEQ ID NO: 1);
(ii) C G G dA dC dA dA dA dA dC dT dG dG T T G (SEQ ID NO: 2);
(iii) A A G dA dG dT dC dT dC dC dT dC dC dT T A A (SEQ ID NO: 3);
(iv) G A T dT dA dG dC dC dC dT dG dT dG dT T C A (SEQ ID NO: 4);
(v) C G G dC dA dC dA dA dG dA dG dC dC dA A A G (SEQ ID NO: 5);
(vi) T C T dC dC dT dC dC dT dT dA dA dG dC C C C (SEQ ID NO: 6); or
(vii) G T C dT dC dC dT dC dC dT dT dA dA dG C C C (SEQ ID NO: 7);
wherein dA, dT, dG and dC are deoxyribonucleotides, or wherein dC is 5-methyl-2'-deoxycytidine (5mdC); and wherein A, T, G and C are modified or unmodified nucleotide building blocks.

**2.** The antisense oligonucleotide of claim 1, wherein the oligonucleotide comprises or consists of one of the following sequences in the 5' to 3' direction:

(i) C G C (5mdC) dG (5mdC)(5mdC) dA dT dA dT (5mdC) dT C C C (SEQ ID NO: 8);
(ii) C G G dA (5mdC) dA dA dA dA (5mdC) dT dG dG T T G (SEQ ID NO: 9);
(iii) AAG dA dG dT (5mdC) dT(5mdC)(5mdC) dT (5mdC)(5mdC) dT T A A (SEQ ID NO: 10);
(iv) G A T dT dA dG (5mdC)(5mdC)(5mdC) dT dG dT dG dT T C A (SEQ ID NO: 11);
(v) C G G (5mdC) dA (5mdC) dA dA dG dA dG (5mdC) (5mdC) dA A A G (SEQ ID NO: 12);
(vi) T C T (5mdC) (5mdC) dT (5mdC) (5mdC) dT dT dA dA dG (5mdC) C C C (SEQ ID NO: 13); or
(vii) G T C dT (5mdC) (5mdC) dT (5mdC) (5mdC) dT dT dA dA dG C C C (SEQ ID NO: 14);
wherein dA, dT, and dG are deoxyribonucleotides;
wherein 5mdC is 5-methyl-2'-deoxycytidine; and
wherein A, T, G and C are modified or unmodified nucleotide building blocks.

**3.** The antisense oligonucleotide of claim 1 or 2, wherein the oligonucleotide comprises bridged nucleic acid (BNA) building blocks, optionally wherein the BNA building blocks are locked nucleic acid (LNA) building blocks.

**4.** The antisense oligonucleotide of claim 3, wherein the LNA building blocks are located at the 3' and/or 5' terminal end of the oligonucleotide, optionally wherein each end comprises no more than 3 LNA building blocks, preferably wherein each end contains 3 LNA building blocks.

**5.** The antisense oligonucleotide of claim 3 or 4, wherein the oligonucleotide comprises one of the following sequences in the 5' to 3' direction:

(i) +C +G+C(5mdC)dG(5mdC)(5mdC)dAdTdAdT(5mdC)dT+C+C+C (SEQ ID NO: 15);
(ii) +C +G +G dA (5mdC) dA dA dA dA (5mdC) dT dG dG +T+T+G (SEQ ID NO: 16);
(iii) +A +A+G dA dG dT (5mdC) dT (5mdC) (5mdC) dT (5mdC) (5mdC) dT +T +A +A (SEQ ID NO: 17);
(iv) +G +A +T dT dA dG (5mdC)(5mdC)(5mdC) dT dG dT dG dT +T +C +A (SEQ ID NO: 18);
(v) +C +G +G (5mdC) dA (5mdC) dA dA dG dA dG (5mdC) (5mdC) dA +A +A +G (SEQ ID NO: 19);
(vi) +T +C +T (5mdC)(5mdC) dT (5mdC)(5mdC) dT dT dA dA dG (5mdC) +C +C +C (SEQ ID NO: 20); or
(vii) +G +T +C dT (5mdC) (5mdC) dT (5mdC)(5mdC) dT dT dA dA dG +C +C +C (SEQ ID NO: 21);
wherein +G, +T, +A, and +C are locked nucleic acid (LNA) building blocks;
wherein dA, dT, and dG are deoxyribonucleotide building blocks; and
wherein 5mdC is 5-methyl-2'-deoxycytidine.

6. The antisense oligonucleotide of any one of claims 1-5, wherein the oligonucleotide comprises one or more internucleoside linkage modifications, optionally wherein the one or more internucleoside linkage modification is a phosphorothioate (PS) linkage, optionally wherein all internucleoside linkages are PS linkage modifications.

7. The antisense oligonucleotide of claim 6, wherein the oligonucleotide comprises or consists of one of the following sequences in the 5' to 3' direction:

(i) +C*+G*+C*(5mdC)*dG*(5mdC)*(5mdC)*dA*dT*dA*dT*(5mdC)*dT*+C*+C*+C (SEQ ID NO: 22);
(ii) +C*+G*+G*dA*(5mdC)*dA*dA*dA*dA*(5mdC) *dT*dG*dG*+T*+T*+G (SEQ ID NO: 23);
(iii) +A*+A*+G*dA*dG*dT*(5mdC)*dT*(5mdC)*(5mdC)*dT*(5mdC)*(5mdC) *dT* +T* +A *+A (SEQ ID NO: 24);
(iv) +G*+A*+T*dT*dA*dG*(5mdC)*(5mdC)*(5mdC)*dT*dG*dT*dG*dT*+T*+C*+A (SEQ ID NO: 25);
(v) +C*+G*+G*(5mdC)*dA*(5mdC)*dA*dA*dG*dA*dG*(5mdC)*(5mdC)*dA*+A* +A*+G (SEQ ID NO: 26);
(vi) +T*+C*+T*(5mdC)*(5mdC)*dT*(5mdC)*(5mdC)*dT*dT*dA*dA* dG* (5mdC)* +C* +C*+C (SEQ ID NO: 27);
(vii) +G*+T*+C*dT* (5mdC)*(5mdC)*dT*(5mdC)* (5mdC)*dT* dT* dA* dA* dG* +C* +C* +C (SEQ ID NO: 28);
wherein dA, dT, and dG are deoxyribonucleotide building blocks;
wherein 5mdC is 5-methyl-2'-deoxycytidine;
wherein +G, +T, +A, and +C are locked nucleic acid (LNA) building blocks; and
wherein * is a phosphorothioate linkage, and/or wherein the oligonucleotide comprises 15-20 nucleotides, preferably wherein the oligonucleotide is 16 or 17 nucleotides long.

8. The antisense oligonucleotide of any one of claims 1-7, wherein the oligonucleotide is conjugated to one or more heterologous moieties, optionally wherein the heterologous moiety enhances cellular uptake of the oligonucleotide, optionally wherein the heterologous moiety is covalently bound to the oligonucleotide via a linker.

9. A cell comprising an antisense oligonucleotide of any one of claims 1-8.

10. A vector comprising an antisense oligonucleotide of any one of claims 1-8.

11. A pharmaceutical composition comprising the antisense oligonucleotide of any one of claims 1-8, and optionally a pharmaceutically acceptable carrier.

12. An antisense oligonucleotide of any one of claims 1-8, a vector of claim 10, or a pharmaceutical composition of claim 11, for prophylactic or therapeutic use in a subject.

13. An antisense oligonucleotide of any one of claims 1-8, a vector of claim 10, or a pharmaceutical composition of claim 11, for use in the prevention or treatment of a cardiac disease in a subject,

optionally wherein the cardiac disease is selected from a group consisting of (acute or subacute) heart failure, chronic and/or worsening chronic heart failure, stable heart failure, a less advanced state of heart failure or an advanced state of heart failure, heart failure of NYHA stage I, II, III and/or IV, myocardial infarction, myocarditis or cardiac fibrosis;
optionally wherein the oligonucleotide, the vector, or the pharmaceutical composition is administered by subcutaneous (s.c.) administration, intravenous (i.v.) administration, intramuscular administration, or oral administration.

14. The antisense oligonucleotide, the vector, or the pharmaceutical composition for use of claims 12 or 13, wherein there is a decrease in lncRNA Meg3 expression, optionally wherein there is an at least 30% reduction in Meg3 expression compared to control, and/or optionally wherein there is a decrease in the expression of fibrosis-related genes MMP2, COL1A1 and/or COL3A1.

15. The antisense oligonucleotide, the vector, or the pharmaceutical composition for use of claims 12-14, wherein the oligonucleotide is administered at a concentration between 0.05 to 20 mg/kg.

(A)
5'-
-3'
(B)
16mer (n=29)
17mer (n=68)

Figure 1

(A)
1.4
1.2
1.0
0.8
0.6
0.4
0.2
0.0
MEG3/TBP
Less than 50% reduction
Less than 60% reduction
HCF Ctrl
1154 16
1154 17
1155 16
1156 16
1209 17
1210 16
1210 17
1211 16
1211 17
1212 16
136 17
14146 17
14185 17
14186 17
14196 17
14197 17
14198 17
14204 17
14205 17
14206 17
14207 17
14208 17
14209 17
14210 17
14343 17
14344 17
14345 17
14421 17
16044 17
16045 16
16045 17
16046 16
16189 17
16191 17
16194 17
16195 17
19749 17
19754 17
19756 16
19756 17
19928 17
19929 16
19930 17
19931 16
19931 17
19933 17
19934 17
19935 16
GapmeR #
(B)
1.4
1.2
1.0
0.8
0.6
0.4
0.2
0.0
MEG3/TBP
Less than 50% reduction
Less than 60% reduction
HCF Ctrl
19935 17
19936 16
19936 17
19937 16
19937 17
19938 16
19938 17
19939 16
19939 17
19940 16
19940 17
19941 16
19941 17
19942 16
19962 17
19964 17
2014 17
2913 17
3068 16
3068 17
3069 16
3069 17
3070 16
4123 17
4642 17
4644 17
4645 17
4646 17
4647 16
4647 17
4648 17
4649 16
4649 17
4680 16
469 17
4734 17
4738 17
4739 17
4740 17
495 16
496 16
497 16
5045 17
6395 17
6396 17
774 17
775 16
775 17
GapmeR #

**Figure 2**

(A)
MMP2/TBP
2.0
1.8
1.6
1.4
1.2
1.0
0.8
0.6
0.4
0.2
0.0
Less than 40% reduction
Less than 50% reduction
HCF Ctrl
1154 16
1154 17
1155 16
1156 16
1209 17
1210 16
1210 17
1211 16
1211 17
1212 16
136 17
14146 17
14165 17
14186 17
14196 17
14197 17
14198 17
14204 17
14205 17
14206 17
14207 17
14208 17
14209 17
14210 17
14343 17
14344 17
14345 17
14421 17
16044 17
16045 16
16045 17
16046 16
16189 17
16191 17
16194 17
16195 17
19749 17
19754 17
19756 16
19756 17
19928 17
19929 16
19930 17
19931 16
19931 17
19933 17
19934 17
19935 16
GapmeR #
(B)
MMP2/TBP
2.0
1.8
1.6
1.4
1.2
1.0
0.8
0.6
0.4
0.2
0.0
Less than 40% reduction
Less than 50% reduction
HCF Ctrl
19935 17
19936 16
19936 17
19937 16
19937 17
19938 16
19938 17
19939 16
19939 17
19940 16
19940 17
19941 16
19941 17
19942 16
19962 17
19964 17
2014 17
2913 17
3068 16
3068 17
3069 16
3069 17
3070 16
4123 17
4642 17
4644 17
4645 17
4646 17
4647 16
4647 17
4648 17
4649 16
4649 17
4680 16
469 17
4734 17
4738 17
4739 17
4740 17
495 16
496 16
497 16
5045 17
6395 17
6396 17
774 17
775 16
775 17
GapmeR #

**Figure 3**

(A)
Less than 30% reduction
Less than 40% reduction
COL1A1/TBP
0.0
0.2
0.4
0.6
0.8
1.0
1.2
1.4
HCF Ctrl
1154 16
1154 17
1155 16
1156 16
1209 17
1210 16
1210 17
1211 16
1211 17
1212 16
136 17
14146 17
14185 17
14186 17
14196 17
14197 17
14198 17
14204 17
14205 17
14206 17
14207 17
14208 17
14209 17
14210 17
14343 17
14344 17
14345 17
14421 17
16044 17
16045 16
16045 17
16046 16
16189 17
16191 17
16194 17
16195 17
19749 17
19754 17
19756 16
19756 17
19928 17
19929 16
19930 17
19931 16
19931 17
19933 17
19934 17
19935 16
GapmeR #
(B)
Less than 30% reduction
Less than 40% reduction
COL1A1/TBP
0.0
0.2
0.4
0.6
0.8
1.0
1.2
1.4
HCF Ctrl
19935 17
19936 16
19936 17
19937 16
19937 17
19938 16
19938 17
19939 16
19939 17
19940 16
19940 17
19941 16
19941 17
19942 16
19962 17
19964 17
2014 17
2913 17
3068 16
3068 17
3069 16
3069 17
3070 16
4123 17
4642 17
4644 17
4645 17
4646 17
4647 16
4647 17
4648 17
4649 16
4649 17
4680 16
469 17
4734 17
4738 17
4739 17
4740 17
495 16
496 16
497 16
5045 17
6395 17
6396 17
774 17
775 16
775 17
GapmeR #


**Figure 4**

(A)
WST-1 assay
p = 0.0001
p = 0.0029
AUC (FC to LNA Ctrl)
# GapmeR
(B)
LDH assay
Cytotoxicity (%)
(LDH release)
# GapmeR

Figure 5

(C)
WST-1 assay
AUC (FC to LNA Ctrl)
# GapmeR
p = 0.0096
p = 0.0285
p = 0.0336
p = 0.0014
p = 0.0075
775_16
4123_16
14198_17
495_17
497_16
14209_16
19754_17
496_17
14207_16
6396_17
16045_17
4642_17
4647_17
19749_16
14208_17
14204_17
774_17
14205_17
14146_17
14197_17
4646_17
1154_16
LNA Ctrl
Lysis Ctrl
Untreated
-WST-1
(D)
LDH assay
Cytotoxicity (%) (LDH release)
# GapmeR
p = 0.0015
p = 0.033
p = 0.0112
p = 0.0054
p < 0.0001
p = 0.0003
p < 0.0001
p = 0.0035
p = 0.0447
p = 0.0005
p = 0.0404
Lysis Ctrl
Untreated
LNA Ctrl

Figure 5 (contd.)

(A)
MEG3
(B)
MMP2
(C)
COL1A1
(D)
COL3A1
FC to LNA Ctrl
1.5
1.0
0.5
0.0
****
LNA Ctrl
14204 17
14205 17
14209 17
19749 17
4647 16
497 16
6396 17
#GapmeR

Figure 6

(E)
MEG3
(F)
MMP2
(G)
COL1A1
(H)
COL3A1
FC to LNA Ctrl
LNA Ctl
14204 17
14205 17
14209 17
19749 17
4647 16
497 16
6396 17
#GapmeR

Figure 6 (contd.)

(I)
MEG3
(J)
MMP2
(K)
COL1A1
(L)
COL3A1
FC to LNA Ctrl
1.5
1.0
0.5
0.0
LNA Ctl
14204 17
14205 17
14209 17
19749 17
4647 16
497 16
6396 17
#GapmeR
****
**


Figure 6 (contd.)

(M)
MEG3
(N)
MMP2
(O)
COL1A1
(P)
COL3A1
FC to LNA Ctrl
#GapmeR
LNA Ctrl
14204 17
14205 17
14209 17
19749 17
4647 16
497 16
6396 17
1.5
1.0
0.5
0.0

Figure 6 (contd.)

WST-1 Assay
LDH Assay
(A)
HCF
(B)
HCF
(C)
HPLF
(D)
HPLF
(E)
HPKF
(F)
HPKF
Cell viability (FC to LNA Ctrl)
Cytotoxicity (%) (LDH release)
Lysis
-WST-1
Untreated
LNA Ctrl
6396_17
14205_17
14209_17
4647_16
14204_17
497_16
19749_17
# GapmeR

Figure 7

(A)
MEG3
FC to LNA Ctrl
1.5
1.0
0.5
0.0
****
****
****
****
****
****
****
LNA Ctl
14204 17
14205 17
14209 17
19749 17
4647 16
497 16
6396 17
#GapmeR
(B)
MMP2
FC to LNA Ctrl
1.5
1.0
0.5
0.0
****
****
****
0.1394
****
****
****
LNA Ctl
14204 17
14205 17
14209 17
19749 17
4647 16
497 16
6396 17
#GapmeR
(C)
COL1A1
FC to LNA Ctrl
1.5
1.0
0.5
0.0
****
****
****
****
***
***
****
LNA Ctl
14204 17
14205 17
14209 17
19749 17
4647 16
497 16
6396 17
#GapmeR
(D)
COL3A1
FC to LNA Ctrl
1.5
1.0
0.5
0.0
****
****
****
*
***
***
****
LNA Ctl
14204 17
14205 17
14209 17
19749 17
4647 16
497 16
6396 17
#GapmeR


Figure 8

(A)

Injection (i.v./s.c.)
Necropsy
Tissue collection
0 (days)
1
2
3
4
5
6
7
8
Blood collection: predose, 9min, 1h, 3h, 9h, 24h, 48h, 8d after administration


(B)

1E+05
1E+04
1000
100
10
1
0.1
Mean (ng/mL)
0
50
100
150
200
Time (hour)
Mean vs Time
1 mg/kg
5 mg/kg
10 mg/kg


(C)

1E+05
1E+04
1000
100
10
1
Mean (ng/mL)
0
50
100
150
200
Time (hour)
Mean vs Time
5 mg/kg
Mean vs Time
Concentration


**Figure 9**

(A)
1000
800
600
400
200
0
Concentration (ng/g)
i.v. 1 mg/kg
i.v. 5 mg/kg
i.v. 10 mg/kg
s.c. 5 mg/kg
(B)
2.5
2.0
1.5
1.0
0.5
0.0
TNFα/Hprt
(FC of low dose)
i.v. 1mg/kg
i.v. 5mg/kg
i.v. 10mg/kg
s.c. 5mg/kg
(D)
2.5
2.0
1.5
1.0
0.5
0.0
IL-6/Hprt
(FC of low dose)
*
i.v. 1mg/kg
i.v. 5mg/kg
i.v. 10mg/kg
s.c. 5mg/kg

Figure 10

(A)
PK Study Part
Injection (i.v.)
Necropsy
Tissue collection
0 (days) 1 2 3 4 5 6 7 8 9 10
Blood collection: predose, 9min, 1h, 3h, 9h, 24h, 48h, 72h 8d after administration
RF Study Part
Injection (i.v.)
Injection (i.v.)
Necropsy
Tissue collection
0 (days) 1 2 3 4 5 6 7 8 9 10 11 12 13 14 15 16 17
Blood collection: predose, 9min, 1h, 3h, 9h, 24h, 48h, 72h 8d after administration
(B)
Concentration (ng/g)
1000000
100000
10000
1000
100
10
1
0.1
0
20
40
60
80
100
150
200
Hours
0
1 mg/kg
5 mg/kg
20 mg/kg
40 mg/kg
(C)
Concentration (ng/g)
15000
10000
5000
0
Vehicle
i.v. 1 mg/kg
i.v. 5 mg/kg
i.v. 20 mg/kg
i.v. 40 mg/kg

Figure 11

(A)
Meg3/Hprt
(FC of 0.0 mg/kg)
2.0
1.5
1.0
0.5
0.0
*
*
0.0 mg/kg
1.0 mg/kg
5.0 mg/kg
20.0 mg/kg
40.0 mg/kg
(B)
IL-6/Hprt
(FC of 0.0 mg/kg)
2.0
1.5
1.0
0.5
0.0
0.0 mg/kg
1.0 mg/kg
5.0 mg/kg
20.0 mg/kg
40.0 mg/kg
(C)
IL-6/Hprt
(FC of 0.0 mg/kg)
2.5
2.0
1.5
1.0
0.5
0.0
0.0 mg/kg
1.0 mg/kg
5.0 mg/kg
20.0 mg/kg
40.0 mg/kg

Figure 12

(A)

Necropsy
Tissue collection
DRF/ Immunotox Study
Injection (i.v.)
Injection (i.v.)
0(days) 1 2 3 4 5 6 7 8 9 10 11 12 13 14 15 16
Blood collection: predose, 2d and 16d after administration


(B)

TNF-α
Day 2
Day 16
Day -14
1.5
1.0
0.5
0.0
pg/mL
male
female
Wistar Han rat
vehicle
FIBEX-003 low dose
FIBEX-003 mid dose
FIBEX-003 high dose


Figure 13

IL-1β
Day 2
Day 16
Day -14
pg/mL
20
15
10
5
0
male
female
Wistar Han rat
vehicle
FIBEX-003 low dose
FIBEX-003 mid dose
FIBEX-003 high dose

Figure 14

IL-6

Day 2
10
8
6
4
2
0
pg/mL
male
female
Wistar Han rat
Day 16
10
8
6
4
2
0
pg/mL
male
female
Wistar Han rat
Day -14
10
8
6
4
2
0
pg/mL
male
female
Wistar Han rat
vehicle
FIBEX-003 low dose
FIBEX-003 mid dose
FIBEX-003 high dose

Figure 15

IFN-γ
Day 2
Day 16
Day -14
pg/mL
1.5
1.0
0.5
0.0
male
female
Wistar Han rat
vehicle
FIBEX-003 low dose
FIBEX-003 mid dose
FIBEX-003 high dose

Figure 16

20000
15000
10000
5000
0
Concentration (ng/g)
Vehicle
i.v. 4 mg/kg
i.v. 20 mg/
i.v. 100 mg/kg

Figure 17

(A)
(B)
(C)
(D)
Meg3/Hprt (FC of vehicle)
IL-6/Hprt (FC of vehicle)
IL-6/Hprt (FC of Vehicle)
TNFa/Hprt (FC of Vehicle)
P=0.0685
*
Vehicle
i.v. 4mg/kg
i.v. 20mg/kg
i.v. 100mg/kg
i.v. 4 mg/kg
i.v. 20 mg/kg
i.v. 100 mg/kg
Vehicle (0 mg/kg)

Figure 18

A
B
300
250
200
150
10
5
0
FC of scramble probe
Scramble
Probe -1
Probe -2
600
400
200
6
4
2
0
C
3
2
1
0
-log (T-test p-value)
-3
-1
1
3
log (Fold Change)
NAT10
REEP5
MAP4
CERS2
PRKAR1A

Figure 19

Europäisches Patentamt
European Patent Office
Office européen des brevets

# EUROPEAN SEARCH REPORT

Application Number
EP 25 15 8845

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PICCOLI MARIA-TERESA ET AL: "Inhibition of the Cardiac Fibroblast-Enriched lncRNA Meg3 Prevents Cardiac Fibrosis and Diastolic Dysfunction", CIRCULATION RESEARCH, vol. 121, no. 5, 18 August 2017 (2017-08-18), pages 575-583, XP093010070, US ISSN: 0009-7330, DOI: 10.1161/CIRCRESAHA.117.310624 * the whole document * -& PICCOLI MARIA-TERESA: "Supplementary Material - Inhibition of the cardiac fibroblast-enriched lncRNA Meg3 prevents cardiac fibrosis and diastolic dysfunction", CIRCULATION RESEARCH, vol. 121, 18 August 2017 (2017-08-18), XP093293228, ----- | 1-15 | INV. C12N15/113 A61K31/7088 |
| X | US 2019/136236 A1 (MEDIZINISCHE HOCHSCHULE HANNOVER [DE]) 9 May 2019 (2019-05-09) * paragraphs [0001] - [0024], [0056] - [0057]; figure 2; example all * ----- | 1-15 | TECHNICAL FIELDS SEARCHED (IPC) C12N A61K |
| A | LI ZINING ET AL: "LncRNA MEG3: Potential stock for precision treatment of cardiovascular diseases", FRONTIERS IN PHARMACOLOGY, vol. 13, 29 November 2022 (2022-11-29), XP093293320, CH ISSN: 1663-9812, DOI: 10.3389/fphar.2022.1045501 * the whole document * ----- -/-- | 1-15 | |

~~The present search report has been drawn up for all claims~~

2

| Place of search | Date of completion of the search | Examiner |
|---|---|---|
| The Hague | 23 July 2025 | Schwarzmueller, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches
Patentamt
European
Patent Office
Office européen
des brevets

# EUROPEAN SEARCH REPORT

Application Number
EP 25 15 8845

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LU DONGCHAO ET AL: "RNA-based diagnostic and therapeutic strategies for cardiovascular disease", NATURE REVIEWS CARDIOLOGY, NATURE PUBLISHING GROUP, GB, vol. 16, no. 11, 11 June 2019 (2019-06-11), pages 661-674, XP036906358, ISSN: 1759-5002, DOI: 10.1038/S41569-019-0218-X [retrieved on 2019-06-11] * the whole document * ----- | 1-15 | |
| A | WO 2016/124628 A1 (JOHANN WOLFGANG GOETHE UNIVERSITÄT [DE]) 11 August 2016 (2016-08-11) * figure 6; example 4 * ----- | 1-15 | |
| A | XING YAN ET AL: "Long Noncoding RNA-Maternally Expressed Gene 3 Contributes to Hypoxic Pulmonary Hypertension", MOLECULAR THERAPY, vol. 27, no. 12, 1 December 2019 (2019-12-01), pages 2166-2181, XP093293750, United States ISSN: 1525-0016, DOI: 10.1016/j.ymthe.2019.07.022 * the whole document * -& XING YAN: "Supplementary Material - Long Noncoding RNA-Maternally Expressed Gene 3 Contributes to Hypoxic Pulmonary Hypertension", MOLECULAR THERAPY, 1 December 2019 (2019-12-01), XP093294212, ----- | 1-15 | |
| | | | TECHNICAL FIELDS SEARCHED (IPC) |

-/--

~~The present search report has been drawn up for all claims~~

2

| Place of search | Date of completion of the search | Examiner |
|---|---|---|
| The Hague | 23 July 2025 | Schwarzmueller, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

# EUROPEAN SEARCH REPORT

Application Number
EP 25 15 8845

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHENG XIAO ET AL: "Multifaceted roles of Meg3 in cellular senescence and atherosclerosis", ATHEROSCLEROSIS, vol. 392, 1 May 2024 (2024-05-01), page 117506, XP093293331, AMSTERDAM, NL ISSN: 0021-9150, DOI: 10.1016/j.atherosclerosis.2024.117506 * the whole document * -& CHENG XIAO: "Supplementary Material - Multifaceted roles of Meg3 in cellular senescence and atherosclerosis", ATHEROSCLEROSIS, 1 May 2024 (2024-05-01), XP093294144, ----- | 1-15 | |

TECHNICAL FIELDS SEARCHED (IPC)

~~The present search report has been drawn up for all claims~~

2

| Place of search | Date of completion of the search | Examiner |
|---|---|---|
| The Hague | 23 July 2025 | Schwarzmueller, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

Europäisches
Patentamt
European
Patent Office
Office européen
des brevets

Application Number

EP 25 15 8845

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

# LACK OF UNITY OF INVENTION SHEET B

Application Number

EP 25 15 8845

The Search Division considers that the present European patent application does not comply with the requirements of unity of invention and relates to several inventions or groups of inventions, namely:

1. claims: 1-15(partially)

An antisense oligonucleotide for targeting long non-coding RNA maternally expressed gene 3 (lncRNA Meg3), wherein the oligonucleotide comprises or consists of a base sequence according to SEQ ID NO: 1, or corresponding SEQ ID NOs: 8, 15 & 22; a cell, a vector or a pharmaceutical composition comprising said oligonucleotide; said oligonucleotide or composition for use in therapy and for use in the treatment of a cardiac disease.

---

2-4. claims: 1-15(partially)

As for invention 1) but wherein the oligonucleotide is characterized by any of SEQ ID NOs: 2, 4 & 5, respectively, or their corresponding sequences defined by SEQ ID NOs: 9, 11, 12, 16, 18, 19, 23, 25 & 26.

---

5. claims: 1-15(partially)

As for invention 1) but wherein the oligonucleotide is characterized by the SEQ ID NOs: 3, 6 or 7 or their corresponding sequences defined by SEQ ID NOs: 10, 13, 14, 17, 20, 21, 24, 27 & 28.

---

## ANNEX TO THE EUROPEAN SEARCH REPORT ON EUROPEAN PATENT APPLICATION NO. EP 25 15 8845

This annex lists the patent family members relating to the patent documents cited in the above-mentioned European search report. The members are as contained in the European Patent Office EDP file on
The European Patent Office is in no way liable for these particulars which are merely given for the purpose of information.

23-07-2025

| Patent document cited in search report | Publication date | Patent family member(s) | Publication date |
|---|---|---|---|
| US 2019136236 A1 | 09-05-2019 | EP 3452593 A1 | 13-03-2019 |
| | | JP 6928001 B2 | 01-09-2021 |
| | | JP 2019518439 A | 04-07-2019 |
| | | US 2019136236 A1 | 09-05-2019 |
| | | WO 2017191021 A1 | 09-11-2017 |
| WO 2016124628 A1 | 11-08-2016 | EP 3054012 A1 | 10-08-2016 |
| | | EP 3253872 A1 | 13-12-2017 |
| | | US 2018023080 A1 | 25-01-2018 |
| | | WO 2016124628 A1 | 11-08-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017191021 A
- EP 3452593 A


### Non-patent literature cited in the description


- **BERK, B. C.** ; **FUJIWARA, K.** ; **LEHOUX, S.** ECM remodeling in hypertensive heart disease. *J. Clin. Invest.*, 2007, vol. 117 (3), 568-575
- **BRAENDLI-BAIOCCO, A.** ; **M. FESTAG** ; **K. DUMONG ERICHSEN et al.** From the Cover: The Minipig is a Suitable Non-Rodent Model in the Safety Assessment of Single Stranded Oligonucleotides. *Toxicol. Sci.*, 2017, vol. 157 (1), 112-128
- **CALEY, D. P.** ; **R. C. PINK** ; **D. TRUJILLANO** ; **D. R. F. CARTER**. Long noncoding RNAs, chromatin, and development. *Scientific World Journal*, 2010, vol. 10, 90-102
- **FRANTZ, S.** ; **HU, K.** ; **ADAMEK, A. et al.** Transforming growth factor beta inhibition increases mortality and left ventricular dilatation after myocardial infarction. *Basic Res. Cardiol.*, 2008, vol. 103 (5), 485-492
- **GOKEY, J. J.** ; **J. SNOWBALL** ; **A. SRIDHARAN** ; **J. P. SPETH et al.** MEG3 is increased in idiopathic pulmonary fibrosis and regulates epithelial cell differentiation. *JCI Insight*, 2018, vol. 3 (17), e122490
- **GROTE, P.** ; **WITTLER, L.** ; **HENDRIX, D.** ; **KOCH, F.** ; **WAHRISCH, S.** ; **BEISAW, A.** ; **MACURA, K.** ; **BLASS, G.** ; **KELLIS, M.** ; **WERBER, M.** The tissue-specific lncRNA Fendrr is an essential regulator of heart and body wall development in the mouse. *Dev Cell.*, 2013, vol. 24, 206-214
- **ISHII et al.** Identification of a novel non-coding RNA, MIAT, that confers risk of myocardial infarction. *J Hum Genet.*, 2006, vol. 51 (12), 1087-1099
- **JIANG W.** ; **XIONG, Y.** ; **LI, X.** ; **YANG, Y.** Cardiac Fibrosis: Cellular Effectors, Molecular Pathways, and Exosomal Roles. *Frontier Cardio. Med.*, 2021, vol. 8 (715258)
- **KLATTENHOFF, C. A.** ; **SCHEUERMANN, J. C.** ; **SURFACE, L. E.** ; **BRADLEY, R. K.. et al.** Braveheart, a Long Noncoding RNA Required for Cardiovascular Lineage Commitment. *Cell*, 2013, vol. 152 (3), 570-583
- **KONG, P.** ; **CHRISTIA, P.** ; **FRANGOGIANNIS, N. G.** The Pathogenesis of Cardiac Fibrosis. *Cell. Mol. Life Sci.*, 2014, vol. 71 (4), 549-574
- **KOROSTOWSKI, L.** ; **N. SEDLAK** ; **N. ENGEL**. The Kcnq1ot1 long non-coding RNA affects chromatin conformation and expression of Kcnq1, but does not regulate its imprinting in the developing heart. *PloS Genet.*, 2012, vol. 8 (9), e1002956
- **KUMARSWAMY, R.** ; **C. BAUTERS** ; **I. VOLKMANN** ; **F. MAURY et al.** Circulating long noncoding RNA, LIPCAR, predicts survival in patients with heart failure. *Circ. Res.*, 2014, vol. 114 (10), 1569-1575
- **MCPHERSON, R. et al.** A common allele on chromosome 9 associated with coronary heart disease. *Science*, 2007, vol. 316 (5830), 1488-1491
- **MORFINO, P.** ; **AIMO, A.** ; **CASTIGLIONE, V.** ; **GALVEZ-MONTON, C.** ; **EMDIN, M.** ; **BAYES-GENIS, A.** Treatment of cardiac fibrosis: from neuro-hormonal inhibitors to CAR-T cell therapy. *Heart Fail. Rev.*, 2022, vol. 11, 1-15
- **PICCOLI, M.-T** ; **GUPTA, S. K.** ; **VIERECK, J.** ; **FOINQUINOS, A.** ; **SAMOLOVAC, S.** ; **KRAMER, F. L.** ; **GARG, A.** ; **REMKE, J.** ; **ZIMMER, K.** ; **BATKAI, S.** Inhibition of the Cardiac Fibroblast-Enriched lncRNA Meg3 Prevents Cardiac Fibrosis and Diastolic Dysfunction. *Circulation Research*, 2017, vol. 121 (5), 575-583
- **SCHONROCK, N.** ; **HARVEY, R. P.** ; **J. S. MATTICK**. Long noncoding RNAs in cardiac development and pathophysiology. *Circ Res.*, 2012, vol. 111 (10), 1349-1362
- **THUM, T.** ; **GROSS, C.** ; **FIEDLER, J.** ; **FISCHER, T.** ; **KISSLER, S.** ; **BUSSEN, M. et al.** MicroRNA-21 contributes to myocardial disease by stimulating MAP kinase signalling in fibroblasts. *Nature*, 2008, vol. 456, 980-984
- **UCAR, A.** ; **GUPTA, S. K. et al.** The miRNA-212/132 family regulates both cardiac hypertrophy and cardiomyocyte autophagy. *Nat Commun.*, 2012, vol. 3, 1078
- **VIERECK, J.** ; **R. KUMARSWAMY** ; **A. FOINQUINOS et al.** Long noncoding RNA Chast promotes cardiac remodeling. *Sci. Tranl Med.*, 2016, vol. 8 (326), 326-22

- **ZHANG, Y.** ; **G. LUO** ; **Y. ZHANG** ; **M. ZHANG** ; **J. ZHOU** ; **W. GAO** ; **X. XUAN** ; **X. YANG** ; **D. YANG** ; **Z. TIAN**. Critical effects of long non-coding RNA on fibrosis diseases. *EMM*, 2018, vol. 50, e428
- **ZHANG, L.** ; **F. ZHAO** ; **W. LI** ; **G. SONG** ; **V. KASIM** ; **S. WU**. The Biological Roles and Molecular Mechanisms of Long Non-Coding RNA MEG3 in the Hallmarks of Cancer. *Cancers (Basel)*, 2022, vol. 14 (24), 6032